# EUROPEAN PATENT SPECIFICATION

(11) **EP 1 709 161 B1**
(45) Date of publication and mention of the grant of the patent: **01.10.2008**
(21) Application number: 05707792.7
(22) Date of filing: 18.01.2005
(51) Int. Cl.: C12N 5/10, C07K 14/48, A61K 9/22, A61K 38/18, C12N 15/85, A61P 25/00

(54) **HUMAN THERAPEUTIC CELLS SECRETING NERVE GROWTH FACTOR**
NERVENWACHSTUMSFAKTOR SEZERNIERENDE MENSCHLICHE THERAPEUTISCHE ZELLEN
CELLULES THERAPEUTIQUES HUMAINES A SECRETION DU FACTEUR DE CROISSANCE NERVEUSE

(30) Priority: 19.01.2004 DK 200400064; 20.01.2004 US 537033 P; 30.04.2004 DK 200400673; 28.05.2004 US 575087 P
(43) Date of publication of application: 11.10.2006
(73) Proprietor: NsGene A/S, 2750 Ballerup (DK)
(72) Inventor: TORNOE, Jens, DK-2100 Kobenhavn O (DK); KUSK, Philip, DK-3540 Lynge (DK); WAHLBERG, Lars, DK-4550 Asnaes (DK)
(74) Representative: HOEIBERG A/S
(86) International application number: PCT/EP2005/050180
(87) International publication number: WO 2005/068498

(56) References cited:
- EP-A- 1 179 350
- KANUGA NAHEED ET AL: "Characterization of genetically modified human retinal pigment epithelial cells developed for in vitro and transplantation studies." INVESTIGATIVE OPHTHALMOLOGY & VISUAL SCIENCE. FEB 2002, vol. 43, no. 2, February 2002 (2002-02), pages 546-555, XP002336959 ISSN: 0146-0404
- HOFFMAN D ET AL: "TRANSPLANTATION OF A POLYMER-ENCAPSULATED CELL LINE GENETICALLY ENGINEERED TO RELEASE NGF" EXPERIMENTAL NEUROLOGY, ACADEMIC PRESS, NEW YORK, NY, US, vol. 122, no. 1, July 1993 (1993-07), pages 100-106, XP008003197 ISSN: 0014-4886
- WINN S R ET AL: "POLYMER-ENCAPSULATED CELLS GENETICALLY MODIFIED TO SECRETE HUMAN NERVE GROWTH FACTOR PROMOTE THE SURVIVAL OF AXOTOMIZED SEPTAL CHOLINERGIC NEURONS" PROCEEDINGS OF THE NATIONAL ACADEMY OF SCIENCES OF USA, NATIONAL ACADEMY OF SCIENCE. WASHINGTON, US, vol. 91, no. 6, 6 March 1994 (1994-03-06), pages 2324-2328, XP001076846 ISSN: 0027-8424 cited in the application
- TORNOE J ET AL: "Generation of a synthetic mammalian promoter library by modification of sequences spacing transcription factor binding sites" GENE: AN INTERNATIONAL JOURNAL ON GENES AND GENOMES, ELSEVIER SCIENCE PUBLISHERS, BARKING, GB, vol. 297, no. 1-2, 4 September 2002 (2002-09-04), pages 21-32, XP004388011 ISSN: 0378-1119 cited in the application

## Description

### FIELD OF INVENTION

The present invention relates to human cell lines genetically modified to overexpress bioactive NGF. In another aspect the present invention relates to encapsulated human cell lines genetically modified to overexpress bioactive NGF, which can be used in therapy of Alzheimer's disease, peripheral neuropathy and other neurological disorders amenable to local and prolonged NGF therapy.

### BACKGROUND OF THE INVENTION

Because NGF does not readily cross the blood brain barrier, its administration into the CNS requires the use of invasive procedures, which may compromise the integrity of the blood brain barrier. For example, in the rodent preclinical studies that demonstrated the potential efficacy of exogenous NGF, the NGF was administered with osmotic minipumps or through chronic intraventricular cannulae. Those techniques require repeated infusions into the brain, either through injections via the cannulae, or from pumps, which must be refilled every time the reservoir is depleted. Every occasion in which the pump reservoir must be replaced or the injection syringe reinserted through the cannulae represents another opportunity that contaminants might be introduced into the brain, which is especially susceptible to infection.

Even with the careful use of sterile procedures, there is risk of infection. It has been reported that even in intensive care units, intracerebroventricular catheters used to monitor intracranial pressure become infected with bacteria after about three days (Saffran, Perspectives in Biology and Medicine, 35, pp. 471-86 (1992)). In addition to the risk of infection, there seems to be some risk associated with the infusion procedure. Infusions into the ventricles have been reported to produce hydrocephalus (Saffran et al., Brain Research, 492, pp. 245-254 (1989)) and continuous infusions of solutions into the parenchyma is associated with cell necrosis in the brain.

Use of foetal tissue to replace degenerated neurons is clouded by ethical concerns and unencapsulated non-foetal cells may be rejected or produce tumours. In addition, tissue taken from foetal sources may be highly variable. Last, direct infection of host cells by viral vectors to produce NGF by in vivo gene therapy is associated with risks of insertional mutagenesis and tumorigenesis as well as the inability to stop NGF secretion if untoward effects should occur. By encapsulating NGF-producing cells, exogenous NGF can be supplied with a minimal risk of infection, without the use of foetal tissue or viral vectors, and without the risk of tissue rejection or tumour development.

Finally, concerns have also been expressed about whether exogenous NGF at the doses previously used itself might prove harmful or toxic, perhaps even accelerating the neurodegenerative processes associated with Alzheimer's disease (Saffran, Perspectives in Biology and Medicine, 35, pp. 471-86 (1992)). It has been suggested that exogenous NGF might accelerate tangle formation, initiate axon sprouting of perivascular sympathetic axons potentially leading to changes in cerebral blood flow, or remodel the projections of basal forebrain neurons in response to the exogenous NGF such that not-yet-affected basal forebrain neurons become dysfunctional and thus accelerating the dementing process (Saffran, Perspectives in Biology and Medicine, 35, pp. 471-86 (1992)).

Administration of the previously reported doses of NGF may have undesirable side effects including severe weight loss, pain, listlessness, hypophagia and recurrence of herpes infection.

The required doses of administered NGF have been relatively high, largely because the half-life of NGF is extremely short. Reports state a half life of 45 minutes when administered to the brain interstitium (Krewson & Salzman, 1996, Brain Res 727:169-181). The serum half-life of NGF in rats has been measured to be 7.2 minutes (Poduslo & Curran, 1996, Brain Res Mol Brain Res, 36:280-286). The short half life also means that the reservoirs associated with minipumps need to be replenished very often and/or the solutions of NGF will need to contain various preservatives, which in themselves may cause side-effects. The side effects reported from NGF infusion studies may also be caused by the uncharacterised breakdown products of NGF.

The prior art discloses methods and devices for delivery of NGF from encapsulated BHK cells (Winn et al 1994, PNAS, 91:2324-2328). Although these cells secreted large amounts of NGF (275-325 pg NGF per 10³ cells per hour) and proof of concept was provided in rats, these cells have not been used in clinical studies despite the long period the cells have been available.

The major reason for this is that BHK cell containing capsules are not useful for therapy of human beings, because of the potential risk of triggering an immune-response or zoonosis from the use of xenogeneic cells and the poor long-term viability BHK cells show after encapsulation due to lack of contact inhibition and continued proliferation. Although BHK cells were genetically modified to secrete human NGF they inherently also secrete a number of hamster proteins, which may be immunogenic to human beings. In any event, BHK cells do not have the required viability when grown under stressed conditions in artificial growth media (US 6,361,771).

Accordingly there is a need for providing human cells suitable for encapsulation, which cells secrete therapeutically relevant amounts of Nerve Growth Factor. Such cells are not found in the prior art.

### SUMMARY OF THE INVENTION

In a first aspect the invention relates to a human cell line comprising a stably integrated expression construct comprising the following sequence:
a mammalian promoter,
a coding sequence operably linked to said promoter, wherein said coding sequence codes for NGF or a bioactive variant of NGF, and
an intron located in the transcript.

The present inventors have determined that pre-pro-NGF is only secreted at very low levels in a human contact inhibited cell line in the absence of an intron in the transcript. In contrast, secretion from traditional producer cell lines such as CHO, and HEK293T cells is high even in the absence of an intron. CHO and HEK293T cells are not suited for encapsulation, primarily because they are not contact inhibited. CHO cells are also not suited for human therapy as they are non-human.

By inserting an intron into the transcript the level of secretion is unexpectedly increased several fold in particular in human contact inhibited cells.

In a further aspect, the invention relates to an implantable cell culture device, the device comprising:
a semipermable membrane permitting the diffusion of NGF therethrough; and
a population of cell from the cell line according to the invention disposed within the semipermeable membrane.

In a further aspect the invention relates to the use of the cell line of the invention, for the preparation of a medicament.

In a further aspect the invention relates to the use of the device of the invention, for the preparation of a medicament.

In a still further aspect the invention relates to a method of treatment of a neurological disorder comprising transplanting the cells of the invention.

In a further aspect the invention relates to a method of treatment of a neurological disorder, comprising insertion into a subject in need thereof at least one device of the invention.

According to this invention safe cellular or capsular delivery of NGF, synthesized in vivo, to the human body can be accomplished using the cells and capsules according to the present invention.

According to one aspect of this invention, the beneficial effects of exogenous NGF for the treatment of age related cognitive defects, including Huntington's disease, Parkinson's disease, Alzheimer's and ALS, may be obtained with higher safety as the therapy may be performed with human - preferably contact-inhibited - cell lines as opposed to rodent and other non-human cell lines.

The invention may be used in a method of treatment comprising transplantation of a cell line of the invention on a solid or semi-solid support such as but not limited gelatine beads or other substrates allowing for contact inhibition and survival.

In a still further aspect the invention relates to a pharmaceutical composition comprising the cell line of the invention attached to a matrix.

The invention may further be used in a method of treatment of Alzheimer's Disease, comprising administering to an individual in need thereof a composition of ARPE-19 cells transfected with a vector selected from the group consisting of pCIn.hNGF, pCInKhNGF, and pNS1n.rlNSintrA-hNGF as defined in the present application, said composition being capable of secreting a therapeutically effective amount of mature human NGF.

The invention may further be used in a method of treatment of Alzheimer's Disease, comprising administering to an individual in need thereof a device, said device comprising a semipermeable membrane permitting the diffusion of human mature NGF therethrough, the device further comprising a composition of ARPE-19 cells transfected with a vector selected from the group consisting of pCIn.hNGF, pCInKhNGF, and pNS1n.rINSintrA-hNGF, said device being capable of releasing a therapeutically effective amount of mature human NGF.

In a further aspect, the invention relates to an ARPE-19 cell line transfected with a vector selected from the group consisting of pCIn.hNGF, pCInKhNGF, and pNS1n.rINSintrA-hNGF.

In a further aspect, the invention relates to a composition of ARPE-19 cells being capable of secreting in excess of 10 ng of human mature NGF/ml/24 hours, when grown in confluent cultures in serumfree medium.

In a further aspect, the invention relates to an implantable cell device, the device comprising:
(a) a semipermable membrane permitting the diffusion of human mature NGF therethrough; and
(b) a population of ARPE-19 cells disposed within the semipermeable membrane, said device being capable of releasing in excess of 0.1 ng human mature NGF per 24 hours when grown in serumfree medium.
Preferably, this device has the layout and dimensions as specified in the present application.

### Drawings

Figure 1. Differential NGF expression in transiently transfected cell lines.
Figure 2: Intron-effect on in vitro NGF release (isolated clones +/- CI).
Figure 4: Vector map of the pNN expression vector with the CMV promoter and a hNGF coding sequence. The pNN expression vector is based on the pNS1n expression vector (figure 5).

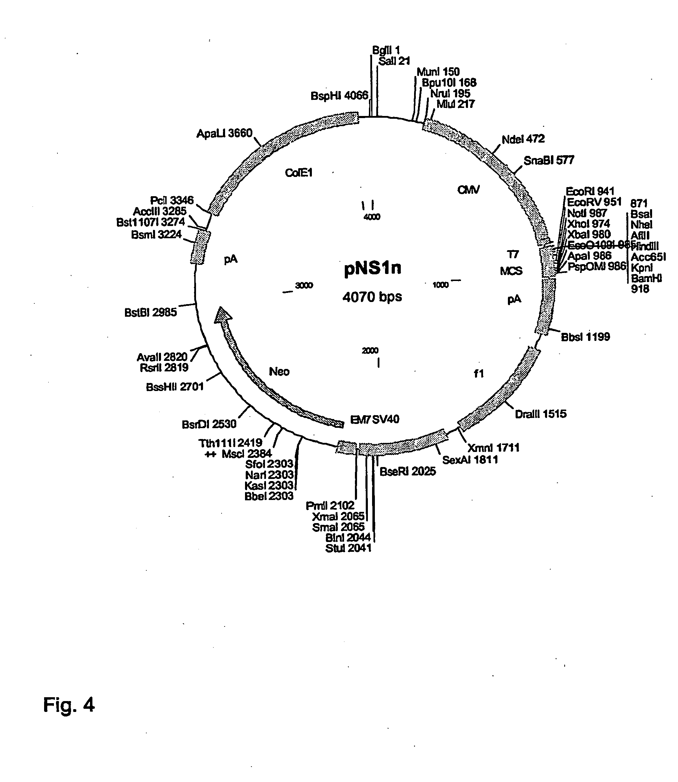

Figure 4: Vector map of the empty pNS1n expression vector. pNS1n is a customised vector derived from pcDNA3 (Invitrogen) with expression under control of the cytomegalovirus promoter. The vector carries the neo resistance marker instead of zeo (Jensen et al 2002, J Biol Chem, 277: 41438-41447).

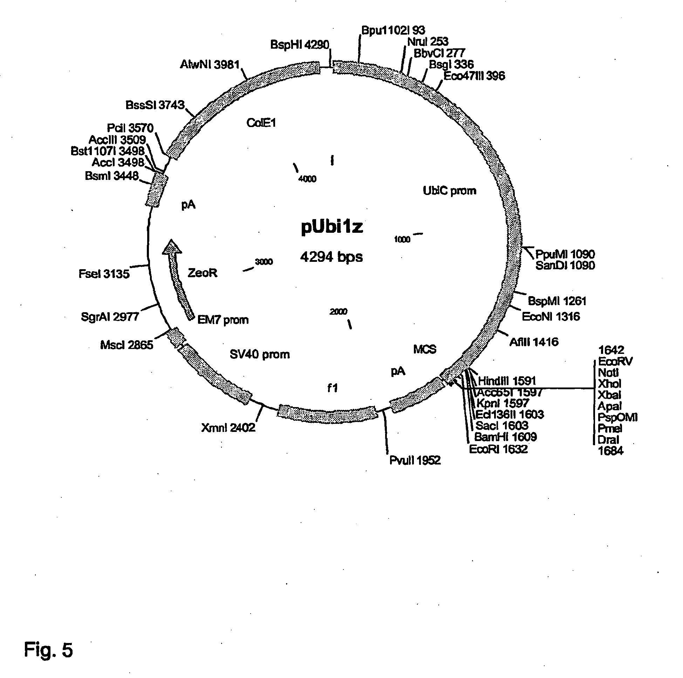

Figure 5: Vector map of empty pUBI1z expression vector (Johansen et al 2003, J Gene Medicine, 5:1080-1089).

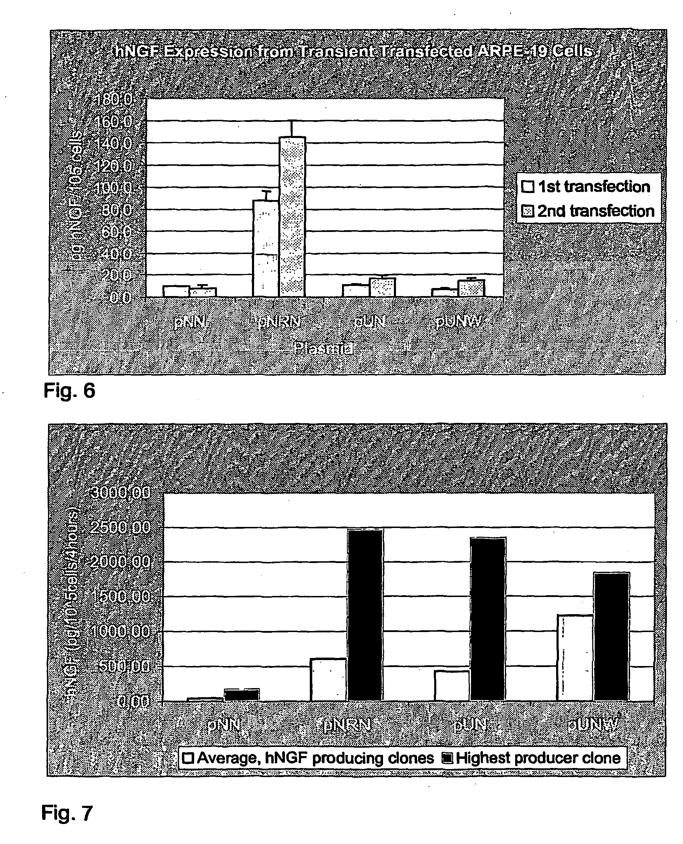

Figure 6: Quantity of hNGF secreted from 10⁵ transiently transfected ARPE-19 cells into cell growth medium during 48 hours. Each bar is the average of three transfections, each transfection measured in duplicate in the hNGF ELISA.
Figure 7: Comparison of hNGF expression levels from stable clones of each of four expression constructs. The average of 5 to 10 stable clones expressing more than 2-fold over background is shown for each construct (grey bars) as well as the highest NGF-secreting clone (black bars).

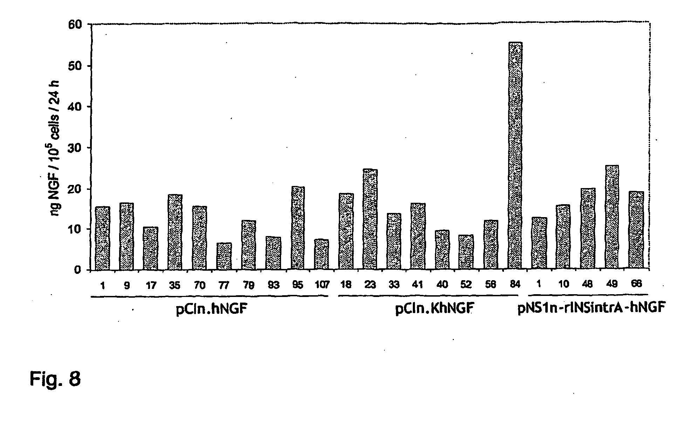

Figure 8: NGF release measured by NGF Elisa from stable clones. For explanation see the examples.

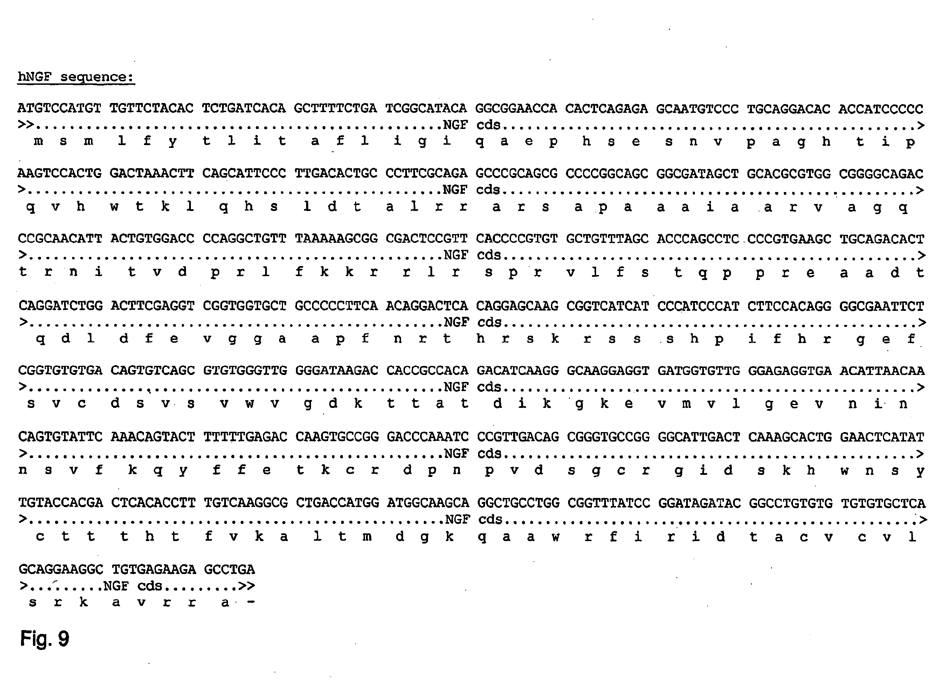

Figure 9: The coding nucleotide sequence of human pre-pro-NGF (SEQ ID No 1) and the translated amino acid sequence of human pre-pro NGF (SEQ ID No 2).

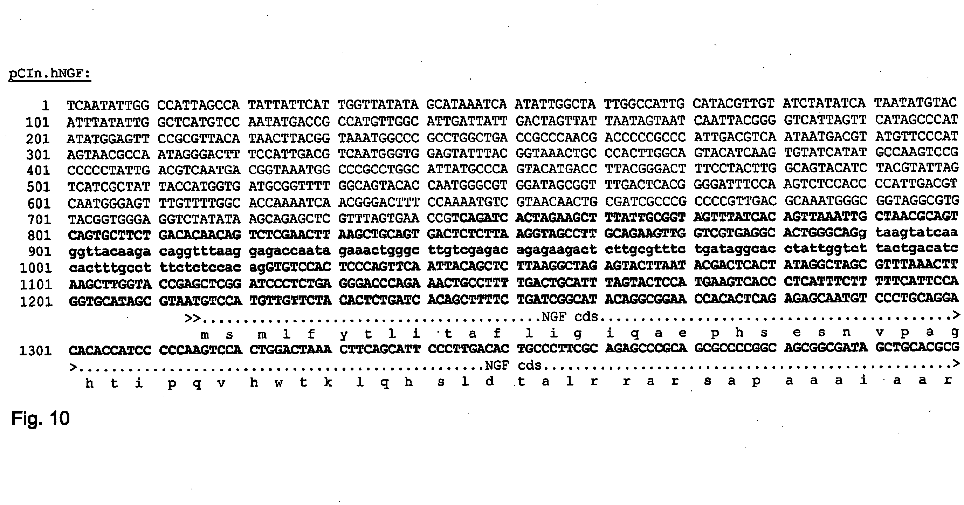

Figure 10: The nucleotide sequence (SEQ ID No 14) of part of the pCIn.hNGF expression vector spanning from the first base of the CMV promoter to the NGF coding sequence. The transcript is shown in bold. The chimeric intron of the pCI vector is shown in lowercase letters. A part of the N-terminal of human pre-pro-NGF is shown (SEQ ID No 15).

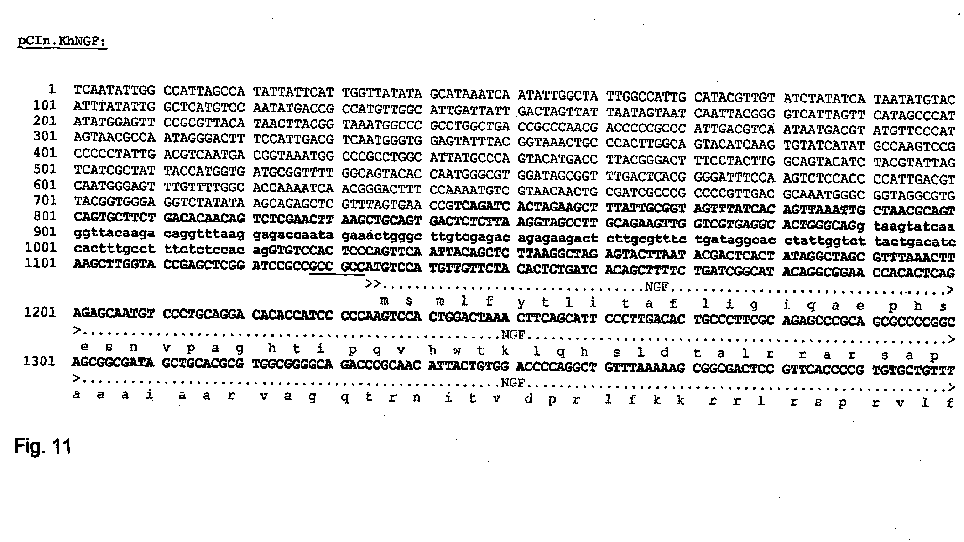

Figure 11. The nucleotide sequence (SEQ ID No 16) of part of the pCIn.KhNGF expression vector spanning from the first base of the CMV promoter to the NGF coding sequence. The transcript is shown in bold. The chimeric intron of the pCI vector is shown in lowercase letters. A Kozak consensus sequence is underlined. A part of the N-terminal of human pre-pro-NGF is shown (SEQ ID No 17).

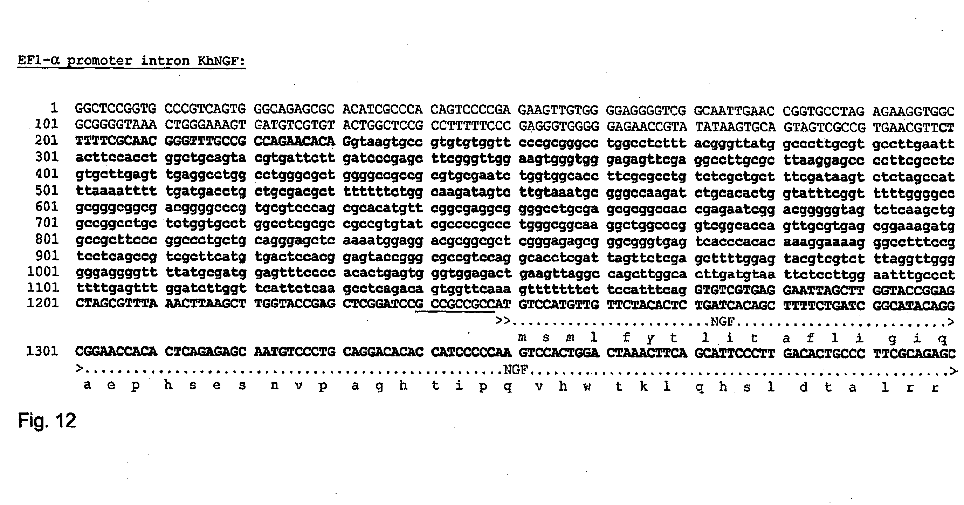

Figure 12. The nucleotide sequence (SEQ ID No 18) of part of the EF-1α promoter spanning from the first base of the promoter to the hNGF coding sequence. The transcript is shown in bold. The EF-1α intron is shown in lowercase letters. A Kozak consensus sequence is underlined. A part of the N-terminal of human pre-pro-NGF is shown (SEQ ID No 19).

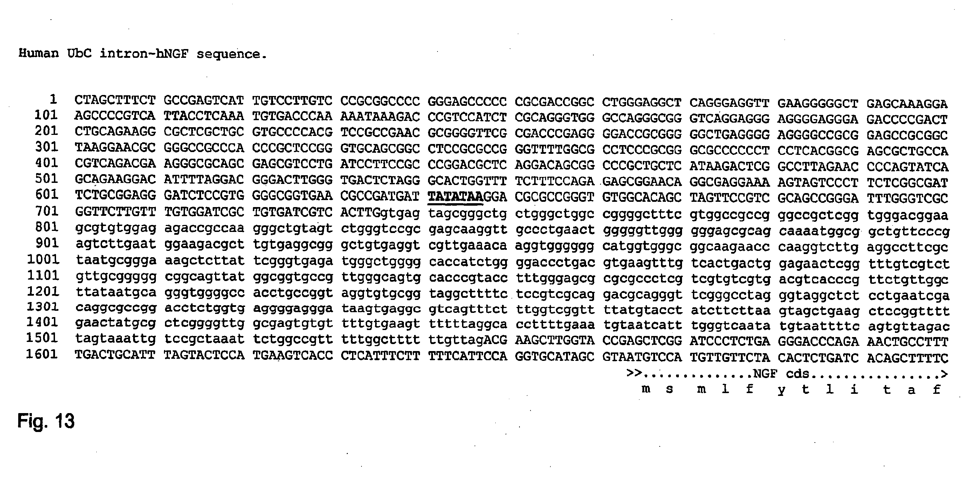

Figure 13. The nucleotide sequence (SEQ ID No 20) of part of the pUN expression vector spanning from the first base of the human UbC promoter to the NGF coding sequence. The TATA box is shown in bold and underlined. The ubiquitin intron is shown in lowercase letters. A part of the N-terminal of human pre-pro-NGF is shown (SEQ ID No 21).

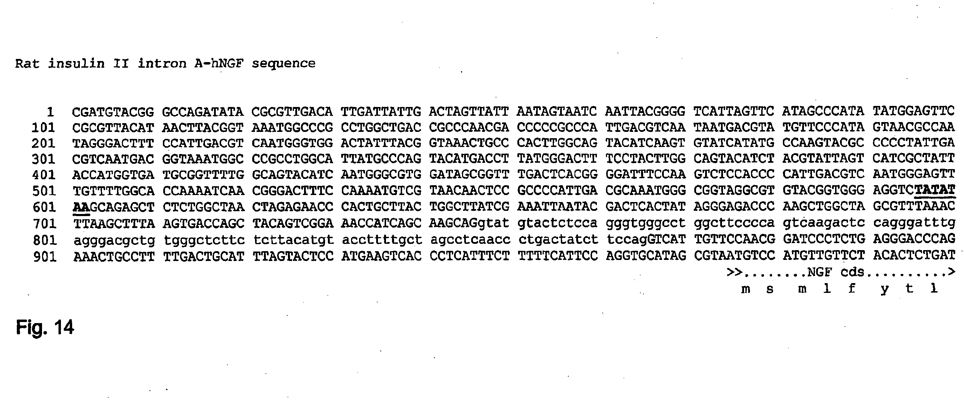

Figure 14. The nucleotide sequence (SEQ ID No 22) of part of the pNRN expression vector spanning from the first base of the CMV promoter to the NGF coding sequence. The TATA box is shown in bold and underlined. The rat insulin intron A is shown in lowercase letters. A part of the N-terminal of human pre-pro-NGF is shown (SEQ ID No 22).

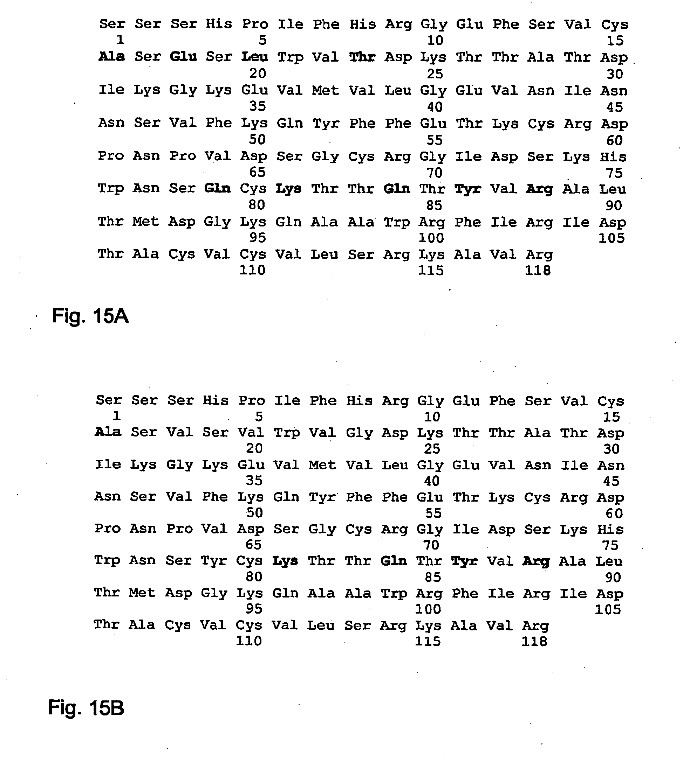

Figure 15 shows the mature part of preferred human pantropic neurotrophins with mutations relative to wildtype NGF shown in bold. Figure 15A shows pantopic NGF with mutations D16A-V18E-V20L-G23T-Y79Q-T81K-H84Q-F86Y-K88R-hNGF (SEQ ID No 7). Figure 15B shows pantropic NGF with mutations D16A-T81 K-H84Q-F86Y-K88R-hNGF (SEQ ID No 8).

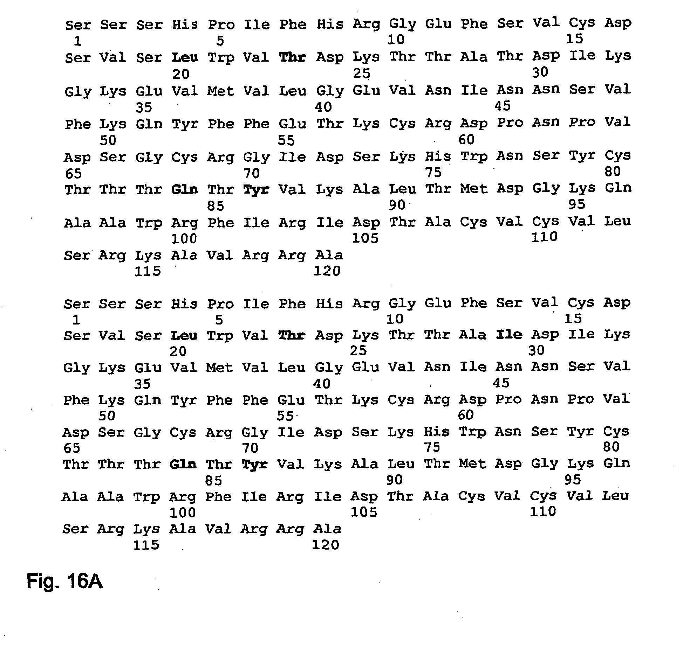

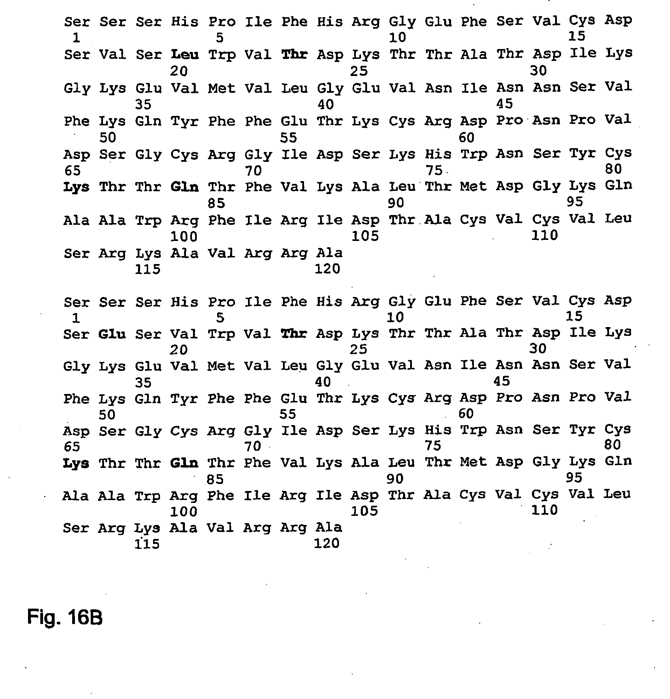

Figure 16 shows the mature part of preferred trkC binding human NGF variants with mutations relative to wildtype NGF shown in bold. Figure 16A shows NGF130 (top; SEQ ID No 9) and NGF131 (bottom; SEQ ID No 10). NGF130 contains the four changes: V20L, G23T, H84Q and F86Y. NGF131 contains the five changes V20L, G23T, T29I, H84Q, AND F86Y. Figure 16B shows NGFR2 (top; SEQ ID No. 11) and NGFR3 (bottom; SEQ ID No. 12). NGFR2 contains the four changes V20L, G23T, T81K, and H84Q. NGFR3 contains the four changes V18E, G23T, T81K, and H84Q.

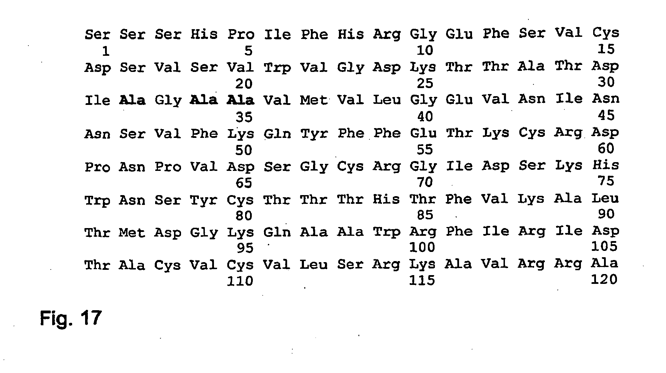

Figure 17 shows the mature part (SEQ ID No. 13) of a preferred human mature NGF with reduced ability to bind p75^{NGFR} NGF. The mutations relative to wildtype NGF are shown in bold.

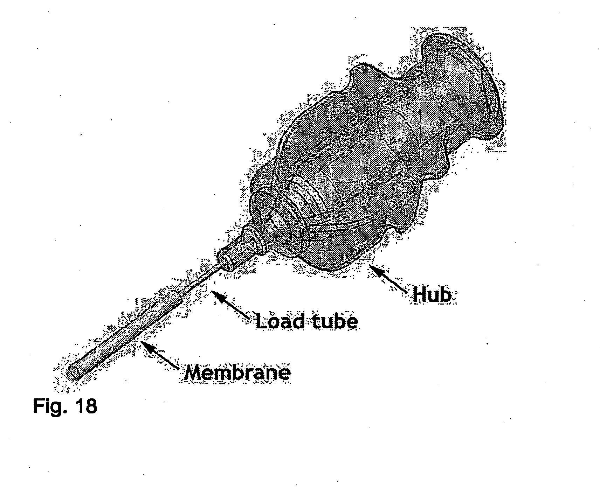

Figure 18 shows an encapsulation device mounted on the hub by the load tube prior to cell loading. Cells in suspension are injected from a syringe through the load tube by attachment of the hub to the syringe. After cell loading, the load tube is retracted from the capsule and the resulting opening is sealed with glue.

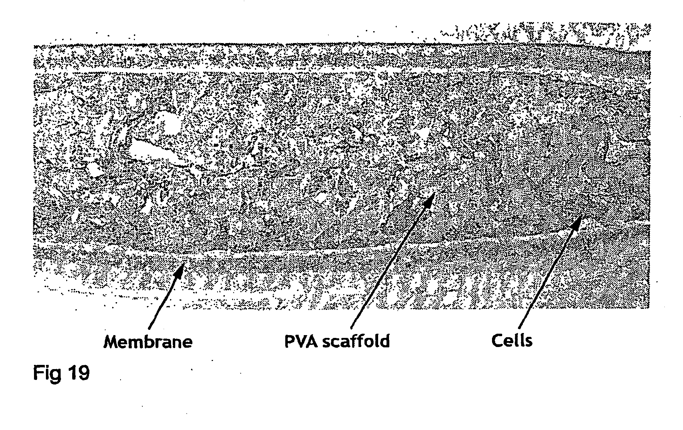

Figure 19 shows encapsulated, biologically active NGF-producing ARPE-19 cells. The capsule membrane, PVA foam scaffolding and the encapsulated cells are marked on the figure. As shown on the figure, the encapsulation device supports cell viability, allowing for sustained NGF production and secretion from the device.

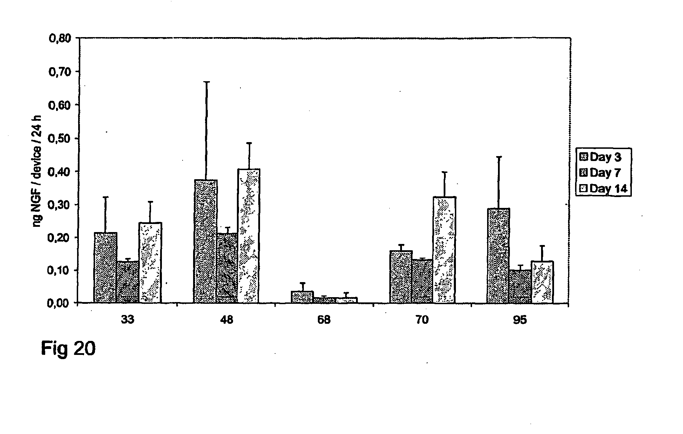

Figure 20 shows levels of NGF released from capsules filled with a series of NGF-producing ARPE-19 clones. While the levels of NGF-release diverge among clones and capsules due to natural variations, released NGF can be detected from all clones at all time points.

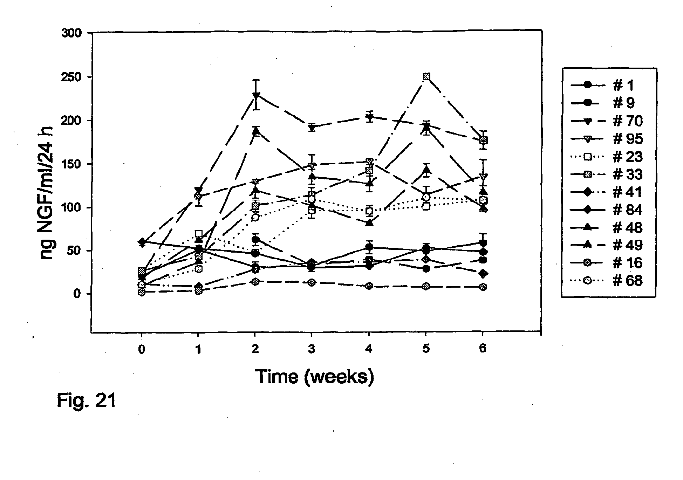

Figure 21 shows NGF release in HE-SFM from confluent cultures of twelve selected NGF producing clones transfected with the constructs pCIn.hNGF, pCIn.KhNGF and pNS1n.rINSintrA-hNGF (see figure 8). Conditioned media were collected every week for determination of NGF release by NGF ELISA analysis.

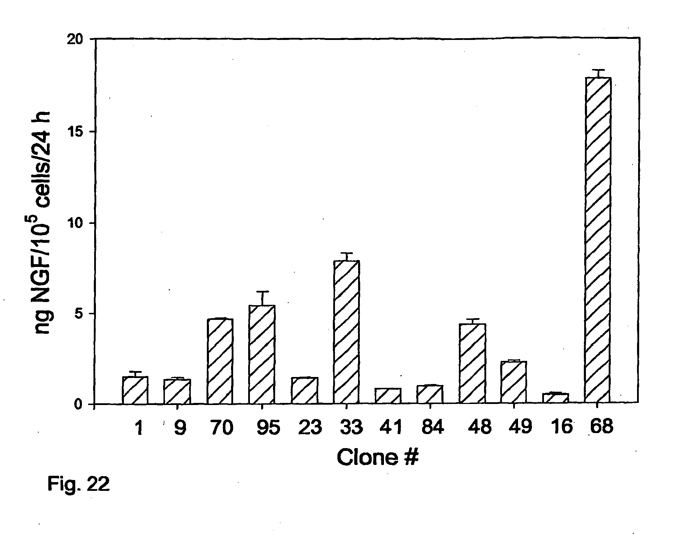

Figure 22 shows NGF release for the twelve clones 6 weeks after plating in HE-SFM, as determined by NGF ELISA.

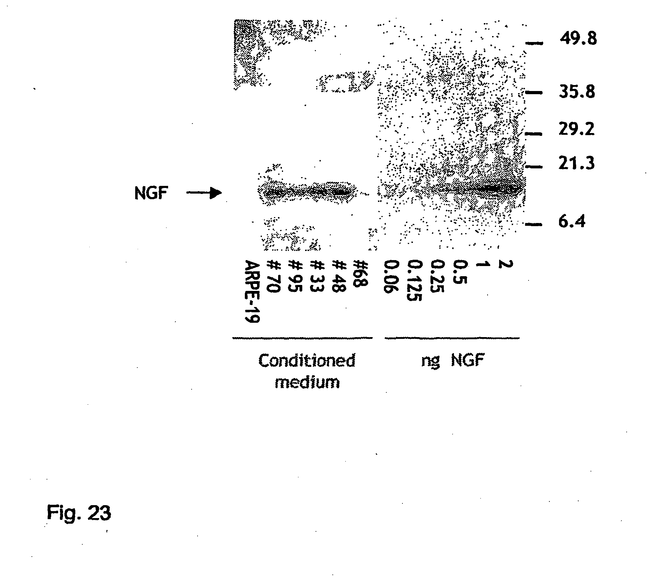

Figure 23 shows NGF Western blot showing release of mature NGF (13 kDa) in conditioned medium from four selected NGF producing ARPE-19 clones. Samples of diluted recombinant ß-hNGF (R&D systems #256-GF-100) show a band of the same molecular weight.

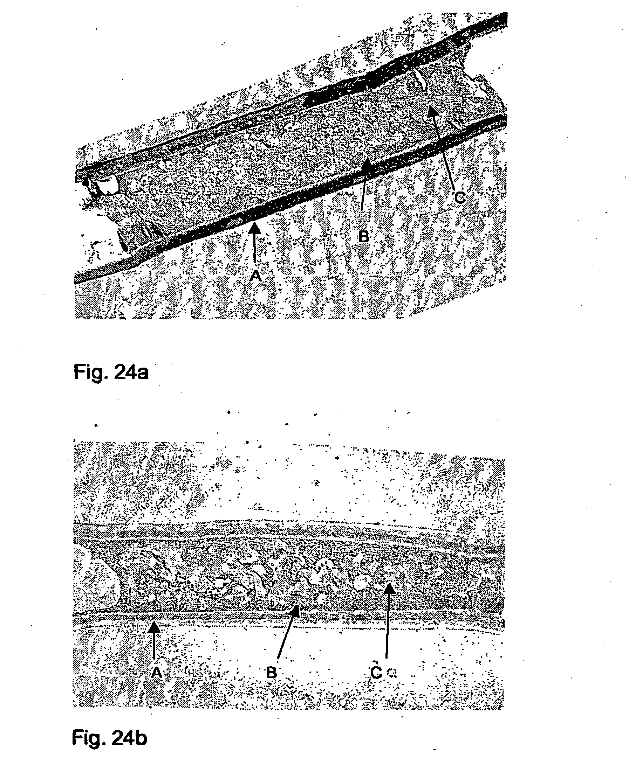

Figure 24 shows longitudinal sections of encapsulated ARPE-19 cells secreting biologically active NGF explanted after 8 weeks in the brain of a normal rat. The capsule membrane (A), PVA foam scaffolding (C) and the encapsulated cells (B) are marked on the figures. As shown on the figure, the encapsulation device supports cell viability, allowing for sustained NGF production and secretion from the device even after 8 weeks in vivo. The inner diameter of the devices is 700 µm +/- 50µm. Figure 24a: Clone #33. Figure 24b: Clone #95.

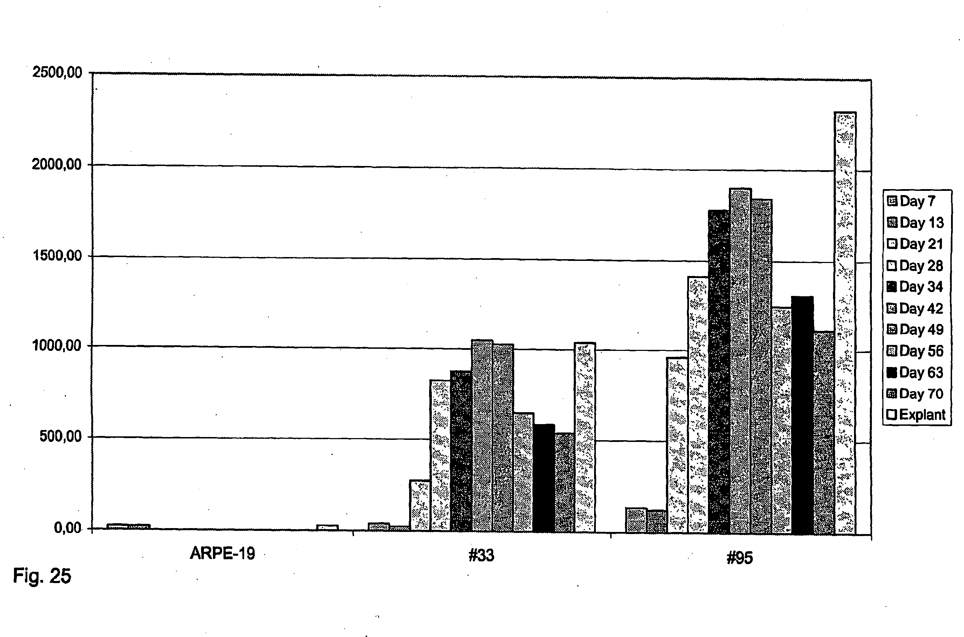

Figure 25 shows NGF secretion as measured by NGF Elisa from capsules before (day 7 and 13) and after 8 weeks in vivo in rat brains (explant). The NGF secretion from capsules maintained simultaneously in vitro is shown (days 21, 28, 35, 42, 49, 56, 63 and 70). The shown results are from two preferred cell lines (#33 and #95) as well as a non-transfected control (parental ARPE-19 cells). The vertical axis shows pg/NGF per capsule. As shown, NGF secretion in capsules which have been implanted in rat brains is comparable to capsules maintained in vitro and remains high after the 10 weeks period.

### Detailed description of the invention

### Definitions:

"Introns" refer in this work to those regions of DNA sequence that are transcribed along with the coding sequences (exons) but are then removed in the formation of the mature mRNA.

Introns may occur anywhere within a transcribed sequence, between coding sequences of a gene, within the coding sequence of a gene, and within the 5' untranslated region (5' UTR) (including the promoter region). Introns in the primary transcript are excised and the exon sequences are simultaneously and precisely ligated to form the mature mRNA. The junctions of introns and exons form the splice sites. The base sequence of an intron conservatively begins with GT and ends with AG in many higher eukaryotes.

As used herein "a biocompatible capsule" means that the capsule, upon implantation in a host mammal, does not elicit a detrimental host response sufficient to result in the rejection of the capsule or to render it inoperable, for example through degradation.

As used herein "an immunoisolatory capsule" means that the capsule upon implantation into a mammalian host minimizes the deleterious effects of the host's immune system on the cells within its core.

Biological activity refers to the biologically useful effects of a molecule on a specific cell. As used herein "a biologically active NGF" is one which is released or secreted from the cell in which it is made and exerts its effect on a separate target cell. Biological activity of the secreted NGF can be verified in the PC-12 assay described in the examples.

By a "mammalian promoter" is intended a promoter capable of functioning in a mammalian cell.

Down regulation of a promoter means the reduction in the expression of the product of transgene to a level, which may lead to a lack of significant biological activity of the transgene product after in vivo implantation. As used herein "a promoter not subject to down regulation" means a promoter, which, after in vivo implantation in a mammalian host, drives or continues to drive the expression of transgene at a level which is biologically active.

As used herein "long-term, stable expression of a biologically active NGF" means the continued production of a biologically active NGF at a level sufficient to maintain its useful biological activity for periods greater than one month, preferably greater than three months and most preferably greater than six months.

### "Sequence identity":

The determination of percent identity between two sequences can be accomplished using a mathematical algorithm. A preferred, non-limiting example of a mathematical algorithm utilized for the comparison of two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. Such an algorithm is incorporated into the BLASTN and BLASTP programs of Altschul, et al. (1990) J. Mol. Biol. 215:403-410.

In order to characterize the identity, subject sequences are aligned so that the highest order homology (match) is obtained. Based on these general principles the "percent identity" of two amino acid sequences may be determined using the BLASTP algorithm [Tatiana A. Tatusova, Thomas L. Madden: Blast 2 sequences - a new tool for comparing protein and nucleotide sequences; FEMS Microbiol. Lett. 1999 174 247-250], which is available from the National Center for Biotechnology Information (NCBI) web site (http://www.ncbi.nlm.nih.gov), and using the default settings suggested here (i.e. Matrix = Blosum62; Open gap = 11; Extension gap = 1; Penalties gap x_dropoff = 50; Expect = 10; Word size = 3; Filter on). The BLAST algorithm performs a two-step operation by first aligning two sequences based on the settings and then determining the % sequence identity in a range of overlap between two aligned sequences. In addition to % sequence identity, BLASTP also determines the % sequence similarity based on the settings.

In order to characterize the identity, subject sequences are aligned so that the highest order homology (match) is obtained. Based on these general principles, the "percent identity" of two nucleic acid sequences may be determined using the BLASTN algorithm [Tatiana A. Tatusova, Thomas L. Madden: Blast 2 sequences - a new tool for comparing protein and nucleotide sequences; FEMS Microbiol. Lett. 1999 174 247-250], which is available from the National Center for Biotechnology Information (NCBI) web site (http://www.ncbi.nlm.nih.gov), and using the default settings suggested here (i.e. Reward for a match = 1; Penalty for a match = -2; Strand option = both strands; Open gap = 5; Extension gap = 2; Penalties gap x_dropoff = 50; Expect = 10; Word size = 11; Filter on). The BLASTN algorithm determines the % sequence identity in a range of overlap between two aligned nucleotide sequences. For the purposes of the introns of the present invention the percent sequence identity is preferably calculated in a range of overlap of at least 10 nucleotides, the range being determined by BLASTN under default settings. More preferably the range of overlap is at least 20 nucleotides, more preferably at least 30, more preferably at least 40, more preferably at least 50, such as at least 75 nucleotides, for example at least 100 nucleotides, such as at least 200 nucleotides, for example at least 300 nucleotides, such as at least 400 nucleotides, for example at least 500 nucleotides, such as at least 600 nucleotides, for example at least 750 nucleotide, such as at least 1000 nucleotides.

The homology between two protein sequences may suitably be determined by means of computer programs known in the art such as GAP provided in the GCG program package [Needleman, S.B. and Wunsch, C.D., Journal of Molecular Biology, 1970 48 443-453]. Using GAP with the following settings for polypeptide sequence comparison: GAP creation penalty of 8 and GAP extension penalty of 2. Full length DNA sequence homology may suitably be determined by means of computer programs known in the art, such as GAP provided in the GCG program package [Needleman, S.B. and Wunsch C.D., Journal of Molecular Biology 1970 48 443-453]. Using GAP with the following settings for DNA sequence comparison: GAP creation penalty of 50 and GAP extension penalty of 3.

A preferred, non-limiting example of a mathematical algorithm utilized for the comparison of sequences is the algorithm of Myers and Miller, CABIOS (1989). Such an algorithm is incorporated into the ALIGN program (version 2.0) which is part of the FASTA sequence alignment software package (Pearson WR, Methods Mol Biol, 2000, 132:185-219). Align calculates sequence identities based on a global alignment. Align0 does not penalise to gaps in the end of the sequences. When utilizing the ALIGN og Align0 program for comparing amino acid sequences, a BLOSUM50 substitution matrix with gap opening/extension penalties of - 12/-2 is preferably used.

### Human cell lines

The present invention in general relates to human cell lines genetically modified to overexpress NGF or a biologically active variant thereof, wherein an intron is located in the transcript comprising the NGF coding sequence. In the broadest aspect the invention relates to any human cell culture or cell line, whether polyclonal or monoclonal. Monoclonal cell lines are more preferable, as they can be better characterised.

The invention relates to human cell lines, which have been immortalised by insertion of a heterologous immortalisation gene and to cell lines that are spontaneously immortal. In a preferred embodiment of the invention, the human cell line has not been immortalised with the insertion of a heterologous immortalisation gene. As the invention relates to cells which are particularly suited for cell transplantation, whether as naked cells or -preferably - as encapsulated cells, such immortalised cell lines are less preferred as there is an inherent risk that they start proliferating in an uncontrolled manner inside the human body and potentially form tumours if they carry known oncogenes.

Preferably, the cell line is a contact inhibited cell line. By a contact inhibited cell line is intended a cell line which when grown in 2-D cultures grows to confluency and then substantially stops dividing. This does not exclude the possibility that a limited number of cells escape the 2D layer. Contact inhibited cells may also be grown in 3D, e.g. inside a capsule. Also inside the capsules, the cells grow to confluency and then significantly slow down proliferation rate or completely stop dividing.

A particularly preferred type of cells include epithelial cells which are by their nature contact inhibited and which form stable monolayers in culture.

Even more preferred are retinal pigment epithelial cells (RPE cells). The source of RPE cells is by primary cell isolation from the mammalian retina.

Protocols for harvesting RPE cells are well-defined (U and Turner, 1988, Exp. Eye Res. 47:911-917; Lopez et al., 1989, Invest Ophthalmol. Vis. Sci. 30:586-588) and considered a routine methodology. In most of the published reports of RPE cell cotransplantation, cells are derived from the rat (Li and Turner, 1988; Lopez et al., 1989). According to the present invention RPE cells are derived from humans. In addition to isolated primary RPE cells, cultured human RPE cell lines may be used in the practice of the invention.

All normal diploid vertebrate cells have a limited capacity to proliferate, a phenomenon that has come to be known as the Hayflick limit or replicative senescence. In human fibroblasts, this limit occurs after 50-80 population doublings, after which the cells remain in a viable but non-dividing senescent state for many months. This contrasts to the behavior of most cancer cells, which have escaped from the controls limiting their proliferative capacity and are effectively immortal.

It is preferable that the cells are capable of undergoing a certain number of cell divisions so they can be genetically modified and expanded to produce enough cells for encapsulated cell therapy or transplantation therapy. Accordingly a preferred cell line is capable of undergoing at least 50 doublings, more preferably at least 60 doublings, more preferably at least 70 doublings, more preferably at least 80 doublings, more preferably at least 90 doublings, such as approximately 100 doublings.

For encapsulation, the cells need to be able to survive and maintain a functional NGF secretion at the low oxygen tension levels of the CNS. Preferably the cell line of the invention is capable of surviving at an oxygen tension below 5%, more preferably below 2%, more preferably below 1%. 1% oxygen tension corresponds to the oxygen level in the brain.

To be a platform cell line for an encapsulated cell based delivery system, the cell line should have as many of the following characteristics as possible: (1) The cells should be hardy under stringent conditions (the encapsulated cells should be functional in the vascular and avascular tissue cavities such as in the central nervous system intraparenchymally or within the ventricular or intrathecal fluid spaces or the eye, especially in the intra-ocular environment). (2) The cells should be able to be genetically modified to express NGF. (3) The cells should have a relatively long life span (the cells should produce sufficient progenies to be banked, characterised, engineered, safety tested and clinical lot manufactured). (4) The cells must be of human origin (which increases compatibility between the encapsulated cells and the host). (5) The cells should exhibit greater than 80% viability for a period of more than one month in vivo in the device (which ensures long-term delivery). (6) The encapsulated cells should deliver an efficacious quantity of NGF (which ensures effectiveness of the treatment). (7) When encapsulated, the cells should not cause a significant host immune reaction (which ensures the longevity of the graft). (8) The cells should be non-tumourigenic (to provide added safety to the host, in case of device leakage).

In a screening and characterisation of several cell lines it has been found that the ARPE-19 cell line (Dunn et al., 62 Exp. Eye Res. 155-69 (1996), Dunn et al., 39 Invest. Ophthalmol. Vis. Sci. 2744-9 (1998), Finnemann et al., 94 Proc. Natl. Acad. Sci. USA 12932-7 (1997), Handa et al., 66 Exp. Eye. 411-9 (1998), Holtkamp et al., 112 Clin. Exp. Immunol. 34-43 (1998), Maidji et al., 70 J. Virol. 8402-10 (1996)) has all of the characteristics of a successful platform cell for an encapsulated cell-based delivery system (US 6,361,771, Tao et al). The ARPE-19 cell line was superior to the other cell lines tested.

The ARPE-19 cell line is available from the American Type Culture Collection (ATCC Number CRL-2302). The ARPE-19 cell line is derived from cultures of normal retinal pigmented epithelial (RPE) cells and express the retinal pigmentary epithelial cell-specific markers CRALBP and RPE-65. ARPE-19 cells form stable monolayers, which exhibit morphological and functional polarity.

ARPE-19 cells may be cultured in Complete Growth Medium, the serum-containing medium recommended by the cell depositor. Complete Growth Medium is either a 1:1 mixture of Dulbecco's modified Eagle's medium and Ham's F12 medium with 3 mM L-glutamine, 90%; foetal bovine serum, 10% or a 1:1 mixture of Dulbecco's modified Eagle's medium and Ham's F12 medium with HEPES buffer containing 10% fetal bovine serum, 56 mM final concentration sodium bicarbonate and 2 mM L-glutamine. The cells are preferably incubated at 37°C. in 5% CO₂. The cells are typically plated and grown in Falcon tissue culture treated 6 or 12-well plates or T25 or T75 flasks.

For subculturing, medium is removed, and the ARPE-19 cells are preferably rinsed with 0.05% trypsin, 0.02% EDTA solution, and the trypsin is removed. One to two ml of additional trypsin solution is added. The culture is incubated at room temperature (or at 37°C.) until the ARPE-19 cells detach. A subcultivation ratio of 1:3 to 1:5 is recommended.

The hardiness of candidate cell lines for encapsulated cell therapy can be tested using the following three-step screen. (a) Cell viability screen (The cells may be evaluated under stressed conditions using artificial aqueous humor (aAH) medium or artificial cerebral spinal fluid (aCSF) medium). (b) In vitro ECM screen (The cells may be evaluated in an in vitro extracellular matrix (ECM) screen). (c) In vivo device viability screen (The encapsulated cells may be evaluated in an in vivo membrane screen). A detailed description of the screens and results with several human and non human cell lines are found in US 6,361,771.

In the three types of screens described above, ARPE-19 cells has proven superior to a number of other cell lines tested (see US 6,361,771). In particular it should be noted that BHK cells which have been used in the prior art to secrete NGF did not pass the cell viability screen.

In another embodiment the cell line is selected from the group consisting of: human immortalised fibroblast cell lines, human immortalised mesencymal stem cell lines, human immortalised astrocyte cell lines, human immortalised mesencephalic cell lines, and human immortalised endothelial cell lines, preferably immortalised with SV40T, vmyc, or the catalytic subunit of telomerase (TERT).

Another type of preferred human cells according to the invention are immortalised human astrocyte cell lines. These cell lines may also have the properties required for the uses according to the present invention.

The method for generating an immortalised human astrocyte cell lines has previously been described (Price TN, Burke JF, Mayne LV. A novel human astrocyte cell line (A735) with astrocyte-specific neurotransmitter function. In Vitro Cell Dev Biol Anim. 1999 May;35(5):279-88.). This protocol may be used to generate astrocyte cell lines.

The following three modifications of that protocol are preferably made to generate additional human astrocyte cell lines.

Human foetal brain tissue dissected from 5-12 weeks old foetuses may be used instead of 12-16 weeks old tissue.
The immortalisation gene *v-myc* may be used instead of the *SV40 T antigen*.
Retroviral gene transfer may be used instead of transfection with plasmids by conventional plasmid transfection techniques (including calcium phosphate precipitation).

### Long term stability

Preferably the cell lines of the present invention are capable of surviving for extended periods (several months and up to one year or more) when transplanted as encapsulated cells in vivo. The cell lines are preferably also capable of maintaining a secretion of bioactive NGF at a level sufficient to ensure the therapeutic efficacy for a period greater than one month, preferably greater than three months, more preferably greater than six months. It is also preferable that the cells are capable of maintaining a relevant secretion of bioactive NGF after encapsulation for at least one month, more preferably at least three months, more preferably at least six months. As shown in Example 14, NGF secretion and viability remained high after two months implantation in the brain of normal rats.

The level of secretion preferably is at least 0.5 ng biologically active NGF per 10⁵ cells per 24 hours is at least 0.5 ng, more preferably at least 0.75 ng, more preferably at least 1 ng, more preferably at least 2 ng, more preferably at least 2.5 ng, more preferably at least 5 ng, more preferably at least 7.5 ng, more preferably at least 10 ng, more preferably at least 15 ng, more preferably at least 20 ng, more preferably at least 25 ng, more preferably at least 50 ng. As evidenced by the appended examples, several human contact inhibited clones secreting in excess of 5 and even 10 ng/10⁵cells/24 hours have been established with different expression expression constructs with different types of introns.

When measured on a device level, the device (comprising encapsulated cells) is preferably capable of secreting in excess of 0.1 ng biologically active NGF per 24 hours. More preferably, the amount of biologically active NGF per 24 hours per device is at least 0.5 ng, more preferably at least 0.75 ng, more preferably at least 1 ng, more preferably at least 2 ng, more preferably at least 2.5 ng, more preferably at least 5 ng, more preferably at least 7.5 ng, more preferably at least 10 ng, more preferably at least 15 ng, more preferably at least 20 ng, more preferably at least 25 ng.

### Introns

From an analysis of the human genome at GenBank it can be derived that the smallest known intron is 4 bp and the longest known intron is 1,022,077 bp. Based on this knowledge, it is contemplated that the length of the intron used in the context of the present invention can be varied considerably. Except from the upper known limit given by Genbank, it is difficult to give any upper limit for the length of an intron, which is functional in the context of the present invention. The upper limit is more determined by the vector used as is well known in the art. In the broadest possible context there is no upper limit for the length of the intron, as long as it can be successfully cloned into the expression vector. For practical reasons one of skill in the art would select an intron, which is less than 100,000 bp long, more preferably less than 10,000 bp long.

The only parts of an intron that are really highly conserved are the sequences immediately within the intron. This identifies the formula of a generic intron as:
GT.....AG

The ends are named proceeding from left to right along the intron, that is as the left (or 5') and right (or 3') splicing sites. Sometimes they are referred to as the donor and acceptor sites. The bases immediately adjacent the donor and acceptor sites are less conserved. The frequency of different bases at specific positions relative to the splicing sites follow the following percentages (Lewin B, Genes V, Oxford University Press, Oxford, 1994, page 914):

| **Exon** | | Intron | | | | | | | | | **Exon** |
|---|---|---|---|---|---|---|---|---|---|---|---|
| **A** | **G** | G | T | A | A | G | T----- C | | A | G | **N** |
| **64%** | **73%** | 100% | 100% | 62% | 68% | 84% | 63% | 65% | 100% | 100% | |

The sequence within these splicing sites can for every single intron be varied considerably by addition, deletion or substitution of bases. In a preferred embodiment of the invention the intron comprises a nucleotide sequence which is derived from a naturally occurring intron, and which has at least 50% sequence identity to said naturally occurring intron. More preferably, the intron has at least 60% sequence identity to said naturally occurring intron, more preferably at least 70%, more preferably at least 75%, more preferably at least 80%, more preferably at least 85%, more preferably at least 90%, more preferably at least 95%, more preferably at least 98%. The higher % sequence identities are preferred as less work is required to assemble to expression construct, and as the possibility of changing the function of the intron increases with the number of differences between a naturally occurring intron and a variant thereof.

In a preferred embodiment the intron is shorter such as less than 10,000 bp, which will considerably ease the cloning. Accordingly the intron may be less than 9,000 bp long, preferably less than 8,000, more preferably less than 7,000, more preferably less than 6,000, more preferably less than 5,000, more preferably less than 4,500, more preferably less than 4,000, more preferably less than 3,500, more preferably less than 3,000, more preferably less than 2,500, more preferably less than 2,000, more preferably less than 1,500, more preferably less than 1,000, such as less than 750, for example less than 500, such as less than 250, for example less than 200.

Similarly, it is expected that the intron should have a certain length to be properly spliced out from the transcript before translation. Therefore preferably the intron is more than 4 bp long, such ad more than 10 bp long, for example more than 20 bp long, preferably more than 50 bp long, more preferably more than 75 bp long.

An intron may be from 4 bp to 1 mio bp long, more preferably from 10-10,000 bp, more preferably from 20-2000 bp, for example from 50-1500 bp, such as preferably from 75-200 bp, for example preferably from 500-1500 bp. The introns exemplified in the examples lie in the range from 100 to 1000 bp.

The origin of the intron may be any. It may even be a synthetic intron as long as it functions as an intron. As the present invention concerns human cell lines, it is preferable to use intron from a species that is as closely related to human beings as possible. Therefore, preferably the intron is of eukaryotic origin. More preferably the intron is of mammalian origin. For example the intron may be of rodent origin or of primate origin. Still more preferably, the intron is of human origin.

It is preferred to have the intron located in the 5' UTR or in the part of the coding sequence closest to the start codon, i.e. the first part of the coding sequence. Cloning is easier when the intron is placed in the 5' UTR of the transcript. It is contemplated by the present inventors that a similar secretion enhancing effect may be obtained by cloning an intron into the sequence coding for NGF. In the case of cloning inside the coding sequence, the intron is preferably placed in the part of the coding sequence closest to the start codon, i.e. the first part of the coding sequence.

In a preferred embodiment the intron is derived from a first intron. A first intron is the intron located closest to the transcription start site in the gene from which it is derived. While first introns are preferred, it is to be understood that any intron such as a second, third, fourth, fifth, or sixth intron may also be used. A first intron of a particular gene is sometimes referred to as intron A.

It is expected that it is sufficient to include one intron in the expression constructs in the human cell lines of the present invention. Including further introns is of course possible, and is also contemplated by the present inventors. In principle there is no upper limit to the number of introns inserted into the transcript but for practical reasons, the skilled practitioner would choose to keep the number as low as possible to keep the length of the expression construct within practical limits. The number of introns may be two, three, four, five or even higher.

One particularly preferred intron is derived from insulin. Preferably rodent insulin II, more preferably rat preproinsulin II intron A (GenBank acc. # J00748). The nucleotide sequence of this intron is set forth in SEQ ID No 22 (bases no 747 to 865, shown in lowercase in Figure 14). The rat insulin II intron A is 119 bp. Cloning of this intron is described in the examples.

The sequence lying between the splice sites of rat insulin II intron A can be varied. Preferably the intron comprises a sequence, which is derived from rat insulin II A intron and therefore has at least 50% sequence identity to the sequence set forth above. More preferably, the intron comprises a sequence having at least 60% sequence identity to said sequence, more preferably at least 70%, more preferably at least 75%, more preferably at least 80%, more preferably at least 85%, more preferably at least 90%, more preferably at least 95%, more preferably at least 98%.

Another preferred intron is the chimeric intron included in the pCI expression vector available from Promega Corp, Madison Wisconsin, USA (Catalogue no.: E1731). This intron is composed of the 5'-donor site from the first intron of the human b-globin gene and the branch and 3'-acceptor site from the intron that is between the leader and the body of an immunoglobulin gene heavy chain variable region (Bothwell et al, 1981, Cell 24:625). The sequences of the donor and acceptor sites along with the branchpoint site have been changed to match the consensus sequences for splicing (Senapathy et al, 1990, Meth. Enzymol. 183:252). The pCI expression vector is available from Promega Corp. The length of the intron is 113 bp and the sequence of the intron is set forth in SEQ ID No 14 (bases no 890-1022, shown in lowercase in Figure 10) and in SEQ ID No 16 (bases no 890-1022, shown in lowercase in Figure 11)

The sequence lying between the splice sites of the "pCI intron" can be varied. Preferably the intron comprises a sequence, which is derived from the "pCI intron", which derived sequence has at least 50% sequence identity to the sequence set forth above. More preferably, the intron comprises a sequence having at least 60% sequence identity to said "pCI intron", more preferably at least 70%, more preferably at least 75%, more preferably at least 80%, more preferably at least 85%, more preferably at least 90%, more preferably at least 95%, more preferably at least 98%.

A further preferred intron is the ubiquitin promoter intron, preferably the human ubiquitin C promoter intron (Johansen et al. 1990, FEBS Lett. 267, 289-294). The UbiC intron is 811 bp long (GenBank acc # D63791). The nucleotide sequence of the intron is set forth in SEQ ID No 20 (bases No. 736 to 1547, shown in lowercase in Figure 13). The ubiquitin C promoter intron is available from the pUbi1z expression vector described in Johansen et al 2003, J Gene Medicine, 5:1080-1089.

The sequence lying between the splice sites of said ubiqutin intron can be varied. Preferably the intron comprises a sequence, which is derived from rat ubiquitin intron, and which has at least 50% sequence identity to the sequence set forth above. More preferably, the intron comprises a sequence which has at least 60% sequence identity to the sequence of said ubiquitin intron, more preferably at least 70%, more preferably at least 75%, more preferably at least 80%, more preferably at least 85%, more preferably at least 90%, more preferably at least 95%, more preferably at least 98%.

Another preferred intron is the EF-1alpha intron A (bases no. 609..1551 of Genbank accession number: J04617 J04616 Human elongation factor EF-1alpha gene, complete cds). This intron is part of the promoter sequence and is 943 bp long. A nucleotide sequence of the EF-1alpha promoter including the intron is set forth in SEQ ID No 18 (Figure 12). The intron is shown in lowercase (bases no 232-1171).

The sequence lying between the splice sites of said EF-1alpha intron A can be varied. Preferably the intron comprises a sequence which is derived from the EF-1alpha intron A, and which has at least 50% sequence identity to the sequence set forth above. More preferably, the intron comprises a sequence which has at least 60% sequence identity to said EF-1alpha intron A, more preferably at least 70%, more preferably at least 75%, more preferably at least 80%, more preferably at least 85%, more preferably at least 90%, more preferably at least 95%, more preferably at least 98%.

Preferably the intron is selected from the group consisting of
the pCI intron, the ratINS-intrA, and the Ubiquitin intron; and
a sequence variant having at least 80 % sequence identity to the sequence of any of said introns.

More preferably the intron is selected from the group consisting of
the pCI intron, and the ratINS-intrA; and
a sequence variant having at least 80 % sequence identity to the sequence of any of said introns.

### Deposit

Two preferred Homo sapiens cell lines have been deposited by NsGene A/S, Baltorpvej 154, DK-2750 Ballerup, Denmark, under the Budapest Treaty with DSMZ, Mascheroder Weg 1 b, D-38124 Braunschweig, Germany on December 15, 2004 under accession number: DSM ACC2706 and DSM ACC2707. Both deposited cell lines are ARPE-19 clones transfected with pCln.hNGF as defined herein. The deposited cell lines are identified in the examples as #33 (NGC-0233) and #95 (NGC-0295), respectively.

### Expression vectors

Construction of vectors for recombinant expression of NGF for use in the invention may be accomplished using conventional techniques which do not require detailed explanation to one of ordinary skill in the art. For review, however, those of ordinary skill may wish to consult Maniatis et al., in Molecular Cloning: A Laboratory Manual, Cold Spring Harbor Laboratory, (NY 1982).

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (*e*.*g*., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector' can be used interchangeably as the plasmid is the most commonly used form of vector. However, the invention is intended to include such other forms of expression vectors, such as viral vectors (*e*.*g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e*.*g*., in an *in vitro* transcription/translation system or in a host cell when the vector is introduced into the host cell).

The term "regulatory sequence" is intended to includes promoters, enhancers and other expression control elements (*e*.*g*., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e*.*g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce NGF and NGF mutants and variants encoded by nucleic acids as described herein.

In a preferred embodiment, the expression vector is a mammalian plasmid expression vector. Examples of mammalian plasmid expression vectors include pCDM8 (Seed, 1987. Nature 329: 840), pCI (Promega Inc), pSI (Promega), pNS (Figure 4), pUbi1z (Figure 5), and pMT2PC (Kaufman, et al., 1987. EMBO J. 6: 187-195). When used in mammalian cells, the expression vector's control functions are often provided by viral regulatory elements. For other suitable expression systems for eukaryotic cells see, *e*.*g*., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

Briefly, construction of recombinant expression vectors employs standard ligation techniques. For analysis to confirm correct sequences in vectors constructed, the genes are sequences using, for example, the method of Messing, et al., (Nucleic Acids Res., 9: 309-, 1981), the method of Maxam, et al., (Methods in Enzymology, 65: 499, 1980), or other suitable methods which will be known to those skilled in the art.

Size separation of cleaved fragments is performed using conventional gel electrophoresis as described, for example, by Maniatis, et al., (Molecular Cloning, pp. 133-134,1982).

Expression of a gene is controlled at the transcription, translation or post-translation levels. Transcription initiation is an early and critical event in gene expression. This depends on the promoter and enhancer sequences and is influenced by specific cellular factors that interact with these sequences. The transcriptional unit of many genes consists of the promoter and in some cases enhancer or regulator elements (Banerji et al., Cell 27: 299 (1981); Corden et al., Science 209: 1406 (1980); and Breathnach and Chambon, Ann. Rev. Biochem. 50: 349 (1981)). For retroviruses, control elements involved in the replication of the retroviral genome reside in the long terminal repeat (LTR) (Weiss et al., eds., The molecular biology of tumor viruses: RNA tumor viruses, Cold Spring Harbor Laboratory, (NY 1982)). Moloney murine leukemia virus (MLV) and Rous sarcoma virus (RSV) LTRs contain promoter and enhancer sequences (Jolly et al., Nucleic Acids Res. 11: 1855 (1983); Capecchi et al., In: Enhancer and eukaryotic gene expression, Gulzman and Shenk, eds., pp. 101-102, Cold Spring Harbor Laboratories (NY 1991). Other potent promoters include those derived from cytomegalovirus (CMV) and other wild-type viral promoters and the UbiC promoter derived from human ubiquitin C (WO 98/32869).

Promoter and enhancer regions of a number of non-viral promoters have also been described (Schmidt et al., Nature 314: 285 (1985); Rossi and deCrombrugghe, Proc. Natl. Acad. Sci. USA 84: 5590-5594 (1987)). Methods for maintaining and increasing expression of transgenes in quiescent cells include the use of promoters including collagen type I (1 and 2) (Prockop and Kivirikko, N. Eng. J. Med. 311: 376 (1984) ; Smith and Niles, Biochem. 19: 1820 (1980) ; de Wet et al., J. Biol. Chem., 258: 14385 (1983)), SV40 and LTR promoters.

According to one embodiment of the invention, the promoter is a constitutive promoter selected from the group consisting of: ubiquitin promoter, CMV promoter, JeT promoter (US 6,555,674), SV40 promoter, Mt1 promoter, and Elongation Factor 1 alpha promoter (EF-1 alpha). A particularly preferred promoter is one which is not subject to down regulation in vivo.

Examples of inducible/repressible promoters include: Tet-On, Tet-Off, Rapamycin-inducible promoter, Mx1.

In addition to using viral and non-viral promoters to drive transgene expression, an enhancer sequence may be used to increase the level of transgene expression. Enhancers can increase the transcriptional activity not only of their native gene but also of some foreign genes (Armelor, Proc. Natl. Acad. Sci. USA 70: 2702 (1973)). For example, in the present invention collagen enhancer sequences may be used with the collagen promoter 2 (I) to increase transgene expression. In addition, the enhancer element found in SV40 viruses may be used to increase transgene expression. This enhancer sequence consists of a 72 base pair repeat as described by Gruss et al., Proc. Natl. Acad. Sci. USA 78: 943 (1981); Benoist and Chambon, Nature 290: 304 (1981), and Fromm and Berg, J. Mol. Appl. Genetics, 1 : 457 (1982). This repeat sequence can increase the transcription of many different viral and cellular genes when it is present in series with various promoters (Moreau et al., Nucleic Acids Res. 9: 6047 (1981).

Further expression enhancing sequences include but are not limited to Kozak consensus sequence, Woodchuck hepatitis virus post-transcriptional regulation element, WPRE, SP163 enhancer, CMV enhancer, non-translated 5' or 3' regions from the tau, TH or APP genes, and Chicken [beta]-globin insulator or other insulators. Preferable enhancing elements include Kozak consensus sequence, WPRE and beta-globin insulator.

Viruses useful as gene transfer vectors include papovavirus, adenovirus, vaccinia virus, adeno-associated virus, herpesvirus, and retroviruses. Suitable retroviruses include the group consisting of HIV, SIV, FIV, EIAV, MoMLV.

A special and preferred type of retroviruses includes the lentiviruses which can transduce a cell and integrate into its genome without cell division. A lentivirus particle can be produced from a lentiviral vector comprising a 5' lentiviral LTR, a tRNA binding site, a packaging signal, a promoter operably linked to a polynucleotide signal encoding NGF, an origin of second strand DNA synthesis and a 3' lentiviral LTR.

Retroviral vectors are the vectors most commonly used in human clinical trials, since they carry 7-8 kb and since they have the ability to infect cells and have their genetic material stably integrated into the host cell with high efficiency. See, e.g., WO 95/30761; WO 95/24929. Oncovirinae require at least one round of target cell proliferation for transfer and integration of exogenous nucleic acid sequences into the patient Retroviral vectors integrate randomly into the cell's genome.

Three classes of retroviral particles have been described; ecotropic, which can infect murine cells efficiently, and amphotropic, which can infect cells of many species. The third class includes xenotropic retrovirus, which can infect cells of another species than the species which produced the virus. Their ability to integrate only into the genome of dividing cells has made retroviruses attractive for marking cell lineages in developmental studies and for delivering therapeutic or suicide genes to cancers or tumours.

For use in cells in human patients, the retroviral vectors must be replication defective. This prevents further generation of infectious retroviral particles in the target tissue-instead the replication defective vector becomes a "captive" transgene stable incorporated into the target cell genome. Typically in replication defective vectors, the gag, env, and pol genes have been deleted (along with most of the rest of the viral genome). Heterologous DNA is inserted in place of the deleted viral genes. The heterologous genes may be under the control of the endogenous heterologous promoter, another heterologous promoter active in the target cell, or the retroviral 5' LTR (the viral LTR is active in diverse tissues). Typically, retroviral vectors have a transgene capacity of about 7-8 kb.

Replication defective retroviral vectors require provision of the viral proteins necessary for replication and assembly in trans, from, e.g., engineered packaging cell lines. It is important that the packaging cells do not release replication competent virus and/or helper virus. This has been achieved by expressing viral proteins from RNAs lacking the ψ signal, and expressing the gag/pol genes and the env gene from separate transcriptional units. In addition, in some 2. and 3. generation retroviruses, the 5' LTR's have been replaced with non-viral promoters controlling the expression of these genes, and the 3' promoter has been minimised to contain only the proximal promoter. These designs minimize the possibility of recombination leading to production of replication competent vectors, or helper viruses. See, e.g., U.S. Pat. No. 4,861,719.

### Gene activation

Gene targeting or gene activation can be used to replace the existing regulatory region of the cells' NGF gene with a regulatory sequence comprising a promoter and an intron located in the region 3' to the promoter as herein defined. Such regulatory sequences may be comprised of promoters, enhancers, scaffold-attachment regions, negative regulatory elements, transcriptional initiation sites, and regulatory protein binding sites or combinations of said sequences.

The targeting event may be a simple insertion of the regulatory sequence, placing the NGF gene under the control of the new regulatory sequence, e.g., inserting a new promoter and intron both upstream of the cell's NGF gene. Alternatively, the targeting event may replace an existing element; for example, the cell's native NGF promoter can be replaced by a strong constitutive promoter and an intron upstream of the NGF gene. Here, the naturally occurring sequences are deleted and new sequences are added. In all cases, the identification of the targeting event may be facilitated by the use of one or more selectable marker genes that are contiguous with the targeting DNA, allowing for the selection of cells in which the exogenous DNA has integrated into the host cell genome. The identification of the targeting event may also be facilitated by the use of one or more marker genes exhibiting the property of negative selection, such that the negatively selectable marker is linked to the exogenous DNA, but configured such that the negatively selectable marker flanks the targeting sequence, and such that a correct homologous recombination event with sequences in the host cell genome does not result in the stable integration of the negatively selectable marker. Markers useful for this purpose include the Herpes Simplex Virus thymidine kinase (TK) gene or the bacterial xanthine-guanine phosphoribosyl-transferase (gpt) gene.

The gene targeting or gene activation techniques which can be used in accordance with this aspect of the invention are more particularly described in U. S. Patent No. 5,272, 071 to Chappel; U. S. Patent No. 5,578, 461 to Sherwin et al.; W0 93/09222 by Selden et al.; and W0 91/06667 by Skoultchi et al.

### Nerve Growth Factors

Table 1: Clustal W (1.82) multiple sequence alignment of preproNGF from human (SEQ ID No 2), chimpanzee (SwissProt Q9N2F1; SEQ ID No 5), pig (incomplete sequence, SwissProt Q29074; SEQ ID No 6), mouse (SEQ ID No 3), and rat (SEQ ID No 4). The mature, bioactive part is shown in bold and may be 120 or 118 amino acids long. The two C-terminal amino acids can be removed without affecting biological activity.

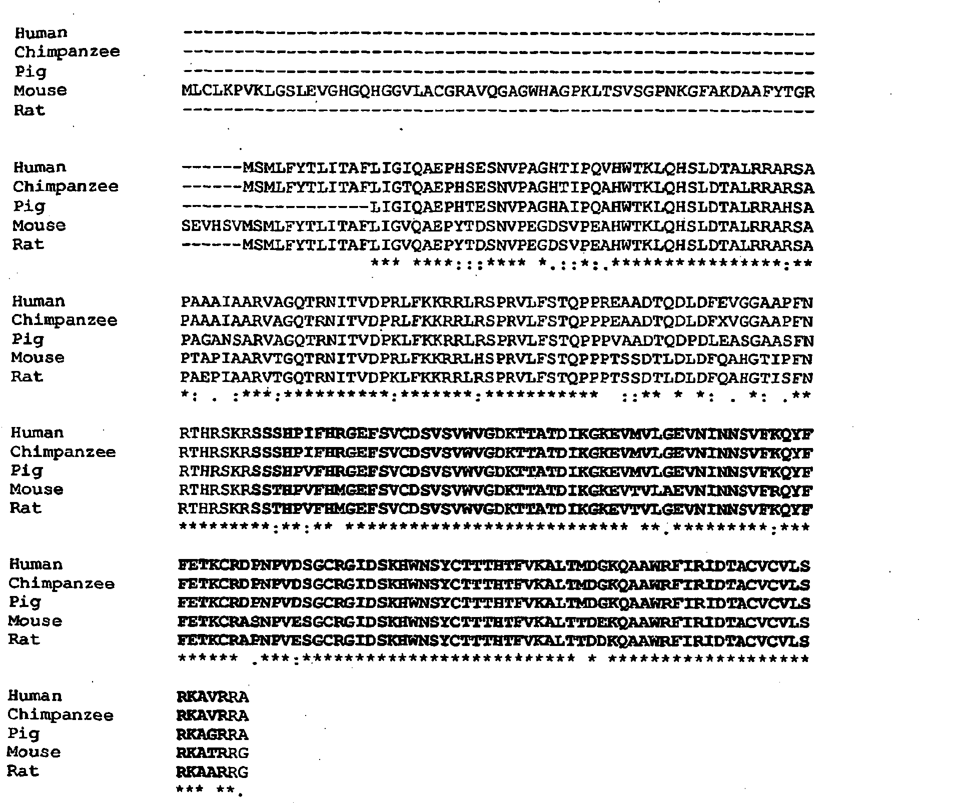

"NGF" refers to nerve growth factor from any species, including murine, bovine, ovine, porcine, equine, avian, and preferably human, in native sequence or in variant form, and from any source. A mouse NGF sequence is known, which has an N-terminal corresponding to the N-terminal of the other NGF's shown in the alignment This mouse NGF has accession no P01139 in SwissProt.

Sequenes preferred for human use comprise a sequence resulting in the secretion from the cell of a human native-sequence, mature NGF, more preferably a 120 amino acid sequence, and even more preferably a 118 amino acid sequence form. The preferred amino acid sequences for human pre-pro-NGF and human mature NGF are provided by U.S. Pat. No. 5,288,622. The DNA sequence coding for human pre-pro-NGF is set forth in SEQ ID No 1 (Figure 9) together with the amino acid sequence of the translated peptide (SEQ ID No 2, Figure 9).

NGF is synthesised in eukaryotic cells as a pre-pro-peptide, which is processed and secreted. In eukaryotic cells NGF is secreted via the endoplasmatic reticulum, to which it is directed by the signal peptide (also referred to at the pre-peptide). NGF is folded and three intramolecular cysteine bridges are generated (Cys58-108, Cys 68-110, and Cys 15-80) (McDonald et al 1991, Nature 354: 411-747). The folded and dimerised NGF can be secreted either as mature NGF (120 or 118 amino acids) or with the prodomain still attached to the mature bioactive protein. The prodomain may be cleaved subsequently.

There are reports in literature that the pro-sequence of NGF facilitates expression and subsequent folding of NGF (Rattenholl et al 2001, Eur J Biochem 268:3296-3303). There are also reports that the absence of the pro-peptide results in reduced or absent expression of NGF (Suter et al, 1991, The EMBO Journal, 10:2395-2400). In the pro-domain, Asn-53 and Asn-8 are glycosylated. It has been shown that mutation of these to Gln results in reduced expression (Heymach et al 1996, J Biol Chem, 271:25430-25437). It has also been shown that mutation of the furin cleavage site adjacent the mature peptide (-1 to -4 in SEQ ID No. 2) results in secretion in mammalian cells of pro-NGF (Heymach op cit).

In the present invention the use of a NGF coding sequence coding for a functional pro-domain of NGF linked to mature NGF is preferred to ensure efficient biosynthesis and correct folding of the secreted mature NGF. By a functional pro-domain is meant a prodomain, with Asn at positions -8 and -53 in the propeptide, and with a functional furinsite located immediately upstream from the mature NGF. Preferably, the functional propeptide has at least 60% sequence identity to the prodomain of SEQ ID No 2 (AA₋₁₀₂-AA₋₁), more preferably at least 70%, more preferably at least 75%, more preferably at least 80%, more preferably at least 90%, more preferably at least 95%, more preferably at least 98%. In a preferred embodiment of the invention, the functional prodomain comprises residues marked in the Clustal alignment of Table 1 as fully conserved (*), more preferably the functional prodomain comprises residues marked in Table 1 as conserved (*) and semiconserved (:), more preferably the functional prodomain comprises residues marked in Table 1 as conserved (*), semiconseved (:), and as less conserved (.). Even more preferably the prodomain is identical to the human NGF prodomain.

The mature 120 amino acid form is a preferred form in the homodimer form (i.e., 120/120), which will form spontaneously when the mature protein is secreted from a human cell according to the invention. Even more preferred is the mature 118 form, which will also form spontaneously as a homodimer (i.e., 118/118). There is no difference in bioactivity between 118 and 120 NGF.

The primary structure of a mammalian NGF (mouse NGF) was first elucidated by Angeletti and Bradshaw, Proc. Natl. Acad. Aci. USA 68:2417 (1971). The primary structure of its precursor, pre-pro-NGF, has been deduced from the nucleotide sequence of the mouse NGF cDNA (Scott et al. Nature 302:538 (1983); Ullrich et al. Nature 303:821 (1983)). The homologous human NGF (hNGF) gene has also been identified (Ullrich, Symp. on Quan. Biol., Cold Spring Harbor 48:435 (1983); U.S. Pat. No. 5,288,622, issued Feb. 22, 1994). Its homology to the mouse NGF is about 90% and 87%, on the amino acid and nucleotide sequence levels, respectively.

Preferably the sequence coding for NGF comprises a sequence selected from the group consisting of:
human betaNGF (preproNGF) coding sequence (SEQ ID No 1);
rat, mouse, or pig betaNGF (preproNGF) coding sequence;
chimpanzee betaNGF (preproNGF) coding sequence;
a sequence comprising a sequence coding for mature NGF of human (SEQ ID No 2), rat (SEQ ID No 4), mouse (SEQ ID No 3), pig (SEQ ID No 6) or chimpanzee (SEQ ID No 5) origin; and
a sequence coding for bioactive NGF variant comprising a mature sequence having at least 50% sequence identity to the mature part of SEQ ID No 2.

By a bioactive NGF is intended a NGF variant which is bioactive in the PC12 assay described in the examples. By substantially the same response in the PC12 assay is intended that the number of neurite bearing cells is at least 50% of the number obtained with wildtype NGF, more preferably at least 60%, more preferably at least 70%, more preferably at least 75%, more preferably at least 80%, more preferably at least 85%, more preferably at least 90%. The PC12 assay may also return the percentage improvement in survival over a control treatment. Substantially the same response in this context means an activity resulting in at least 50% of the improvement of wildtype NGF, more preferably at least 60%, more preferably at least 70%, more preferably at least 75%, more preferably at least 80%, more preferably at least 90%. The biological activity of a variant NGF may of course also be higher than that of the wildtype NGF.

More preferably said bioactive NGF variant has at least 60% sequence identity to the mature part of SEQ ID No 2, more preferably at least 70%, more preferably at least 75%, more preferably at least 80%, more preferably at least 90%, more preferably at least 95%, more preferably at least 98%.

In a particularly preferred embodiment the NGF coding sequence comprises the sequence coding for mature human beta NGF coding sequence.

In another preferred embodiment the NGF coding sequence comprises a sequence coding for mature human NGF (mature part of SEQ ID No 2).

Other modifications may be done to the coding sequence without departing from the inventive concept of the present invention. Thus it is contemplated that the signal peptide can be replaced with another, preferably mammalian, more preferably human signal peptide to enhance secretion further.

Similarly, it is contemplated that the propeptide may be replaced with another functional propeptide, preferably a mammalian propeptide, more preferably a human propeptide.

In other embodiments the cell lines of the present invention express NGF chimeric and pantropic neurotrophins, such as those reported in U.S. Pat. No. 5,488,099, issued Jan. 30, 1996, in Urfer et al., EMBO J. 13(24):5896-909 (1994), and in WO 95/33829, published Dec. 14, 1995 in which the NGF has been modified to bind to more than one receptor or contains a receptor binding activity not normally present to a significant degree in the native NGF. Of particular interest are chimeras having an NGF amino acid backbone but modified to bind receptors other than trkA, such as trkB or trkC. These NGF forms have an amino acid sequence homologous (usually greater than 80% sequence identity, preferably greater than 90%, and more preferably greater than 95%, and most preferably greater than 97%) to the amino acid sequence of NGF, with substitutions which confer other neurotrophin specificities. In the preferred embodiment, the domains are substituted for NGF residues; that is, some number of amino acids are deleted from the NGF sequence, and an identical or similar number of amino acids are substituted, conferring an additional specificity. For example, a pantropic NGF is made with a D16A substitution, which confers BDNF specificity (trkB-binding activity), while retaining trkA-binding activity. Preferred are those in which amino acid substitutions have been made in NGF with an amino acid from a corresponding position in NT-3 that is responsible for binding the trkC receptor for NT-3. Such NGF mutants have NT-3-like trkC receptor binding activity while retaining NGF conformation, pharmacokinetics and purification behavior (Urfer, et al., Biochemistry 36(16):4775-4781 (1997)). For example, substitutions in the pre-variable region 1 (V18E+V20L+G23T) and in variable region 4 (Y79Q+T81 K+H84Q+F86Y+K88R) are included which allow trkC-binding activity. Amino acid numbering starts with amino acid no. 1 of mature NGF in accordance with the numbering in the sequence listing. The substitutions in the pre-variable region 1 can be made with only single amino acid substitutions in variable region 4; for example, V18E+V20L+G23T and one or more of Y79Q, T81K, H84Q, F86Y, or K88R may be made. These NGF mutants can also be engineered to lack trkA binding activity, for example, by removing or modifying the N-terminal 1 to 9 amino acids of NGF. The 109 amino acid species (10-118)hNGF, resulting from the loss of the first 9 residues of the N-terminus and the last two residues from the C-terminus of purified recombinant human NGF, is 300-fold less efficient in displacing mouse ¹²⁵I-NGF from the human trkA receptor compared to (1-118)hNGF (Shih et al., J. Biol. Chem. 269 (44):27679-86 (1994)).

In a preferred embodiment, a pantropic NGF is a pantropic neurotrophin, which has an amino acid sequence homologous to the amino acid sequence of NGF, with domains that confer other neurotrophin specificities. In one preferred embodiment, the domains are substituted for NGF residues; that is, some number of amino acids are deleted from the NGF sequence, and an identical or similar number of amino acids are substituted, conferring an additional specificity. For example, a pantropic NGF is made with a D16A substitution, which confers BDNF specificity, plus substitutions in the pre-variable region 1 (V18+V20L+G23T) and in variable region 4 (Y79Q+T81K+H84Q+F86Y+K88R). Alternatively, the substitutions in the pre-variable region 1 can be made with only single amino acid substitutions in variable region 4; for example, V18E+V20L+G23T and one of Y79Q, T81 K, H84Q, F86Y or K88R may be made. US 6,503,728 discloses how to obtain pantropic neurotrophin based on NGF.

Preferred pantropic neurotrophins include NGF selected from the group consisting of D16A-V18E-V20L-G23T-Y79Q-T81K-H84Q-F86YK88R-hNGF (Fig. 15A) and D16A-T81K-H84Q-F86Y-K88R-hNGF (Fig. 15B).

An NGF variant of the invention can be derived from NGF by substituting at amino acid positions G23, H84, and V18 or V20 to impart trkC binding in order to obtain an NGF variant that binds trkC. In a preferred embodiment the V20 is substituted. Optionally, the NGF variant can further contain a substitution of F86, T81, or T29, which can further enhance or fine tune NT-3 specificity. The trkC-binding or signal inducing activity is recruited or imparted by changes in NGF that consist essentially of the changes discussed herein. Preferred NGF variants include NGF130, NGF131, NGFR2, and NGFR3, as shown in Figure 16. Most preferred substitutions at these positions are G23T, H84Q, V18E, V20L, F86Y, T81K, and T29I. However, other substitutions are appropriate, preferably conservative or homologous series substitutions. For example, V18 can be conservatively substituted with leucine or threonine to minimally effect trkC binding or a glutamic acid conservative amino acid or homolog, such as with aspartic acid or glutamine, to enhance trkC binding. V20 can be substituted with larger hydrophobic amion acids isoleucine, methionine, or threonine to enhance trkC binding, and perhaps alanine to have a minimal trkC binding effect. G23 can be substituted with serine. T29 can be substituted with isoleucine, valine, leucine or serine. Preferably, T29I is present when F86Y is present. Y79 can be substituted with glutamine, phenylalanine, or asparagine, but preferably remains Y79. T81 can be substituted with arginine to effect trkC binding or with isoleucine, valine, leucine or serine with minimal trkC binding effect. H84 can be substituted with asparagine. F86 can be substituted with methionine, tryptophan, threonine or serine to effect trkC binding. US 6,330,310 discloses the characteristics of the NGF variants and methods to obtain the coding sequences.

Preferred trkC binding NGF variants are shown in Figure 16A and B. Figure 16A shows NGF130 (top) and NGF131 (bottom. Figure 16B shows NGFR2 (top) and NGFR3 (bottom). NGF130 contains the four changes: V20L, G23T, H84Q and F86Y. NGF131 contains the five changes V20L, G23T, T29I, H84Q, AND F86Y. NGFR2 contains the four changes V20L, G23T, T81K, and H84Q. NGFR3 contains the four changes V18E, G23T, T81K, and H84Q.

According to one embodiment of the invention, the NGF is a modified NGF, which has been modified to reduce its affinity for p75^{NGFR}. The genes coding for such modified NGF are described in U.S. Pat. No. 5,349,055. Specifically, the positively charged side chain of Lys32 may be replaced by, for instance the methyl group of Ala. Such reduction reduces the binding of the molecule to p75^{NGFR} to 5% of the binding seen with parent NGF. In another embodiment, the change of Lys34 into Ala reduces binding to A875 cells to 55% of the parent levels. In yet another embodiment, the simultaneous replacement of Lys32, Lys34 and Glu35 by alanine is used to completely abolish the binding of the mutant molecule to p75^{NGR}. In the case of each of these mutants, despite reduced or absent binding to p75^{NGFR}, the molecules retain wild-type biological activity on explants of sympathetic ganglia.

A preferred human pre-pro-NGF with reduced ability to bind p75^{NGFR} NGF is set forth in Figure 17. The mutations relative to wildtype NGF are shown in bold.

In an additional embodiment, mutant NGF may be designed based on the discovery that positively charged amino acids around amino acid 95 in a second beta-hairpin loop may interact with Lys32 and Lys34 to form a positively charged interface involved in binding to p75^{NGFR}. Simultaneous modification of Lys32 with either of the two other lysines (Lys 34 or Lys 95) results in loss of binding to p75^{NGFR}. Despite the lack of binding to p75^{NGFR}, these mutants bind to p140^{trk} and retain their biological activity, as measured by neuronal differentiation of PC12 cells and survival of cultured sympathetic neurons.

Also embodied herein are modifications of NGF to enhance the stability. As described herein, selective modification of one or more of the amino acid residues occurring between amino acid 25 and 36 of nerve growth factor appears to alter the stability of the factor. Accordingly, the invention contemplates alteration of such amino acids, followed by measuring the stability and biological activity of the altered molecule to select those factors that retain their biological activity but have enhanced stability as compared to the parent molecule.

Another type of modified NGF includes Multifunctional NGF such as those disclosed in US 5,512,661. A chimeric protein which has neurotrophic activity and which consists essentially of a NGF, wherein about 3 to about 13 consecutive amino acids of said NGF are replaced by a similarly sized amino acid sequence of a second neurotrophic factor such that the resulting chimeric protein differs in sequence by at least 3 amino acids from NGF. Especially preferred chimeric neurotrophic factors include those in which the second neurotrophic factor is BDNF, CNTF, NT-3 or NT-4. Particularly preferred is the chimer between NGF and BDNF.

Such chimeric NGF may be cloned by splicing together portions of NGF and BDNF genes using overlap extension of primers in polymerase chain reaction. Chimeric molecules may be produced in which relatively large (chimeras R1-R5) or small (R6-R10) regions are substituted by NGF sequences. The amino acid sequences of R1-R10 are presented in FIG. 5A-D of US 5,512,661. Chimeras R1 through R6 comprise portions of human BDNF and human NGF and chimeras R7 through R10 comprise portions of human BDNF and mouse NGF. Table 2 presents a summary of the approximate extent of BDNF and NGF sequences in chimeras R1 through R10.

**TABLE 2**

| Chimera | BDNF Sequence | NGF Sequence |
|---|---|---|
| R1 | preprosequence | full mature sequence |
| R2 | preprosequence | residues 18-120 |
| | residues 1-17 | |
| R3 | preprosequence | residues 59-120 |
| | residues 1-58 | |
| R4 | preprosequence | residues 73-120 |
| | residues 1-72 | |
| R5 | preprosequence | residues 82-120 |
| | residues 1-81 | |
| R6 | preprosequence | residues 111-120 |
| | residues 1-110 | |
| R7 | preprosequence | residues 34-41 |
| | residues 1-33 | |
| | residues 42-118 | |
| R8 | preprosequence | residues 44-50 |
| | residues 1-43 | |
| | residues 51-118 | |
| R9 | preprosequence | residues 59-66 |
| | residues 1-58 | |
| | residues 67-118 | |
| R10 | preprosequence | residues 73-79 |
| | residues 1-72 | |
| | residues 80-118 | |

### Neurological disorders

According to this invention, capsular delivery of NGF, synthesised by human cells in vivo, to the brain ventricles, brain parenchyma, or other suitable CNS location, ranging from 1-1500 ng/day is contemplated. The actual dosage of NGF, or other suitable factor, can be varied by implanting high or low producing clones, more or less cells or fewer or greater number of capsules. We contemplate delivery of 0.1-1500, preferably 1 to 1000, more preferably 10-600, most preferably 50-500, ng NGF/human/day, for ventricular delivery and 0.1-1500, preferably 10-150 ng NGF/human/day for parenchymal delivery. These dosage ranges are significantly lower than those previously reported doses of NGF needed for cholinergic basal forebrain neuronal sparing/sprouting in rodent studies and in primate studies (17-350 µg/day), especially if the dosages are normalized to account for brain volume differences between rodents, primates and humans. Tuzynski et al., J. Neurosci., 10, pp. 3604-14 (1990); Koliatsos et al., Ann. Neurol., 30, pp. 831-840 (1991), Koliatsos et al., Experimental Neurol., 112, pp. 161-73 (1991); Dekker et al., Neuroscience, 60, pp. 299-309 (1994). In the one clinical patient evaluated, the dose of NGF delivered was 75 µg/day. (Olson et al., J. Neural Trans., 4, pp. 79-95 (1992)). In one embodiment, genetically modified human cells secreting human NGF (hNGF) are encapsulated in semipermeable membranes, and implanted intraventricularly or intraparenchymally in a suitable mammalian host, preferably a primate, most preferably a human.

Accordingly, NGF-expressing cell lines of the invention are believed to be useful in promoting the development, maintenance, or regeneration of neurons in vivo, including central (brain and spinal chord), peripheral (sympathetic, parasympathetic, sensory, and enteric neurons), and motorneurons. NGF-expressing cell lines of the invention are utilised in methods for the treatment of a variety of neurologic diseases and disorders. In a preferred embodiment, the cell lines of the present invention are administered to a patient to treat neurological disorders. By "neurological disorders" herein is meant disorders of the central and/or peripheral nervous system that are associated with neuron degeneration or damage. Specific examples of neural disorders include, but are not limited to, Alzheimer's disease, Parkinson's disease, Huntington's disease, stroke, CNS trauma, ALS, peripheral neuropathies, neuropathic pain, and other conditions characterized by necrosis or loss of neurons or their processes, whether central or peripheral, in addition to treating damaged nerves due to trauma, bums, kidney disfunction, injury, and the toxic effects of chemotherapeutics used to treat cancer or AIDS. For example, peripheral neuropathies associated with certain conditions, such as neuropathies associated with diabetes, AIDS, or chemotherapy may be treated using the formulations of the present invention. The cell lines may also be used as a component of culture media for use in culturing nerve cells in vitro or ex vivo.

In various applications of the invention, NGF-expressing cell lines are administered to patients in whom the nervous system has been damaged by idiopathic processes, trauma, surgery, stroke and ischemia, infection, metabolic disease, nutritional deficiency, malignancy, or toxic agents, to promote the survival or growth of neurons, or in whatever conditions have been found treatable with NGF. For example, NGF-expressing cell lines of the invention can be used to promote the survival or growth of motorneurons that are damaged by trauma or surgery. Also, NGF-expressing cell lines of the invention can be used to treat degenerative motoneuron disorders, such as amyotrophic lateral sclerosis (Lou Gehrig's disease), Bell's palsy, and various conditions involving spinal muscular atrophy, or paralysis. NGF-expressing cell lines of the invention can be used to treat human neurodegenerative disorders, such as Alzheimer's disease and other dementias, minimal cognitive impairment (MCI), Parkinson's disease, epilepsy, multiple sclerosis, Huntington's disease, Down's Syndrome, nerve deafness, and Meniere's disease. NGF-expressing cell lines of the invention can be used as cognitive enhancer, to enhance learning particularly in dementias or trauma.

Alzheimer's disease, which has been identified by the National Institutes of Aging as accounting for more than 50% of dementia in the elderly, is also the fourth or fifth leading cause of death in Americans over 65 years of age. Four million Americans, 40% of Americans over age 85 (the fastest growing segment of the U.S. population), have Alzheimer's disease. Twenty-five percent of all patients with Parkinson's disease also suffer from Alzheimer's disease-like dementia and in about 15% of patients with dementia, Alzheimer's disease and multi-infarct dementia coexist. The third most common cause of dementia, after Alzheimer's disease and vascular dementia, is cognitive impairment due to organic brain disease related directly to alcoholism, which occurs in about 10% of alcoholics. However, the most consistent abnormality for Alzheimer's disease, as well as for vascular dementia and cognitive impairment due to organic brain disease related to alcoholism, is the degeneration of the cholinergic system arising from the basal forebrain (BF) to both the codex and hippocampus (Bigl et al. in Brain Cholinergic Systems, M. Steriade and D. Biesold, eds., Oxford University Press, Oxford, pp.364-386 (1990)). Furthermore, there is a number of other neurotransmitter systems affected by Alzheimer's disease (Davies Med. Res. Rev. 3:221 (1983)). However, cognitive impairment, related for example to degeneration of the cholinergic neurotransmitter system, is not limited to individuals suffering from dementia. It has also been seen in otherwise healthy aged adults and rats. Studies that compare the degree of learning impairment with the degree of reduced cortical cerebral blood flow in aged rats show a good correlation (Berman et al. Neurobiol. Aging 9:691 (1988)). In chronic alcoholism the resultant organic brain disease, like Alzheimer's disease and normal aging, is also characterized by diffuse reductions in cortical cerebral blood flow in those brain regions where cholinergic neurons arise (basal forebrain) and to which they project (cerebral cortex) (Lofti et al., Cerebrovasc. and Brain Metab. Rev 1:2 (1989)). Such dementias can be treated by administration of NGF-secreting cell lines or devices of the invention.

Further, NGF-secreting cell lines or devices of the invention implanted either in the peripheral tissues or within the CNS, are preferably used to treat neuropathy, and especially peripheral neuropathy. "Peripheral neuropathy" refers to a disorder affecting the peripheral nervous system, most often manifested as one or a combination of motor, sensory, sensorimotor, or autonomic neural dysfunction. The wide variety of morphologies exhibited by peripheral neuropathies can each be attributed uniquely to an equally wide number of causes. For example, peripheral neuropathies can be genetically acquired, can result from a systemic disease, or can be induced by a toxic agent. Examples include, but are not limited to, diabetic peripheral neuropathy, distal sensorimotor neuropathy, AIDS-associated neuropathy, or autonomic neuropathies such as reduced motility of the gastrointestinal tract or atony of the urinary bladder. Examples of neuropathies associated with systemic disease include post-polio syndrome; examples of hereditary neuropathies include Charcot-Marie-Tooth disease, Refsum's disease, Abetalipoproteinemia, Tangier disease, Krabbe's disease, Metachromatic leukodystrophy, Fabry's disease, and Dejerine-Sottas syndrome; and examples of neuropathies caused by a toxic agent include those caused by treatment with a chemotherapeutic agent such as taxol, vincristine, cisplatin, methotrexate, or 3'-azido-3'-deoxythymidine.

A therapeutically effective dose of NGF-secreting cells or devices is administered to a patient. By "therapeutically effective dose" herein is meant a dose that produces the effects for which it is administered or that amount which provides therapeutic effect in a particular administration regimen. Dosage of the NGF released from the cell lines or devices of the present invention is that needed to achieve an effective concentration of NGF in vivo, for the particular condition treated, though the dosage varies with the type of NGF variant, the desired duration of the release, the target disease, the subject animal species and other factors, such as patient condition. The exact dose will depend on the disorder to be treated and the implantation site, and will be ascertainable by one skilled in the art using known techniques. Currently, rhNGF has been administered subcutaneously to patients having diabetic- or AIDS-related peripheral neuropathy, with Phase III trials in progress. The weekly dosage amounts used in these clinical studies provides a good starting point for the clinician to determine dosages for administration of NGF of the invention. Data indicate that in situ cell synthesised NGF is 3-4 orders of magnitude more potent than infused or injected recombinant protein as evidenced by primate and rodent studies using encapsulated NGF-secreting BHK cells. Due to the documented higher potency of in situ synthesised NGF compared to administered protein formulations and the improved dosage profile obtainable by the continuous and local release from capsules, in general, the NGF-secreting cell lines or devices of the present invention are administered to give about 0.01 ng NGF/kg body weight to about 100 µg/kg per day, preferably from 0.02 ng/kg to 10 µg/kg, more preferably 0.03 to 500 ng/kg, and most preferably 0.5 ng/kg to 100 ng/kg. In some embodiments doses of 0.03 to 1.0 ng/kg, more preferably 0.1 to 0.3 ng/kg, are given. In addition, as is known in the art, adjustments for age as well as the body weight, general health, sex, diet, time of administration, drug interaction and the severity of the disease may be necessary, and will be ascertainable with routine experimentation by those skilled in the art. Typically, the clinician will administer NGF-secreting cell lines or devices of the invention until a dosage is reached that ameliorates, repairs, maintain, and/or, optimally, reestablishes neuron function. The dosis may also be a prophylactic dose which prevents or reduces degeneration of neurons. The progress of this therapy is easily monitored by conventional assays.

Results from Phase II clinical trials indicate that patients with peripheral neuropathy disease require three dosings per week of rhNGF at either 0.3 or 0.1 µg/kg. This means that only 21 or 7 µg per dosing of rhNGF is needed for an average patient of body weight 70 kg. The current rhNGF liquid formulation is 2 mg/mL in 10 mM sodium acetate, pH 5.5, 142 mM NaCl. This dosing information provides a starting point for dosing with the NGF-secreting cell lines or devices taking the higher potency and local delivery into consideration.

### Ophthalmic disorders.

Most, if not all, ophthalmic diseases and disorders are associated with one or more of three types of indications: (1) angiogenesis, (2) inflammation, and (3) degeneration. To treat these disorders, the devices of the present invention permit delivery of NGF and oprionally other factos acting as anti-angiogenic factors; anti-inflammatory factors; factors that retard cell degeneration, promote cell sparing, or promote cell growth; and combinations of the foregoing. Based on the indications of a particular disorder, one of ordinary skill in the art can administer any suitable molecule or combination of molecules from the three groups at the dosages specified below.

Diabetic retinopathy, for example, is characterized by angiogenesis and retinal degeneration. This invention contemplates treating diabetic retinopathy by implanting devices delivering NGF either intraocularly, preferably in the vitreous, or periocularly, preferably in the sub-Tenon's region. We most prefer delivery into the vitreous for this indication. It is also desirable to co-deliver one or more anti-angiogenic factors, either intraocularly or periocularly, preferably intraocularly, and most preferably intravitreally.

Uveitis involves inflammation and secondary degeneration. This invention contemplates treating uveitis by intraocular, preferably vitreal or anterior chamber, implantation of devices secreting NGF.

Retinitis pigmentosa, by comparison, is characterized by primary retinal degeneration. This invention contemplates treating retinitis pigmentosa by intraocular, preferably vitreal, placement of devices secreting NGF.

Age-related macular degeneration involves both angiogenesis and retinal degeneration. This invention contemplates treating this disorder by using the inventive devices to deliver NGF intraocularly, preferably to the vitreous, and/or one or more anti-angiogenic factors intraocularly or periocularly, preferably periocularly, most preferably to the sub-Tenon's region.

Glaucoma is characterized by increased ocular pressure and loss of retinal ganglion cells. Treatments for glaucoma contemplated in this invention include delivery of NGF that protect retinal cells from glaucoma associated damage, delivered intraocularly, preferably intravitreally.

Intraocularly, preferably in the vitreous, we contemplate delivery of a neurotrophic factor in a dosage range of 50 pg to 500 ng, preferably 100 pg to 100 ng, and most preferably 1 ng to 50 ng per eye per patient per day. For periocular delivery, preferably in the sub-Tenon's space or region, slightly higher dosage ranges are contemplated of up to 1 µg per patient per day.

We contemplate use of the present invention to treat a wide variety of ophthalmic diseases and disorders characterized by, but not limited to, angiogenesis, inflammation, degeneration, or some combination thereof. Some examples of ophthalmic disorders that may be treated by various embodiments of the present invention include uveitis, retinitis pigmentosa, age-related macular degeneration and other acquired disorders, retinopathy, retinal vascular diseases and other vascular anomalies, endophthalmitis, infectious diseases, inflammatory but non-infectious diseases, ocular ischemia syndrome, peripheral retinal degenerations, retinal degenerations and tumors, choroidal disorders and tumors, vitreous disorders, retinal detachment, non-penetrating and penetrating trauma, post-cataract complications, and inflammatory optic neuropathies.

Age-related macular degeneration includes, but is not limited to, dry age-related macular degeneration, exudative age-related macular degeneration, and myopic degeneration.

Retinopathy includes, but is not limited to, diabetic retinopathy, proliferative vitreoretinopathy, and toxic retinopathy.

The present invention may be useful for the treatment of ocular neovascularization, a condition associated with many ocular diseases and disorders and accounting for a majority of severe visual loss. For example, we contemplate treatment of retinal ischemia-associated ocular neovascularization, a major cause of blindness in diabetes and many other diseases; corneal neovascularization, which predisposes patients to corneal graft failure; and neovascularization associated with diabetic retinopathy, central retinal vein occlusion, and possibly age-related macular degeneration.

The present invention may also be used to treat ocular symptoms resulting from diseases or conditions that have both ocular and non-ocular symptoms. Some examples include AIDS-related disorders such as cytomegalovirus retinitis and disorders of the vitreous; pregnancy-related disorders such as hypertensive changes in the retina; and ocular effects of various infectious diseases such as tuberculosis, syphilis, lyme disease, parasitic disease, toxocara canis, ophthalmonyiasis, cyst cercosis, and fungal infections.

In one application of the present invention, living cells are encapsulated and surgically inserted (under retrobulbar anesthesia) into the vitreous of the eye. For vitreal placement, the device may be implanted through the sclera, with a portion of the device or tether protruding through the sclera. Most preferably, the entire body of the device is implanted in the vitreous, with no portion of the device protruding into or through the sclera. Preferably the device is tethered to the sclera (or other suitable ocular structure). The tether may comprise a suture eyelet, or any other suitable anchoring means (see e.g. US 6,436,427). The device can remain in the vitreous as long as necessary to achieve the desired prophylaxis or therapy. Such therapies for example include promotion of neuron or photoreceptor survival or repair, or inhibition and/or reversal of retinal or choroidal neovascularization, as well as inhibition of uveal, retinal, and optic nerve inflammation. This embodiment is preferable for delivering NGF to the retina.

With vitreal placement, NGF may be delivered to the retina or the RPE.

In another embodiment, cell-loaded devices are implanted periocularly, within or beneath the space known as Tenon's capsule. This embodiment is less invasive than implantation into the vitreous and thus is generally preferred. This route of administration also permits delivery of NGF to the RPE or the retina. This embodiment is especially preferred for treating choroidal neovascularization and inflammation of the optic nerve and uveal tract. In general, delivery from this implantation site will permit circulation of NGF to the choroidal vasculature, the retinal vasculature, and the optic nerve.

According to this embodiment we prefer periocular delivery (implanting beneath Tenon's capsule) of NGF to the choroidal vasculature to treat macular degeneration (choroidal neovascularization).

Delivery of NGF directly to the choroidal vasculature (periocularly) or to the vitreous (intraocularly) using the devices and methods of this invention may reduce the above-mentioned problems and may permit the treatment of poorly defined or occult choroidal neovascularization. It may also provide a way of reducing or preventing recurrent choroidal neovascularization via adjunctive or maintenance therapy.

Two or more separately engineered cell lines can be co-encapsulated, or the delivery cell line engineered to secrete a factor in addition to NGF, or more than one device can be implanted at the site of interest. Devices may be implanted in two or more different sites in the eye concurrently, to deliver NGF and other factors. For example, it may be desirable to deliver NGF to the vitreous to supply the neural retina (ganglion cells to the RPE) and to deliver an anti-angiogenic factor via the sub-Tenon's space to supply the choroidal vasculature. While treatment using more than one device is contemplated and up to five devices per eye, we prefer implantation of three devices or less per eye.

Dosage can be varied by any suitable method known in the art. This includes changing (1) the number of cells per device, (2) the number of devices per eye, or (3) the level of NGF production per cell. We prefer use of 10³ to 10⁸ cells per device, more preferably 5*10⁴ to 5*10⁶ cells per device.

The present cell lines and devices are useful for treating chronic wounds in areas that are poorly vascularised and poorly innervated. Thus, chronic wounds that can be treated by the present method include, but are not limited to, wounds caused by or associated with the following conditions: corneal epithelial defects which can result from varying causes, eg. from severing or disruption of the first branch of the trigeminal nerve, or from chemical bums such as acid or alkali bums, or as a complication of viral infections eg. ophthalmicus zoster or herpes keratitis; epithelial breakdown following penetrating keratoplasty; delayed healing following photorefractive keratectomy (PRK); diabetic foot ulcers; decubitus ulcers; and venous stasis ulcers. These wounds are present in the eye or in other areas of the body, eg. the lower leg and foot, sacral region, buttocks.

The invention may be used in a method of healing a corneal wound or defect by administering a therapeutically effective amount of NGF-secreting cells or devices to the subject presenting the wound. In another example, the present method is applied to the treatment of cutaneous wounds.

The present methods of healing chronic wounds will typically be practiced in a human subject or patient but can be applied to other mammals including but not limited to horses, dogs, cats and other pets.

In accordance with the treatment method, one would administer to the subject or patient suffering from the wound, an amount of NGF-secreting cells or devices therapeutically effective to heal the wound. For treatment of eye or corneal wounds, a therapeutically effective amount of NGF-secreting cells or devices is the amount which will provide for an observable healing effect in the epithelium within twenty-four to seventy-two hours. The observable effect is improved clarity of the epithelium and/or a decrease in size of the epithelial defect. The healing effect on the epithelium in the eye can be observed using the slit lamp microscope which is a standard instrument used by ophthalmologists. In areas other than the eye, such as a diabetic foot ulcer or a decubitus ulcer, the therapeutically effective amount is the amount that will produce a beneficial healing effect visible to a physician within 3-7 days, generally within about 72 hours. The beneficial healing effect can be determined by daily measurement of the width, length and depth of the wound in millimeters, using a ruler. Preferably, the amount of NGF-secreting cells or devices administered is sufficient to reduce the size of the wound by at least 50%, preferably, by 75% and even more preferably, by 90-100%.

The amount to produce a desired therapeutic effect will vary in part with the type and extent of disease or injury in the particular subject at issue, biological activity of NGF employed, the manner and frequency of administration of the cells or devices, overall health of the individual being treated and the like. It would be expected that a subject having a minor injury could be benefited by a minor amount to speed recovery of the epithelial trauma. With greater epithelial disruption, it is expected that a greater amount may be desirable to provide the desired therapeutic effect. The therapeutically effective amount of NGF-secreting cells or devices used to treat cutaneous wounds will also vary depending on such factors as condition and age of the skin. The precise amount for use with any particular patient can be determined by those of skill in the pharmaceutical art taking into consideration these factors and the present disclosure.

### Encapsulation

Encapsulated cell therapy is based on the concept of isolating cells from the recipient host's immune system by surrounding the cells with a semipermeable biocompatible material before implantation within the host. The invention includes a device in which cells are encapsulated in an immunoisolatory capsule. An "immunoisolatory capsule" means that the capsule, upon implantation into a recipient host, minimises the deleterious effects of the host's immune system on the cells in the core of the device. Cells are immunoisolated from the host by enclosing them within implantable polymeric capsules formed by a microporous membrane. This approach prevents the cell-to-cell contact between host and implanted tissues, eliminating antigen recognition through direct presentation. The membranes used can also be tailored to control the diffusion of molecules, such as antibody and complement, based on their molecular weight (Lysaght et al., 56 J. Cell Biochem. 196 (1996), Colton, 14 Trends Biotechnol. 158 (1996)). Using encapsulation techniques, cells can be transplanted into a host without immune rejection, either with or without use of immunosuppressive drugs. Useful biocompatible polymer capsules usually contain a core that contains cells, either suspended in a liquid medium or immobilised within an immobilising matrix, and a surrounding or peripheral region of permselective matrix or membrane ("jacket") that does not contain isolated cells, that is biocompatible, and that is sufficient to protect cells in the core from detrimental immunological attack. Encapsulation hinders elements of the immune system from entering the capsule, thereby protecting the encapsulated cells from immune destruction. The semipermeable nature of the capsule membrane also permits the biologically active molecule of interest to easily diffuse from the capsule into the surrounding host tissue.

The capsule can be made from a biocompatible material. A "biocompatible material" is a material that, after implantation in a host, does not elicit a detrimental host response sufficient to result in the rejection of the capsule or to render it inoperable, for example through degradation. The biocompatible material is relatively impermeable to large molecules, such as components of the host's immune system, but is permeable to small molecules, such as insulin, growth factors, and nutrients, while allowing metabolic waste to be removed. A variety of biocompatible materials are suitable for delivery of growth factors by the composition of the invention. Numerous biocompatible materials are known, having various outer surface morphologies and other mechanical and structural characteristics. Preferably the capsule of this invention will be similar to those described by WO 92/19195 or WO 95/05452, or U.S. Pat. Nos. 5,639,275; 5,653,975; 4,892,538; 5,156,844; 5,283,187; or U.S. Pat. No. 5,550,050. Such capsules allow for the passage of metabolites, nutrients and therapeutic substances while minimizing the detrimental effects of the host immune system. Components of the biocompatible material may include a surrounding semipermeable membrane and the internal cell-supporting scaffolding. Preferably, the recombinant cells are seeded onto the scaffolding, which is encapsulated by the permselective membrane. The filamentous cell-supporting scaffold may be made from any biocompatible material selected from the group consisting of acrylic, polyester, polyethylene, polypropylene polyacetonitrile, polyethylene teraphthalate, nylon, polyamides, polyurethanes, polybutester, silk, cotton, chitin, carbon, or biocompatible metals. Also, bonded fiber structures can be used for cell implantation (U.S. Pat. No. 5,512,600). Biodegradable polymers include those comprised of poly(lactic acid) PLA, poly(lactic-coglycolic acid) PLGA, and poly(glycolic acid) PGA and their equivalents. Foam scaffolds have been used to provide surfaces onto which transplanted cells may adhere ( WO 98/05304). Woven mesh tubes have been used as vascular grafts (WO 99/52573). Additionally, the core can be composed of an immobilizing matrix formed from a hydrogel, which stabilizes the position of the cells. A hydrogel is a 3-dimensional network of cross-linked hydrophilic polymers in the form of a gel, substantially composed of water.

The jacket preferably has a molecular weight cutoff of less than 1000 kD, more preferably between 50-700 kD, most preferably between 70-300 kD. The molecular weight cutoff should be selected to ensure that the bioactive NGF can escape from the capsule while protecting the encapsulated cells from the immune system of the patient.

The thickness of the jacket typically lies in the range of 2 to 200 microns, more preferably from 50 to 150 microns. The jacket should have a thickness to give the capsule sufficient strength to keep the cells encapsulated and should with this in mind be kept as thin as possible to take up as little space as possible.

Various polymers and polymer blends can be used to manufacture the surrounding semipermeable membrane, including polyacrylates (including acrylic copolymers), polyvinylidenes, polyvinyl chloride copolymers, polyurethanes, polystyrenes, polyamides, cellulose acetates, cellulose nitrates, polysulfones (including polyether sulfones), polyphosphazenes, polyacrylonitriles, poly(acrylonitrile/covinyl chloride), as well as derivatives, copolymers and mixtures thereof. Preferably, the surrounding semipermeable membrane is a biocompatible semipermeable hollow fiber membrane. Such membranes, and methods of making them are disclosed by U.S. Pat. Nos. 5,284,761 and 5,158,881. The surrounding semipermeable membrane may be formed from a polyether sulfone hollow fiber, such as those described by U.S. Pat. No. 4,976,859 or U.S. Pat. No. 4,968,733. An alternate surrounding semipermeable membrane material is poly(acry/onitrile/covinyl chloride).

The capsule can be any configuration appropriate for maintaining biological activity and providing access for delivery of the product or function, including for example, cylindrical, rectangular, disk-shaped, patch-shaped, ovoid, stellate, or spherical. Moreover, the capsule can be coiled or wrapped into a mesh-like or nested structure. If the capsule is to be retrieved after it is implanted, configurations, which tend to lead to migration of the capsules from the site of implantation, such as spherical capsules small enough to travel in the recipient host's blood vessels, are not preferred. Certain shapes, such as rectangles, patches, disks, cylinders, and flat sheets offer greater structural integrity and are preferable where retrieval is desired. A particularly preferred shape is cylinder-shaped as such a shape is easily produced from hollow fibers which can produces industrially.

When macrocapsules are used, preferably at least 10³ cells are encapsulated, such as between 10³ and 10⁸ cells are encapsulated, most preferably 10⁵ to 10⁷ cells are encapsulated in each device. Of course, the number of cells in each capsule depends on the size of the capsule. As a rule of thumb, in a capsule with foam (described below) the present inventors have found that loading between 10,000 and 100,000 cells per µL of capsule (volume calculated as the internal volume including foam), more preferably from 25,000 to 50,000 cells per µL, more preferably from 30,000 to 40,000 cells per µL. The number of cells to be loaded also depends on the size of the cells.

Dosage may be controlled by implanting a fewer or greater number of capsules, preferably between 1 and 10 capsules per patient.

A macrocapsule in the present context is a capsule having a volume of at least 1 µL, such as from 1 to 10 µL.

The scaffolding may be coated with extracellular matrix (ECM) molecules. Suitable examples of extracellular matrix molecules include, for example, collagen, laminin, and fibronectin. The surface of the scaffolding may also be modified by treating with plasma irradiation to impart charge to enhance adhesion of cells.

Any suitable method of sealing the capsules may be used, including the use of polymer adhesives or crimping, knotting and heat sealing. In addition, any suitable "dry" sealing method can also be used, as described, e.g., in U.S. Pat. No. 5,653,687.

The encapsulated cell devices are implanted according to known techniques. Many implantation sites are contemplated for the devices and methods of this invention. These implantation sites include, but are not limited to, the central nervous system, including the brain, spinal cord (see, U.S. Pat. Nos. 5,106,627, 5,156,844, and 5,554,148), and the aqueous and vitreous humors of the eye (see WO 97/34586).

### Foam scaffolds:

The foam scaffold may be formed from any suitable material that forms a biocompatible foam with an open cell or macroporous structure with a network of pores. An open-cell foam is a reticulate structure of interconnected pores. The foam scaffold provides a non-biodegradable, stable scaffold material that allows attachment of adherent cells. Among the polymers that are useful in forming the foam scaffolds for the devices of this invention are thermoplastics and thermoplastic elastomers.

Some examples of materials useful in forming suitable foam scaffolds are listed in Table 3.

**TABLE 3**

| | Thermoplastic |
|---|---|
| Thermoplastics: | Elastomers: |
| | |
| Acrylic | Polyamide |
| Modacrylic | Polyester |
| Polyamide | Polyethylene |
| Polycarbonate | Polypropylene |
| Polyester | Polystyrene |
| Polyethylene | Polyurethane |
| Polypropylene | Polyvinyl Alcohol |
| Polystyrene | Silicone |
| Polysulfone | |
| Polyethersulfone | |
| Polyvinylidene fluoride | |

Thermoplastic foam scaffolds made from polysulfone and polyethersulfone, and thermoplastic elastomer foam scaffolds made from polyurethane and polyvinyl alcohol are preferred.

The foam must have some (but not necessarily all) pores of a size that permits cells to attach to the walls or surfaces within the pores. The pore size, pore density and void volume of the
foam scaffold may vary. The pore shape may be circular, elliptical or irregular. Because the pore shape can vary considerably, its dimensions may vary according to the axis being measured. For the purposes of this invention, at least some pores in the foam should have a. pore diameter of between 20-500 µm, preferably between 50-150 µm. Preferably the foregoing dimensions represent the mean pore size of the foam. If non-circular, the pore may have variable dimensions, so long as its size is sufficient to permit adherent cells to attach to the walls or surfaces within the pore. In one embodiment, foams are contemplated having some elliptical pores that have a diameter of 20-500 µm along the minor axis and a diameter of up to 1500 µm along the major axis.

In addition to the foregoing cell permissive pores sizes, preferably a least a fraction of the pores in the foam should be less than 10 µm to be cell impermissive but still provide channels for transport of nutrients and biologically active molecules throughout the foam.

Pore density of the foam (i.e., the number per volume of pores that can accommodate cells, as described above) can vary between 20-90%, preferably between 50-70%.

Similarly, the void volume of the foam may vary between 20-90%, preferably between 30-70%.

The walls or surfaces of the pores are typically coated with an extracellular matrix molecule or molecules, or other suitable molecule. This coating can be used to facilitate adherence of the cells to the walls of the pores, to hold cells in a particular phenotype and/or to induce cellular differentiation.

Preferred examples of extracellular matrix molecules (ECM) that can be adhered to the surfaces within the pores of the foams include: collagen, laminin, vitronectin, polyornithine and fibronectin. Other suitable ECM molecules include glycosaminoglycans and proteoglycans; such as chrondroitin sulfate, heparin sulfate, hyaluron, dermatan sulfate, keratin sulfate, heparan sulfate proteoglycan (HSPG) and elastin.

The ECM may be obtained by culturing cells known to deposit ECM, including cells of mesenchymal or astrocyte origin. Schwann cells can be induced to synthesize ECM when treated with ascorbate and cAMP. See, e.g., Baron-Van Evercooren et al., "Schwann Cell Differentiation in vitro: Extracellular Matrix Deposition and Interaction," Dev. Neurosci., 8, pp. 182-96 (1986).

In addition, adhesion peptide fragments, e.g., RGD containing sequences (ArgGlyAsp), YIGSR-containing sequences (TyrIleGlySerArg), as well as IKVAV containing sequences (IleLysValAlaVal), have been found to be useful in promoting cellular attachment. Some RGD-containing molecules are commercially available-e.g., PepTite-2000.TM. (Telios).

The foam scaffolds of this invention may also be treated with other materials that enhance cellular distribution within the device. For example, the pores of the foam may be filled with a non-permissive hydrogel that inhibits cell proliferation or migration. Such modification can improve attachment of adherent cells to the foam scaffold. Suitable hydrogels include anionic hydrogels (e.g., alginate or carageenan) that may repel cells due to charge. Alternately, "solid" hydrogels (e.g., agarose or polyethylene oxide) may also be used to inhibit cell proliferation by discouraging binding of extracellular matrix molecues secreted by the cells.

Treatment of the foam scaffold with regions of a non-permissive material allows encapsulation of two or more distinct cell populations within the device without having one population overgrow the other. Thus non-permissive materials may be used within the foam scaffold to segregate separate populations of encapsulated cells. The distinct populations of cells may be the same or different cell types, and may produce the same or different biologically active molecules. In one embodiment, one cell population produces a substance that augments the growth and/or survival of the other cell population. In another embodiment, multiple cell types producing multiple biologically active molecules are encapsulated. This provides the recipient with a mixture or "cocktail" of therapeutic substances.

The devices of this invention may be formed according to any suitable method. In one embodiment, the foam scaffold may be pre-formed and inserted into a pre-fabricated jacket, e.g., a hollow fiber membrane, as a discrete component.

Any suitable thermoplastic or thermoplastic elastomer foam scaffold material may be preformed for insertion into a pre-fabricated jacket In one embodiment we prefer polyvinyl alcohol (PVA) sponges for use as the foam scaffold. Several PVA sponges are commercially available. For example, PVA foam sponges #D-3, 60 µm pore size are suitable (Rippey Corp, Kanebo). Similarly, PVA sponges are commercially available from Ivalon Inc. (San Diego, Cailf.). PVA sponges are water-insoluble foams formed by the reaction of aerated Poly(vinyl alcohol) solution with formaldehyde vapor as the crosslinker. The hydroxyl groups on the PVA covalently crosslink with the aldehyde groups to form the polymer network. The foams are flexible and elastic when wetted and semi-rigid when dried.

As an alternative, support a mesh or yam may be used as described in US 6,627,422.

For easy retrival the capsule may be equipped with a tether anchor.

### Support matrix for NGF producing cells

The method of the present invention further comprises the culturing of the NGF producing cells in vitro on a support matrix prior to implantation into the mammalian brain. The preadhesion of cells to microcarriers prior to implantation in the brain is designed to enhance the long-term viability of the transplanted cells and provide long term functional benefit. Methods for culturing cells on a support matrix and methods for implanting said cells into the brain are described in US 5,750,103 (Cherksey).

To increase the long term viability of the transplanted cells, i.e., transplanted NGF-secreting cells, the cells to be transplanted can be attached in vitro to a support matrix prior to transplantation. Materials of which the support matrix can be comprised include those materials to which cells adhere following in vitro incubation, and on which cells can grow, and which can be implanted into the mammalian body without producing a toxic reaction, or an inflammatory reaction which would destroy the implanted cells or otherwise interfere with their biological or therapeutic activity. Such materials may be synthetic or natural chemical substances, or substances having a biological origin.

The matrix materials include, but are not limited to, glass and other silicon oxides, polystyrene, polypropylene, polyethylene, polyvinylidene fluoride, polyurethane, polyalginate, polysulphone, polyvinyl alcohol, acrylonitrile polymers, polyacrylamide, polycarbonate, polypentent, nylon, amylases, natural and modified gelatin and natural and codified collagen, natural and modified polysaccharides, including dextrans and celluloses (e.g., nitrocellulose), agar, and magnetite. Either resorbable or non-resorbable materials may be used. Also intended are extracellular matrix materials, which are well-known in the art. Extracellular matrix materials may be obtained commercially or prepared by growing cells which secrete such a matrix, removing the secreting cells, and allowing the cells which are to be transplanted to interact with and adhere to the matrix. The matrix material on which the cells to be implanted grow, or with which the cells are mixed, may be an indigenous product of RPE cells. Thus, for example, the matrix material may be extracellular matrix or basement membrane material, which is produced and secreted by RPE cells to be implanted.

To improve cell adhesion, survival and function, the solid matrix may optionally be coated on its external surface with factors known in the art to promote cell adhesion, growth or survival. Such factors include cell adhesion molecules, extracellular matrix, such as, for example, fibronectin, laminin, collagen, elastin, glycosaminoglycans, or proteoglycans or growth factors.

Alternatively, if the solid matrix to which the implanted cells are attached is constructed of porous material, the growth- or survival promoting factor or factors may be incorporated into the matrix material, from which they would be slowly released after implantation in vivo.

When attached to the support according to the present invention, the cells used for transplantation are generally on the "outer surface" of the support. The support may be solid or porous. However, even in a porous support, the cells are in direct contact with the external milieu without an intervening membrane or other barrier. Thus, according to the present invention, the cells are considered to be on the "outer surface" of the support even though the surface to which they adhere may be in the form of internal folds or convolutions of the porous support material which are not at the exterior of the particle or bead itself.

The configuration of the support is preferably spherical, as in a bead, but may be cylindrical, elliptical, a flat sheet or strip, a needle or pin shape, and the like. A preferred form of support matrix is a glass bead. Another preferred bead is a polystyrene bead.

Bead sizes may range from about 10 µm to 1 mm in diameter, preferably from about 90 µm to about 150 µm. For a description of various microcarrier beads, see, for example, Fisher Biotech Source 87-88, Fisher Scientific Co., 1987, pp. 72-75; Sigma Cell Culture Catalog, Sigma Chemical Co., St, Louis, 1991, pp. 162-163; Ventrex Product Catalog, Ventrex Laboratories, 1989. The upper limit of the bead's size may be dictated by the bead's stimulation of undesired host reactions, which may interfere with the function of the transplanted cells or cause damage to the surrounding tissue. The upper limit of the bead's size may also be dictated by the method of administration. Such limitations are readily determinable by one of skill in the art.

### Suicide systems

The devices of the present invention, which encapsulate NGF-secreting cells, may be retrieved from the patient when required. As a further safety precaution the cells may be equipped with a suicide system, which ensures that the cells may be selectively killed upon administration of a suitable drug to the patient in question.

The suicide system is particularly preferred for naked cell transplantation according to the present invention, as the possibilities for removing naked cells after transplantation are very limited.

One such suicide system is based on thymidine kinases. By having a built-in suicide system in which a thymidine kinase is expressed constitutively or inducibly, the cells can be killed by administering to the individual a therapeutically effective amount of a nucleoside analog, such as AZT. The nucleoside analogue can be administered if the encapsulated cells start to proliferate in an uncontrolled manner. One may also wish to terminate the treatment simply because there is no need for the NGF-secreting cells anymore, because termination must be immediate and cannot await surgical removal of the encapsulated cells or because further treatment is by some other route.

In the cases where transplanted or encapsulated cells have been conditionally immortalised before transplantation there is a theoretical risk that the oncogene initiates transcription after transplantation and that the transplanted cells consequently become tumorigenic. Whenever cells are immortalised by transduction with an oncogene under the control of an inducible promoter (e.g. the Tet on-off system, the Mx1 promoter or the like), a thymidine kinase (TK) enzyme coding sequence is inserted into the vector construct under the control of the same promoter (e.g. by using an IRES construct) or the TK coding sequence is inserted into another vector with an identical promoter. This ensures that whenever the oncogene is transcribed, the TK is also transcribed and the transduced and tumorigenic cells can be selectively killed by administering a prodrug.

There are several examples of thymidine kinase (TK) genes described in the art. One preferred TK is the HSV-thymidine kinase. Other preferred kinases include Drosophila melanogaster thymidine kinase described in Munch-Petersen et al 2000, J. Biol. Chem. 275:6673-6679. Mutants of this particular kinase are even more preferred as they have decreased LD₅₀ with respect to several nucleoside analogues (WO 01/88106). Another group of preferred thymidine kinases include plant kinases described in WO 03/100045.

### Immunostimulatory cell surface proteins

In one embodiment there is provided encapsulated human cells capable of expressing an immunostimulatory cell surface polypeptide in addition to NGF or an NGF variant. These immunostimulatory cell surface expressing cells are particularly useful when encapsulated for implantation in a human patient, because cells escaping from a ruptured capsule are destroyed by the patient's immune system. A host immune response will not be triggered by the recombinant cells expressing an immunostimulatory cell surface polypeptide in an intact device. In case of a device failure, however, the released cells are effectively eliminated by phagocytes without complement activation or the creation of an immune memory.

In a specific embodiment, a chimeric polypeptide containing the human transferrin receptor membrane domain anchors a human IgG₁ Fc to the surface of the cell plasma membrane in a "reversed orientation", thus mimicking the configuration of IgG during opsonisation. The human IgG₁ chimeric polypeptide binds the Fc receptor to activate phagocytes, such as macrophages, but avoids the undesirable characteristics of also activating the complement cascade ("complement fixation"). A chronically activated complement system can kill host cells, and accumulating evidence suggests that this mechanism can cause many degenerative diseases, including inflammation and neurodegenerative diseases. Further details of this embodiment of the invention are described in US 6,197,294 (Neurotech)

According to this embodiment the cell line further comprises a construct comprising a promoter operatively linked to a polynucleotide sequence encoding a fusion protein comprising an immunostimulatory cell surface protein linked at the amino terminus to a second cell surface polypeptide, wherein the second cell surface polypeptide comprises a transmembrane region, wherein upon expression, the fusion protein is expressed on the cell surface.

Preferably the immunostimulatory cell surface polypeptide activates phagocytes but does not fix complement. In one embodiment the immunostimulatory cell surface polypeptide is a region of IgG, preferably Fc. The second cell surface polypeptide may be a transferrin receptor hinge region.

### Examples

### Comparative example 1: Cloning and expression of NGF

### Cloning of NGF from genomic DNA

Genomic DNA was purified from HEK-293 cells as described previously (Current Protocols in Molecular Biology). The entire NGF open reading frame was amplified from the genomic DNA by PCR using the primers hNGFs+BamHI (5'-TATAGGATCCCTCTGAGGGACCCAGAAACT-3'; SEQ ID No 24) and hNGFas+Xhol (5'-TATACTCGAGCAGGTCAGGCTCTTCTCAC-3'; SEQ ID No 25) under standard conditions. The resulting PCR product was purified and cloned in the BamHI/Xhol sites in pNS1n to yield pNS1n.hNGF. pNS1n is a customised vector derived from pcDNA3 (Invitrogen) with expression under control of the cytomegalovirus promoter carrying the neo resistance marker instead of zeo (Jensen et al 2002, J Biol Chem, 277: 41438-41447). The coding sequence of the cloned NGF is shown in Figure 9.

### Addition of Kozak

A Kozak consensus sequence (Marylin Kozak, Nucleic Acids Res. 1987 Oct 26; 15(20): 8125-48.) was added to the NGF sequence by PCR using the primers NGF+Kzk s (5'-TACAGGATCCGCCGCCGCCATGTCCATGTTGTTCTACAC-3'; SEQ ID No 26) and NGF 457as (5'-TTGATGTCTGTGGCGGTGGT-3'; SEQ ID No 27) under standard conditions using pNS1n.hNGF as template. The resulting product was digested with BamHl and EcoRI and cloned back into pNS1n.hNGF using the same restriction sites. The resulting vector was named pNS1n.KhNGF.

### Growth and transfection of cell lines

ARPE-19 cells (ATCC accession number CRL-2302, Dunn KC et al. ARPE-19, A human retinal pigment epithelial cell line with differentiated properties. Exp. Eye Res. 62: 155-169, 1996.) were grown in DMEM/F12 medium (REF Invitrogen) supplemented with 10% FBS (REF Hyclone) in 5% CO₂ at 37°C.

### Analysis of transiently transfected cells

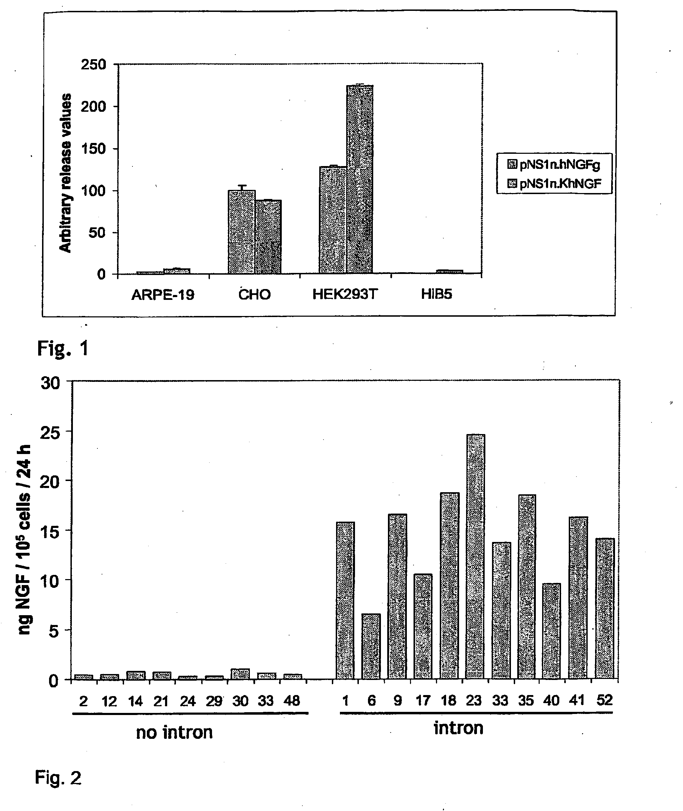

ARPE-19, CHO, HEK293T and HiB5 cells were grown as described previously (Tornøe et al, Gene, 297: 21-33 (2002)) and seeded in 6-well plates followed by transfection in triplicate with pNS1n.hNGF or pNS1n.KhNGF using Fugene6 according to the manufacturer's recommendations. Transfection efficiencies were determined to be comparable among cell lines by parallel transfections with an EGFP-expressing plasmid followed by flow cytometry analysis. 72 h after transfection, growth media was harvested and subjected to NGF ELISA analysis using the DuoSet human β-NGF ELISA kit (DuoSet ELISA Development System, R&D systems) according to the manufacturer's recommendations. All values were calculated as means of triplicates and normalized to the NGF release obtained from CHO cells transfected with pNS1n.hNGF set to the arbitrary value 100. Results of the experiment are shown in Figure 1. As expected, ARPE-19 and HiB5 produced significantly less NGF compared to the well-established protein production cell lines HEK293T and CHO. Furthermore, in all cell lines except CHO, the addition of a Kozak consensus sequence appeared to substantially increase the secretion of recombinant NGF.

### Example 2. Expression of NGF using intron containing transcripts.

### Addition of chimeric intron

BstZ17I/NheI fragments were released from pNS1n.hNGF and pNS1n.KhNGF. pCl-neo (Promega Inc) was digested with BamHI, blunted and digested with Nhel. The NGF-containing fragments from pNS1n.hNGF and pNS1n.KhNGF were cloned in the backbone fragment of pCI-neo to yield pCIn.hNGF and pCIn.KhNGF, respectively. A part of the nucleotide sequence of the pCIn.hNGF is set forth in Figure 10 starting with the first base of the CMV promoter. For comparison, a similar part of the pCIn.KhNGF is shown in Figure 11. The Kozak consensus sequence (underlined) is located in bases no 1128 to 1133 (Figure 11).

### pNN

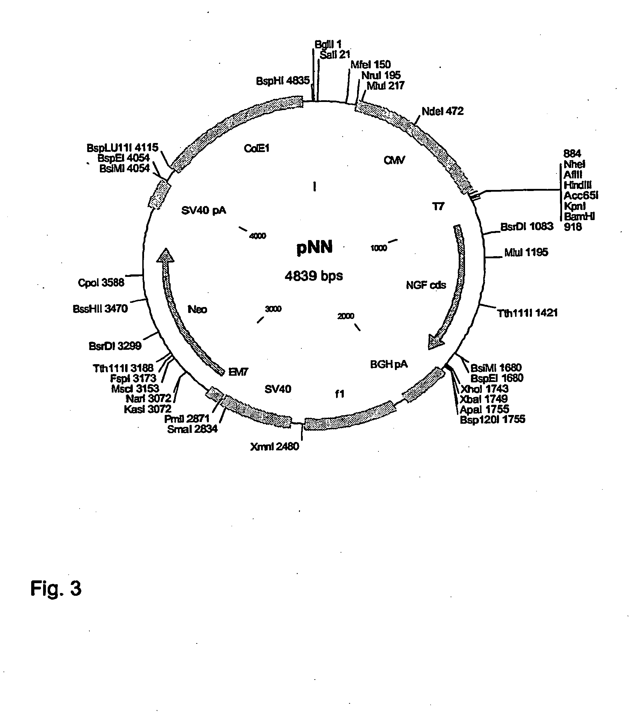

Human NGF was PCR cloned from human genomic DNA using the following primers: 5' primer: 5'-TATAGGATCCCTCTGAGGGACCCAGAAACT-3' (SEQ ID No 24), 3' primer: 5'-TATACTCGAGCAGGTCAGGCTCTTCTCAC-3' (SEQ ID No 25). The 839 bp PCR fragment was cut with BamHI and Xhol and inserted between BamHl and Xhol sites of pNS1n. pNS1n is a customised vector derived from pcDNA3 (Invitrogen) with expression under control of the cytomegalovirus promoter carrying the neo resistance marker instead of zeo (Jensen et al 2002, J Biol Chem, 277: 41438-41447). A vector map of pNS1n is shown in Figure 4. A vector map of pNN is shown in Figure 3.

### pNRN

Rat preproinsulin II intron A was PCR amplified from genomic DNA isolated from HiB5 cells, as a 189bp fragment using the following primers: 5' primer: 5'-TATAAAGCTTTAAGTGACCAGCTACAGTCG-3' (SEQ ID No 28) and 3' primer: 5'-TAGGATCCGTTGGAACAATGACCTGGAAG-3' (SEQ ID No 29). The PCR fragment was cut with HinDIII and BamHI and inserted between HindIII and BamHI sites of pNN resulting in the expression vector pNRN. A part of the nucleotide sequence of pNRN spanning from the first base of the promoter to the NGF coding sequence is shown in Figure 14. The intron is shown in lowercase.

### pUN

Human NGF (hNGF) was excised from pNN with BamHI and Xhol and inserted between BamHI and Xhol sites of pUbi1z (Johansen et al 2003, J Gene Medicine, 5:1080-1089.). A vector map of the resulting expression vector is shown in Figure 5. The part of the nucleotide sequence spanning from the first base of the promoter and to the NGF coding sequence is shown in Figure 13 with the ubiquitin intron shown in lowercase.

### pUNW

The woodchuck post-regulatory element (WPRE) was excised from from pHsCXW (GenBank acc. # AY468486) by first restricting with KpnI followed by blunting with Klenow polymerase and then cutting with BamHI. This semi-blunt 831 bp WPRE fragment was inserted between XbaI and PmeI sites of pUN.

### Transfections

The contact-inhibited cell line ARPE-19 was used in all transfections. ARPE-19-cells were grown in DMEM/F-12 w/glutamax-1 w/pyridoxin (Life Technologies) and 10% FCS serum Transfections were carried out using FuGene6 (Roche) according to the manufacturer's manual. For each transfection, 10⁵ cells in 6-well plates were grown 24 hours before transfection. 3µg plasmid was used total pr. transfection (1.5µg NGF plasmid and 1.5 µg EGFP plasmid, the latter for normalisation purposes). Medium was exchanged 24 hours post transfection.

For transient transfection experiments, 1 ml medium was harvested 72 hours post-transfection for ELISA testing (see below), and cell number in each well was determined, i.e. NGF production over 48 hours was measured. Furthermore, cells were submitted to FACS analysis to determine transfection efficiency. Stable transfections were accomplished by transfecting 2.4*10⁶ cells using 10 µg plasmid. 72 hours post-transfection the pNRN-transfectants were subjected to G418 selection (800 µg/ml), while pUN-, pUNW- og pUNWI-transfectants were grown in the presence of 150 µg/m Zeocin. Colonies were picked after 13-14 days and transferred to 48-well plates and grown. Colonies were grown to confluency in the 48-well plates. hNGF secretion levels were measured by NGF ELISA (see below) from 400 µl culture supernatant incubated 4 hours with the cells. Cell number was determined at the time of supernatant harvest.

### Analysis of stable clones based on the pCl expression vector

To generate recombinant cell clones, cells were transfected with linearised plasmids using FuGENE 6 Transfection Reagent (REF Roche) according to the manufacturer's recommendations. Following transfection, recombinant clones were selected in growth media supplemented with G418 (REF Sigma) and isolated using conventional cell culture techniques. A fixed number of cells were seeded in growth medium followed by media replacement after cell attachment. After 4 h incubation, media was removed and subjected to NGF ELISA analysis using the DuoSet human β-NGF ELISA kit (REF R&D systems) according to the manufacturer's recommendations. ELISA values were calculated as ng NGF / 10⁵ cells / 24 h. The NGF levels for representative clones with and without intron transfected with pCIn.hNGF and pNS1n.hNGF, respectively, are shown in Figure 2. Addition of an intron significantly increased NGF release by 10-20 fold from all pCIn.hNGF-transfected clones compared to clones transfected with the intron-less pNS1n.hNGF. Apparently, the presence of an intron is essential in obtaining high levels of recombinant NGF from a transfected cell line.

### NGF secretion from pNN, pNRN, pUN, and pUNW expression vectors

The four different plasmid constructs described above were transfected into ARPE-19 cells. Transient levels of human NGF secretion were measured by an NGF ELISA on culture supernatants. Stable clones were also isolated from each transfection and tested for NGF secretion by the NGF ELISA. The results are shown in figures 6 (transient transfections) and 7 (stable clones).

In transient cultures, the presence of rat insulin II intron A leads to an approximately 10-fold increase in secreted NGF levels (compare pNN with pNRN, figure 6). In stable clones the rat insulin II intron A causes an even larger increase in secreted NGF (compare pNN with pNRN in figure 7). Stable clones transfected with constructs with the ubiquitin promoter (also containing an intron) display secreted NGF levels comparable to the rat insulin intron construct (pNRN and pUN, figure 7) and much higher than the NGF levels form stable clones transfected tith the pNN construct. From the averaged bars in figure 7 it appears that the WPRE element does not exert a noticeable effect on secreted NGF levels when there is an intron in the transcript.

### Example 3: Comparison of stable transfected clones.

Clones transfected with pCIn.hNGF, pCIn.KhNGF and pNS1n-rINSintroA-hNGF (pNRN) were selected as described above. These three expression vectors gave the highest NGF release levels in transient and stable transfection. The NGF release was measured as described in Example 1. The results are shown in Figure 8. All the selected clones secreted in excess of 5 ng NGF/10⁵ cells/24 hours.

Clones #33 (NGC-0233) and #95 (NGC-0295) have been deposited under the Budapest Treaty with DSMZ, Mascheroder Weg 1b, D-38124 Braunschweig, Germany, under accession numbers DSM ACC2706 and DSM ACC2707 respectively.

### Example 4: Evaluation of processing of NGF from transfected clones

The purpose of this experiment was to analyse NGF secreted from transfected ARPE-19 clones in Western blot analysis to confirm correct processing of the secreted NGF.

### Experiment 1

Briefly, conditioned medium was collected from ARPE-19/pClKhNGF clone 23, clone 84 (Figure 8) and parental ARPE-19 cells. After collection of medium, cells were counted and lysed in sample buffer. As a control recombinant βhNGF from R&D systems cat#256-GF-100 was used.

Secreted NGF was partially purified from the conditioned medium. In brief, a 96-well plate was coated with 25 µg/ml Goat anti human Fc (Jackson cat#109 005 098) and blocked with 1% BSA. After washing, wells were incubated with recombinant human TrkA/Fc fusion protein (R&D Systems # 175-TK). The conditioned medium was subsequently added to the wells where NGF bound to the immobilised TrkA. Unbound protein was washed away and the bound NGF was eluted with sample buffer (including DTT).

Boiled and denatured samples were loaded on a 8-18% gradient SDS gel, exposed to SDS-PAGE and blotted to a PVDF membrane. NGF was detected using an NGF antibody (Goat Anti-human β-NGF antibody cat # AF-256-NA from R&D systems) and a HRP-linked anti-goat antibody. Detection with ECL+.

### Experiment 2

As experiment 1, but cells were grown in serum-free medium for 24 hours prior to harvesting of cells and conditioned medium.

### Results:

Lysates: only lysates from the clones transfected with pCInKhNGF showed a band corresponding to pro-NGF.
Conditioned medium: conditioned medium from both clone 23 and 84 showed bands corresponding to the βNGF monomer as well as the β-NGF dimer. The bands from the control recombinant β-NGF showed a corresponding ratio between dimer and monomer.

### Conclusion:

Only cells transfected with pCInKhNGF showed secretion of detectable amounts of NGF in contrast to parental ARPE-19 cells. The molecular weight of the bands corresponding to NGF monomer and dimer confirmed that NGF was secreted with the correct processing.

### Experiment 3

The purpose of this experiment was to analyse NGF secreted from NGF producing clones in Western blot analysis to confirm correct processing of the secreted NGF. Four ARPE-19 clones (#70, 95, 33 and 48) and one NTC200 clone (#68, transfected with pCIn,hNGF) were chosen. The NTC200 cell is a subpopulation of the ARPE-19 cell line and was grown similarly (Tao et al, Invest Ophthalmol Vis Sci (43), 10:3292-3298 (2002)).

To evaluate the expression and processing of NGF in stable NGF producing clones, a fixed number of cells were seeded in growth medium. The next day, medium was replaced with Human Endothelial Serum-free Medium (HE-SFM) from Invitrogen (cat#11111-044) or HyQ^{®}CDM4-Retino^{™} from HyClone (cat# SH30520). After 24 h incubation, conditioned media from NGF producing clones and parental ARPE-19 cells were collected and 5X concentrated sample buffer (with DTT) was added. Recombinant β-hNGF (R&D systems #256-GF-100) was diluted in 1x sample buffer and used as a positive control.

Boiled and denatured samples were loaded on a 8-18% gradient SDS gel, exposed to SDS-PAGE and blotted to a PVDF membrane. NGF was detected using an NGF antibody (Goat Anti-human β-NGF antibody cat # AF-256-NA from R&D systems) and a HRP-linked anti-goat antibody. Detection with ECL+.

### Results (Figure 23):

Conditioned medium from the four ARPE-19 clones (#70, 95, 33 and 48) showed bands corresponding to the β-NGF monomer (13 kDa), whereas there were no immunoreactive bands in conditioned medium from parental ARPE-19 cells. NGF in conditioned medium from the NTC200 clone #68 was undetectable by Western blotting.

### Conclusion:

Only cells transfected with the constructs pCIn.hNGF, pCIn.KhNGF and pNS1n.rINSintrA-hNGF showed secretion of NGF. The molecular weight of the band corresponding to NGF monomer confirmed that NGF was secreted in HE-SFM with the correct processing.

### Example 5: Evaluation of NGF release from confluent stable clones

To evaluate the stability of NGF release in confluent cultures of stable clones, a fixed number of cells were seeded in growth medium. The next day, medium was replaced with Human Endothelial Serum-free Medium (HE-SFM) from Invitrogen (cat#11111-044). After 4 h incubation, media were removed and subjected to NGF ELISA analysis using the DuoSet human β-NGF ELISA kit (REF R&D systems) according to the manufacturer's recommendations. ELISA values were calculated as ng NGF / ml / 24 h. Cells were allowed to grow to confluency and maintained in culture for 6 weeks. NGF release in 4 h media was determined every week. The NGF levels in HE-SFM for representative clones transfected with the constructs pCIn.hNGF, pCIn.KhNGF and pNS1n.rINSintrA-hNGF, respectively, are shown in Figure 21. At the end of the experiment, cells were trypsinated and counted. Figure 22 shows NGF release for week 6 calculated as ng NGF / 10⁵ cells / 24 h. Note that cells in clone # 68 are larger than other clones, leading to a lower cell number in confluent cultures, explaining the relative difference between NGF release for this clone in fig. 21 and figure 22. Clones # 68 and # 16 are NTC-200 (see example 4) cells transfected with pCIn.hNGF. The remaining clones are ARPE-19 clones transfected with expression constructs as shown in Figure 8.

### Example 6. Bioactivity of NGF measured in PC12 assay

PC12 cells are pheochromocytoma cells expressing TrkA and have been widely used as a model system for NGF-induced differentiation. They respond to NGF by growth arrest and extension of long neurites and a phenotype similar to sympathetic neurons. In this experiment, a subclone of PC12 cells was used for testing bioactivity of NGF produced by transfected ARPE-19 clones. Recombinant β-hNGF (R&D systems #256-GF-100) was used as a positive control.

Parental ARPE-19 cells and NGF-producing clones were seeded in T25 flasks (750,000 cells/flask). The next day, cells were changed to fresh growth medium. After 24 h the conditioned medium was collected and added to the PC12 cells.

PC12 cells were cultured in Dulbecco's modified Eagle's medium (DMEM) with 4.5 g/l glucose and glutamax (Life Technologies #32430-027) with 7.5% donor horse serum (Life Technologies #16050-098) and 7.5% FBS (Life Technologies # 10099-141) in the presence of 5% CO₂ at 37°C. Medium was changed every 2-3 days and cells were subcultured 1:3 - 1:6 twice a week by tapping the flask and dispensing into new flasks.

PC12 cells were seeded for differentiation in 24-well dishes coated with collagen at a density of 15,000 cells/cm² (30,000/ml, 1 ml/well) in growth medium with serum. The next day, medium was changed to 1) ARPE-19 growth medium supplemented with recombinant human β-NGF, to give final concentrations of 0, 0.1, 0.2, 0.5, 1 and 2 nM, 2) conditioned medium from NGF-producing ARPE-19 clones (in different dilutions), 3) conditioned medium from parental ARPE-19 cells (undiluted), 4) ARPE-19 growth medium.
Cultures were inspected daily for neurite outgrowth and photos were taken after 3-5 days incubation.

Results: Conditioned medium from ARPE-19 clones transfected to overexpress NGF induced neurite outgrowth as expected.

### Example 7: Preparation of capsules for CNS use

Devices are fabricated from polysulphone (PS), or polyether sulfone (PES) or an equivalent polymer hollow fiber membrane with an outside diameter of 800-1000 µm and a wall thickness of approximately 100 µm. A porous scaffolding material consisting of polyvinyl alcohol (PVA) inserted into the membrane fiber cavity ensures proper cell distribution and attachment of the cell line. Finally, a tether fabricated from polyurethane (PU) or an equivalent material fixed to the device end provides a means for capsule retrieval post-implantation.

Capsules used for pre-clinical testing (in rats) are approximately 5 mm long. Capsules contemplated for implantation into human brains are approximately 20 mm long.

Cellular loading occurs through a hub segment and port attached to the hollow fiber device at the end distal to the tether (Figure 18). NGF cells prepared as a single-cell suspension are infused into the port, the hub segment is retrieved and the infusion hole is sealed with glue. For each mm length of the devices, approximately 10,000 NGF-expressing cells are loaded. The devices are maintained in media until use.

Capsules for implantation into rat brains were made with the following materials:
Membrane: Polysulphone hollow fiber membrane (PS90/700 from Minntech Corp, Minneapolis, Minnesota, USA), with a 90 kDA molecular weight cutoff. Dimensions: 700 µm +/-50 µm inner diameter, 100 µm +/- 20 µm wall. The hollow fiber was cut to lengths of approximately 5 mm.
Foam: PVA foam, product no. 160 LD from Hydrofera Inc, Cleveland, Ohio, USA. The PVA foam was cut to fit the inner diameter of the hollow fiber.
Load tube: Perfluoroalkoxy copolymer, .0037" +/- .0005" ID; .005" +/- .001" wall. From Zeus Industrial Products, Orangeburg, South Carolina, USA. The load tube is glued to the hollow fiber in one end and to the hub in the other end.
Hub: Product no P/N 02030200 Rev 1, from Abtec, Bristol, PA, USA.
Glue for gluing load tube to hub: Dymax 201-CTH from Diatom, Hvidovre, Denmark
Glue for hollow fiber: Dymax 1181-M from Diatom, Hvidovre, Denmark.
Capsules were assembled in a controlled environment, packaged in Falcon 15 mL polypropylene test tubes (Becton Dickinson, Cat #352096) and sterilised by injection of 70% ethanol and drying by sterile air in a flowhood, prior to filling with cells.

### Example 8: In vitro testing of encapsulated NGF-producing ARPE-19 cell clones

Cells were expanded in growth media as described above. The day before encapsulation, the cells were trypsinized, counted and re-seeded in growth media. Immediately before encapsulation, cells were trypsinized, counted, washed and resuspended in Human Endothelial Serum Free Media (HE-SFM, Invitrogen). The suspension was kept on ice through the experiment. Devices were made according to the specifications in Example 7. Cells were carefully injected into the device using a Hamilton syringe. For the experimental devices, 50,000 cells in 8 µl were injected in each device. After cell encapsulation and device sealing, the devices were transferred to HE-SFM. Remaining cells were seeded in growth media in conventional cell culture dishes to confirm cell viability. Throughout the experiment, the devices were maintained in HE-SFM at 37°C / 5% CO₂. Samples were taken at desired time points for NGF ELISA: Devices were washed in HE-SFM and subsequently incubated in 1 ml HE-SFM for 4 h. Released NGF was measured using the DuoSet Human NGF-ELISA (R&D systems). The amount of secreted NGF per device is shown in Figure 20.

At the end of the experiment, devices were fixed in paraformaldehyde, embedded and sectioned, and eosin/hematoxylin-stained using standard histology techniques (see Figure 19). Cell survival was determined from device sections. The device sections confirmed that in vitro survival was excellent.

### Example 9. Intrathecal implantation of capsules.

Intrathecal implantation can be accomplished along the spinal canal, preferably at the lumbar level below the conus medullaris. A small incision is made at the lumbar level, and a spinal needle is used to enter the intrathecal space. After CSF flow has been established, a guide wire is inserted into the intrathecal space and a dilator system is used to enter the space. The guidewire is withdrawn and the encapsulated device inserted into the space so that the active part is completely enclosed in the CSF compartment. The tether is secured to the lumbar fascia by a non-resorbable suture and preferably using a securing clip. The skin is closed using standard surgical procedures.

### Example 10. Implantation in the basal cholinergic forebrain.

Under general anesthesia or local anesthesia and sedation, a neurosurgical stereotactic frame is secured to the patient's head. A fiducial box and subsequent MRI imaging is applied to determine the anatomical area and implantation coordinates. The implantation can also be guided by diffusion tensor imaging and dose mapping, utilising custom software and navigational equipment supplied by BrainLAB AG. The patient is next brought to the operating room where he/she is prepped and draped. Based on the stereotactic image data a small skin incision is made frontolaterally and a small burrhole made through the skull. The dura and underlying meninges are penetrated by incision and a guide cannula with a trochar is inserted into the basal forebrain target area. The trochar is removed and the device is slided into position. The guide is removed and the device tether secured to the skull with a titanium plate or custom retaining clip. The skin is sutured closed with interrupted 3-0 Nylon suture. The procedure is repeated on the opposite side.

### Example 11. Preparation of capsules for ophthalmic use.

The encapsulation procedure is as follows: The hollow fibers are fabricated from polyether sulfone (PES) with an approximate outside diameter of 720 µm and a wall thickness of approximately 100 µm (AKZO-Nobel Wuppertal, Germany). These fibres are described in U.S. Pat. Nos. 4,976,859 and 4,968,733.
The devices comprise:
a semipermeable poly (ether sulfone) hollow fiber membrane fabricated by AKZO Nobel Faser AG;
a hub membrane segment;
a light cured methacrylate (LCM) resin leading end; and
a silicone tether.

The devices has a septal fixture at the proximal end for cellular loading access and are sealed at the distal end. NGF cells are prepared as a single-cell suspension and infused into the septal port at a density of 15K cells per µl after mixing 1:1 with physiologic collagen (Vitrogen: PC-1). After infusing 1.5 µl of the cellular suspension, the septum is removed, and the access port is sealed with LCM 23 resin. The components of the device are commercially available. The LCM glue is available from Ablestik Laboratories (Newark, Del.); Luxtrak Adhesives LCM23 and LCM24).

### Example 12 Implantation of Encapsulated Cells into the Sub-Tenon's Space (under Tenon's Capsule)

The patient is prepared and draped in the usual fashion after a retrobulbar injection of 3 cc 2% xylocaine is given to the eye. A speculum is inserted beneath the upper and lower lids. The operating microscope is brought into position. A perpendicular incision is made through both conjunctiva and Tenon's capsule in the superotemporal quadrant approximately 4 mm back from the limbus. The incision is extended approximately 4-5 mm back from the limbus. At that point, a blunt-tipped scissor is inserted through the incision and is used to bluntly dissect back an additional 5 mm or so on the scleral surface. At that point, a membrane device as described in Example 7 is placed in position through this incision to come to rest on the surface of the sclera. The end of the device that is closest to the limbus has a small loop that is attached to the cell-loaded device. At this point, a #10-0 nylon suture is passed through this loop and sutured into the superficial sclera to anchor the membrane to the sclera. At that point, both Tenon's capsule and the conjunctiva are closed with #6-0 plain gut sutures. The speculum is removed and the procedure is concluded.

### Example 13: Implantation of Encapsulated Cells into the Vitreous

The patient is prepared and draped in the usual fashion after a retrobulbar injection of 2% xylocaine is given to the eye. At that point, a speculum is inserted into the upper and lower lids and the microscope is brought into position. A small incision is made through both the conjunctiva and Tenon's capsule parallel to and approximately 4 mm from the limbus in the supranasal quadrant. The area exposed is cauterized with a wet-field cautery apparatus. A 3 mm incision is then made perpendicular to the limbus approximately 4 mm back from the limbus. The incision is made through the sclera and into the vitreous cavity with a #65 blade. Any of the vitreous which presents itself in the incision is cut away and removed. At this point, a membrane device as described in Example 7 is inserted through the incision into the vitreous cavity. At the end of the membrane, there is a small 2 mm loop that is attached to the membrane. The loop remains outside the sciera. The sclera is closed with interrupted #9-0 nylon sutures. The #9-0 nylon sutures are also used to anchor this loop of the device to the sclera. The conjunctiva is closed with #60 plain gut sutures.

### Example 14: In vivo testing of encapsulated NGF-producing ARPE-19 cell clones

14 days before implantation, cells were encapsulated as described in Example 8. Samples of clone #33 and clone #95 as well as parental ARPE-19 cells were encapsulated. Until implantation, the encapsulated cells were maintained in HE-SFM at 37°C / 5% CO₂. One lot of capsules was maintained in vitro for ten weeks. NGF-release (for 4 hours) was measured twice a week as described elsewhere. Medium was exchanged in connection with the NGF release measurements. After two weeks in vitro, one lot of capsules were implanted in rat brains.

Adult, 220 gram female Sprague-Dawley rats housed under 12 hours light:dark cycle and with free access to rat chow and water were used for implantation surgery. Devices were implanted intrastriatally in isofluoran anesthesised (1.5-2%) animals in the site described by the following coordinates with respect to bregma: AP: 0.0, ML: -3.0, DV: -7,5, TB: 0.0. Following surgery, assessment of general behaviour and weight was monitored every other weight. At 8 weeks post implantation animals were decapitated. Capsules were removed from the brain and rinsed once in PBS. The PBS was replaced with 1 ml Serum-Free Medium and incubated for NGF release measurement as described previously. Devices were subsequently fixed in 4% paraformaldehyde and processed for histological analysis by hematoxylin & eosin staining. The brain was dissected out and rinsed in cold saline for 1 minute before preparation in 4% paraformaldehyde and immersion fixation overnight at 4°C. Brains were then transferred into 25% sucrose/0.1M phosphate buffer and after 48 hours, 40 µm sections were cut on a freezing microtome. Brain sections were subsequently processed for general morphology (hematoxylin & eosin, cresyl violet) (Figure 24) and NGF immunohistochemistry (data not shown).

Weight and behaviour of the rats was normal and did not differ among the different treatments.

Results depicted in figure 24 show that cell survival after 8 weeks in normal rats was excellent for both NGF-secreting clones (Fig. 24a: clone #33; Fig 24b: clone #95). Results in figure 25 show that for the capsules maintained in vitro, NGF secretion increased markedly during the first four weeks, probably due to growth of cells within the capsules. From approximately week 4 and onwards the NGF levels were stable, between 0.5 and 1 ng/capsule for clone #33 and between 1 and 2 ng/capsule for clone #95. Capasules explanted after 8 weeks in vivo (explant) showed NGF levels at least as high as the levels form capsules maintained in vitro.

### SEQUENCES

| SEQ ID No | Type | Annotation | SEQ ID No in DK PA 2004 00064 |
|---|---|---|---|
| 1 | N | hpreproNGF | 1 |
| 2 | P | hpreproNGF | 2 |
| 3 | P | mpreproNGF | 3 |
| 4 | P | rpreproNGF | 4 |
| 5 | P | chimppreproNGF | 5 |
| 6 | P | pigpreproNGF (partial) | 6 |
| 7 | P | panNGF (Fig 15a) | |
| 8 | P | panNGF (Fig 15b) | |
| 9 | P | NGF130 (Fig 16a, top) | |
| 10 | P | NGF131 (Fig 16a, bottom) | |
| 11 | P | NGFR2 (Fig 16b, top) | |
| 12 | P | NGFR3 (Fig 16b, bottom) | |
| 13 | P | p75lessNGF (Fig 17) | |
| 14 | N | pCIn.hNGF martial | 7 |
| 15 | P | N-term. of hNGF | |
| 16 | N | pCIn.KhNGF partial | 8 |
| 17 | P | N-term. of hNGF | |
| 18 | N | EF1a.KhNGF partial | 9 |
| 19 | P | N-term. of hNGF | |
| 20 | N | UbC.hNGF | 10 |
| 21 | P | N-term. of hNGF | |
| 22 | N | rINSintrA.hNGF | 11 |
| 23 | P | N-term. of hNGF | |
| 24 | N | hNGFs+BamHI primer | |
| 25 | N | hNGFas+Xhol primer | |
| 26 | N | NGF+Kzk s primer | |
| 27 | N | NGF 457as primer | |
| 28 | N | rINSintrA 5' primer | |
| 29 | N | rINSintrA 3' primer | |

## Claims

1. A human cell line comprising a stably integrated expression construct comprising the following sequences:
a mammalian promoter,
a coding sequence operably linked to said promoter, wherein said coding sequence codes for NGF, and
an intron located in the transcript.

2. The cell line according to claim 1, being a monoclonal cell line.

3. The cell line of claim 1, being a polyclonal cell line.

4. The cell line of claim 1, originating from a primary culture.

5. The cell line of any of the preceding claims, wherein the cell line has not been immortalised by insertion of a heterologous immortalisation gene.

6. The cell line of any of the preceding claims, wherein the cell line is contact-inhibited.

7. The cell line of any of the preceding claims being non-tumourigenic in mammals.

8. The cell line of any of the preceding claims, being able to survive at low oxygen tension.

9. The cell line of any of the preceding claims, originating from a primary culture.

10. The cell line of any of the preceding claims, being capable of undergoing at least 50 doublings.

11. The cell line of any of the preceding claims, triggering a low level of human host immune reaction.

12. The cell line of any of the preceding claims, further comprising a suicide construct under the control of an inducible and/or constitutive promoter.

13. The cell line of any of the preceding claims, further comprising a construct comprising a promoter operatively linked to a polynucleotide sequence encoding a fusion protein comprising an immunostimulatory cell surface protein linked at the amino terminus to a second cell surface polypeptide, wherein the second cell surface polypeptide comprises a transmembrane region, wherein upon expression, the fusion protein is expressed on the cell surface.

14. The cell line of any of the preceding claims, wherein the intron is less than 10,000 bp long.

15. The cell line of any of the preceding claims, wherein the intron is more than 50 bp long.

16. The cell line of any of the preceding claims, wherein said intron is of eukaryotic origin.

17. The cell line of claim 16, wherein said intron is of mammalian origin.

18. The cell line of claim 17, wherein said intron is of rodent, primate, or human origin.

19. The cell line of any of the preceding claims, wherein said intron is placed in the 5' UTR of the transcript.

20. The cell line of any of the preceding claims 1 to 18, wherein said intron is placed inside the sequence coding for NGF.

21. The cell line of claim 20, wherein the intron is placed in the part of the coding sequence closest to the start codon.

22. The cell line of any of the preceding claims, wherein said intron is derived from a first intron.

23. The cell line of claim 1, wherein said intron is an insulin intron or comprises a sequence having at least 50% sequence identity to bases no. 747-865 of SEQ ID No 22.

24. The cell line of claim 1, wherein said intron is the intron from the pCI vector (bases no 890 to 1022 of SEQ ID No 14) or comprises a sequence having at least 50% sequence identity to bases no 890 to 1022 of SEQ ID No 14.

25. The cell line of claim 1, wherein said intron is a ubiquitin promoter intron or comprises a sequence having at least 50% sequence identity to bases no 736-1547 of SEQ ID No 20.

26. The cell line of claim 1, wherein said intron is an EF1 alpha intron (bases no 609-1551 of SEQ ID No 18) or comprises a sequence having at least 50% sequence identity to bases no 609-1551 of SEQ ID No 18.

27. The cell line of any of the preceding claims, wherein the sequence coding for NGF comprises a sequence coding for a functional NGF propeptide having at least 60% sequence identity to the prodomain of SEQ ID No. 2.

28. The cell line of any of the preceding claims, wherein the sequence coding for NGF comprises a sequence selected from the group consisting of:
(a) human betaNGF coding sequence (SEQ ID No 1);
(b) rat, mouse, or pig betaNGF (preproNGF) coding sequence;
(c) chimpanzee betaNGF (preproNGF) coding sequence;
(d) a sequence coding for mature human (SEQ ID No 2), rat (SEQ ID No 4), mouse (SEQ ID No 3), pig (SEQ ID No 6) or chimpanzee betaNGF (SEQ ID No 5);
(e) a sequence coding for bioactive NGF variant comprising a mature sequence having at least 50% sequence identity to the mature part of SEQ ID No 2.

29. The cell line of any of the preceding claims, wherein the NGF coding sequence comprises a sequence coding for mature human NGF (mature part of SEQ ID No 2).

30. The cell line of any of the preceding claims, wherein the NGF coding sequence codes for an NGF molecule wherein the mature part is selected from the group consisting of:
(a) a mature NGF molecule wherein the 25-35 region is replaced by the 25-35 region from BDNF;
(b) a mature NGF molecule wherein the 94-98 aa region is replaced by the 94-98 region from BDNF;
(c) a mature NGF molecule wherein the 23-33 region is replace by the 21-33 region from BDNF;
(d) a mature NGF molecule wherein the 92-101 region is replaced by the 92-101 region from BDNF;
(e) a mature NGF molecule wherein one or more positively charged amino acids in amino acids 30-34 and/or 94-98 are replaced by uncharged or negatively charged amino acids;
(f) a modified mature NGF molecule with reduced ability to bind p75^{NGFR};
(g) a modified mature NGF molecule having amino acid substitutions at positions: G23, H84 and either V18 or V20;
(h) a modified mature NGF molecule comprising at least the N-terminal 117 amino acids of mature NGF and having amino acid substitions at positions G23, H84, v18 and V20 and either H84 or T81.

31. The cell line of any of the preceding claims, wherein the NGF coding sequence codes for mature NGF with a heterologous signal peptide.

32. The cell line of any of the preceding claims, wherein the NGF coding sequence codes for a mature NGF with a heterologous pro-peptide.

33. The cell line of any of the preceding claims, wherein the stably integrated construct further comprises a sequence selected from the group consisting of: Kozak consensus sequence, WPRE, β-globin insulator, SP163 enhancer, non-translated 5' or 3' prime regions from the tau, TH or APP genes.

34. The cell line of any of the preceding claims, where the promotor is a constitutive promotor.

35. The cell line of claim 34, wherein said promotor is selected from the group consisting of CMV, human UbiC, JeT, RSV, EF-1alpha.

36. The cell line of any of the preceding claims 1 to 33, where the promotor is an inducible promotor.

37. The cell line of claim 36, where the inducible promoter is selected from the group consisting of Tet-regulatable promoter, Mo-MLV-LTR, Mx1, progesteron, RU486.

38. The cell line of any of the preceding claims, wherein the stably integrated construct is a mammalian plasmid expression vector.

39. The cell line of claim 38, wherein said expression vector is selected from the group consisting of: pCI, pSI, pNS, pUbi1z.

40. The cell line of any of the preceding claims 1 to 34, wherein said stably integrated construct is a virus vector.

41. The cell line of claim 40, wherein said virus vector is derived from the Retroviridae family including lentivirus, HIV, SIV, FIV, EAIV, CIV.

42. The cell line of claim 40, being selected from the group consisting of alphavirus, adenovirus, adeno associated virus, baculovirus, HSV, coronavirus, Bovin papiloma virus, Mo-MLV.

43. The cell line of any of the preceding claims, being derived from an epithelial cell.

44. The cell line of claim 43, being derived from a retinal pigment epithelial (RPE) cell, including but not limited to primary RPE cells.

45. The cell line of claim 44, being an ARPE-19 derived cell line.

46. The cell line of claim 45, having accession number DSM ACC2706.

47. The cell line of claim 45, having accession number DSM ACC2707.

48. The cell line of any of the preceding claims 1 to 42, being selected from the group consisting of: astrocyte cell lines, human mesencephalic cell line, and human endothelial cell line.

49. The cell line of any of the preceding claims, being able to maintain the production of a biologically active NGF at a level sufficient to maintain its useful biological activity for a period greater than one month.

50. The cell line according to any of the preceding claims, being capable of secreting in excess of 0.5 ng biologically active NGF per 10⁵ cells per 24 hours.

51. An implantable cell culture device, the device comprising:
(a) a semipermable membrane permitting the diffusion of NGF therethrough; and
(b) a population of cell from the cell line according to any of claims 1 to 50 disposed within the semipermeable membrane.

52. The device of claim 51, being capable of secreting in excess of 0.1 ng biologically active NGF per 24 hours.

53. The device of claim 51, comprising cells obtainable from the cell line having accession number DSM ACC2706.

54. The device of claim 51, comprising cells obtainable from the cell line having accession number DSM ACC2707.

55. The device of claim 51, wherein the semipermeable membrane is immunoisolatory.

56. The device of claim 51, wherein the semipermeable membrane is microporous.

57. The device of claim 56, wherein the molecular weight cutoff of the membrane is 1000 kD or less.

58. The device of any of the preceding claims 51 to 57, comprising at least 10³ cells.

59. The device of any of the preceding claims 51 to 58, wherein the volume of the device is volume 1-10 µL.

60. The device of any of the preceding claims 51 to 59, wherein the length is at least 1 mm.

61. The device of any of the preceding claims 51 to 60, wherein thickness is up to 1000 µm depth.

62. The device of any of the preceding claims 51 to 61, wherein the thickness of the jacket is 2-200 microns.

63. The device of any of the preceding claims 51 to 62, wherein the device further comprises a tether anchor.

64. The device of any of the preceding claims 51 to 63, wherein the device comprises a core comprising a reticulate foam scaffold having interconnected pores, the pore density of the foam varying between 20% and 90%, the cells being dispersed in the pores.

65. The device of claim 64, wherein the foam scaffold is a thermoplastic or a thermoplastic elastomer.

66. Use of the device of any of the claims 51 to 65, for the preparation of a medicament.

67. The use of claim 66, for the treatment of a neurological disorder.

68. The use of claim 67, wherein the neurological disorder is Alzheimer's disease, Huntingdons' disease, CNS trauma, ALS, Parkinson's Disease, peripheral neuropathy, neuropathic pain, and other conditions **characterised by** necrosis or loss of neurons or their processes, whether central or peripheral.

69. The use of claim 66, wherein the eye disorder is selected from the group consisting of: diabetic retinopathy, uveitis, retinitis pigmentosa, age related macular degeneration, glaucoma, ocular neovascularisation, retinitis, and corneal wounds.

70. A pharmaceutical composition comprising a cell line according to any of the preceding claims 1 to 50 attached to a matrix.

71. The composition of claim 70, wherein the matrix is selected from the group consisting of glass and other silicon oxides, polystyrene, polypropylene, polyethylene, polyvinylidene fluoride, polyurethane, polyalginate, polysulphone, polyvinyl alcohol, acrylonitrile polymers, polyacrylamide, polycarbonate, polypentent, nylon, amylases, natural and modified gelatin and natural and modified collagen, natural and modified polysaccharides, including dextrans and celluloses (e.g., nitrocellulose), agar, and magnetite.

72. The composition of claim 70, wherein the solid matrix may be coated on its external surface with factors promoting cell adhesion, growth or survival, including but not limited to cell adhesion molecules, extracellular matrix, fibronectin, laminin, collagen, elastin, glycosaminoglycans, or proteoglycans or growth factors.

73. The composition of claim 70, wherein growth- or survival promoting factor or factors are incorporated into the matrix material.

74. The composition of claim 70, wherein the configuration of the support is spherical, cylindrical, elliptical, a flat sheet or strip, a needle or pin shape, and the like.

75. The composition of claim 74, wherein the bead sizes ranges from about 10 µm to 1 mm in diameter.

## Patentansprüche

1. Humane Zelllinie, umfassend ein stabil integriertes Expressionskonstrukt, das die folgenden Sequenzen umfasst:
einen Säugetierpromotor,
eine kodierende Sequenz, die funktionsfähig mit dem Promotor verbunden ist, wobei die kodierende Sequenz NGF kodiert, und
ein Intron, das sich in dem Transkript befindet.

2. Zelllinie nach Anspruch 1, die eine monoklonale Zelllinie ist.

3. Zelllinie nach Anspruch 1, die eine polyklonale Zelllinie ist.

4. Zelllinie nach Anspruch 1, die aus einer Primärkultur entstanden ist.

5. Zelllinie nach einem der vorstehenden Ansprüche, worin die Zelllinie nicht durch Einfügen eines heterologen Immortalisierungsgens immortalisiert wurde.

6. Zelllinie nach einem der vorstehenden Ansprüche, worin die Zelllinie kontaktinhibiert ist.

7. Zelllinie nach einem der vorstehenden Ansprüche, die in Säugetieren nicht onkogen ist.

8. Zelllinie nach einem der vorstehenden Ansprüche, die in der Lage ist bei niedrigem Sauerstoffpartialdruck zu überleben.

9. Zelllinie nach einem der vorstehenden Ansprüche, die aus einer Primärkultur entstanden ist.

10. Zelllinie nach einem der vorstehenden Ansprüche, die in der Lage ist, mindestens 50 Verdoppelungen zu durchlaufen.

11. Zelllinie nach einem der vorstehenden Ansprüche, die bei einem humanen Wirt einen geringen Grad an Immunreaktion auslöst.

12. Zelllinie nach einem der vorstehenden Ansprüche, weiter umfassend ein Selbstmord-Konstrukt unter der Steuerung eines induzierbaren und/oder konstitutiven Promotors.

13. Zelllinie nach einem der vorstehenden Ansprüche, weiter umfassend ein Konstrukt, das einen Promotor umfasst, der mit einer ein Fusionsprotein kodierenden Polynukleotidsequenz funktionsfähig verbunden ist, das ein immunstimulatorisches Zelloberflächenprotein umfasst, welches mit dem Aminoterminus mit einem zweiten Zelloberflächenpolypeptid verbunden ist, worin das zweite Zelloberflächenpolypeptid eine Transmembranregion umfasst, worin bei Expression das Fusionsprotein an der Zelloberfläche exprimiert wird.

14. Zelllinie nach einem der vorstehenden Ansprüche, worin das Intron kürzer als 10.000 bp ist.

15. Zelllinie nach einem der vorstehenden Ansprüche, worin das Intron länger als 50 bp ist.

16. Zelllinie nach einem der vorstehenden Ansprüche, worin das Intron von eukaryotischer Herkunft ist.

17. Zelllinie nach Anspruch 16, worin das Intron von Säugetier Herkunft ist.

18. Zelllinie nach Anspruch 17, worin das Intron von Nager-, Primaten- oder humaner Herkunft ist.

19. Zelllinie nach einem der vorstehenden Ansprüche, worin das Intron in der 5'UTR des Transkripts angeordnet ist.

20. Zelllinie nach einem der vorstehenden Ansprüche 1 bis 18, worin das Intron innerhalb der NGF kodierenden Sequenz angeordnet ist.

21. Zelllinie nach Anspruch 20, worin das Intron in dem Teil der kodierenden Sequenz angeordnet ist, der dem Startcodon am nächsten ist.

22. Zelllinie nach einem der vorstehenden Ansprüche, worin das Intron von einem ersten Intron abgeleitet ist.

23. Zelllinie nach Anspruch 1, worin das Intron ein Insulinintron ist oder eine Sequenz umfasst, die mindestens 50 % Sequenzübereinstimmung mit Basen Nr. 747-865 der SEQ ID NO: 22 aufweist.

24. Zelllinie nach Anspruch 1, worin das Intron das Intron des pCl Vektors (Basen Nr. 890 bis 1022 von SEQ ID NO: 14) ist oder eine Sequenz umfasst, die mindestens 50 % Sequenzübereinstimmung mit Basen Nr. 890 bis 1022 von SEQ ID NO: 14 aufweist.

25. Zelllinie nach Anspruch 1, worin das Intron ein Ubiquitinpromotor-Intron ist oder eine Sequenz umfasst, die mindestens 50 % Sequenzübereinstimmung mit Basen Nr. 736-1547 von SEQ ID NO: 20 aufweist.

26. Zelllinie nach Anspruch 1, worin das Intron ein EF1alpha Intron (Basen Nr. 609-1551 von SEQ ID NO: 18) ist oder eine Sequenz umfasst, die mindestens 50 % Sequenzübereinstimmung mit Basen Nr. 609-1551 von SEQ ID NO: 18 aufweist.

27. Zelllinie nach einem der vorstehenden Ansprüche, worin die NGF kodierende Sequenz eine Sequenz umfasst, die ein funktionales NGF-Propeptid kodiert, das mindestens 60 % Sequenzübereinstimmung mit der Prodomäne von SEQ ID NO: 2 aufweist.

28. Zelllinie nach einem der vorstehenden Ansprüche, worin die NGF kodierende Sequenz eine Sequenz umfasst, die ausgewählt ist aus der Gruppe bestehend aus:
(a) humanes betaNGF kodierende Sequenz (SEQ ID NO: 1),
(b) Ratten, Mäuse oder Schweine betaNGF (preproNGF) kodierende Sequenz,
(c) Schimpansen betaNGF (preproNGF) kodierende Sequenz,
(d) eine Sequenz, die voll entwickeltes humanes (SEQ ID NO: 2), Ratten (SEQ ID NO: 4), Mäuse (SEQ ID NO: 3), Schweine (SEQ ID NO: 6) oder Schimpansen betaNGF (SEQ ID NO: 5) kodiert,
(e) eine Sequenz, die eine bioaktive NGF Variante kodiert, umfassend eine voll entwickelte Sequenz mit einer Sequenzübereinstimmung von mindestens 50% zu dem voll entwickelten Teil von SEQ ID NO: 2.

29. Zelllinie nach einem der vorstehenden Ansprüche, worin die NGF kodierende Sequenz eine Sequenz umfasst, die voll entwickeltes humanes NGF (voll entwickeltes Teil von SEQ ID NO: 2) kodiert.

30. Zelllinie nach einem der vorstehenden Ansprüche, worin die NGF kodierende Sequenz ein NGF Molekül kodiert, worin der voll entwickelte Teil ausgewählt ist aus der Gruppe bestehend aus:
(a) einem voll entwickelten NGF Molekül, worin die 25-35 Region durch die 25-35 Region von BDNF ersetzt ist,
(b) einem voll entwickelten NGF Molekül, worin die 94-98 AS Region durch die 94-98 Region von BDNF ersetzt ist,
(c) einem voll entwickelten NGF Molekül, worin die 23-33 Region durch die 21-33 Region von BDNF ersetzt ist,
(d) einem voll entwickelten NGF Molekül, worin die 92-101 Region durch die 92-101 Region von BDNF ersetzt ist,
(e) einem voll entwickelten NGF Molekül, worin eine oder mehrere positiv geladenen Aminosäuren in Aminosäuren 30-34 und/oder 94-98 durch ungeladene oder negativ geladene Aminosäuren ersetzt sind,
(f) einem modifizierten voll entwickelten NGF Molekül mit verminderter Fähigkeit p75^{NGFR} zu binden,
(g) einem modifizierten voll entwickelten NGF Molekül mit Aminosäurensubstitutionen an Positionen: G23, H84 und entweder V18 oder V20,
(h) einem modifizierten voll entwickelten NGF Molekül, das mindestens die N-terminalen 117 Aminosäuren von voll entwickeltem NGF umfasst und Aminosäurensubstitutionen an Positionen G23, H84, V18 und V20 und entweder H84 oder T81 aufweist.

31. Zelllinie nach einem der vorstehenden Ansprüche, worin die NGF kodierende Sequenz ein voll entwickeltes NGF mit einem heterologem Signalpeptid kodiert.

32. Zelllinie nach einem der vorstehenden Ansprüche, worin die NGF kodierende Sequenz ein voll entwickeltes NGF mit einem heterologem Pro-Peptid kodiert.

33. Zelllinie nach einem der vorstehenden Ansprüche, worin das stabil integrierte Konstrukt weiter umfasst, eine Sequenz ausgewählt aus der Gruppe bestehend aus: Kozak Consensus Sequenz, WPRE, β-Globin Isolator, SP-163 Verstärker, nicht translatierte 5' oder 3' Prime-Regionen von tau, TH oder APP Genen.

34. Zelllinie nach einem der vorstehenden Ansprüche, worin der Promotor ein kostitutiver Promotor ist.

35. Zelllinie nach Anspruch 34, worin der Promotor ausgewählt ist aus der Gruppe bestehend aus CMV, humaner UbiC, JeT, RSV, EF-1alpha.

36. Zelllinie nach einem der vorstehenden Ansprüche 1 bis 33, worin der Promotor ein induzierbarer Promotor ist.

37. Zelllinie nach Anspruch 36, worin der induzierbare Promotor ausgewählt ist aus der Gruppe bestehend aus Tet-steuerbarem Promotor, Mo-MLV-LTR, Mx1, Progesteron, RU486.

38. Zelllinie nach einem der vorstehenden Ansprüche, worin das stabil integrierte Konstrukt ein Säugetier Plasmid-Expressionsvektor ist.

39. Zelllinie nach Anspruch 38, worin der Expressionsvektor ausgewählt ist aus der Gruppe bestehend aus: pCI, pSI, pNS, pUbilz.

40. Zelllinie nach einem der vorstehenden Ansprüche 1 bis 34, worin das stabil integrierte Konstrukt ein Virusvektor ist.

41. Zelllinie nach Anspruch 40, worin der Virusvektor von der Retroviridae Familie abgeleitet ist, einschließlich Lentivirus, HIV, SIV, FIV, EAIV, CIV.

42. Zelllinie nach Anspruch 40, die ausgewählt ist aus der Gruppe bestehend aus Alphavirus, Adenovirus, Adeno-assoziiertes Virus, Baculovirus, HSV, Coronavirus, Rinderpapilomavirus, Mo-MLV.

43. Zelllinie nach einem der vorstehenden Ansprüche, die von einer Epithelzelle abgeleitet ist.

44. Zelllinie nach Anspruch 43, die von einer Netzhautpigment-Epithelzelle (RPE) abgeleitet ist, einschließlich aber nicht begrenzt auf primäre RPE Zellen.

45. Zelllinie nach Anspruch 44, die eine von ARPE-19 abgeleitete Zelllinie ist.

46. Zelllinie nach Anspruch 45, die die Hinterlegungsnummer DSM ACC2706 aufweist.

47. Zelllinie nach Anspruch 45, die die Hinterlegungsnummer DSM ACC2707 aufweist.

48. Zelllinie nach einem der vorstehenden Ansprüche 1 bis 42, die ausgewählt ist aus der Gruppe bestehend aus Astrozyten Zelllinien, humaner mesencephaler Zelllinie und humaner endothelialer Zelllinie.

49. Zelllinie nach einem der vorstehenden Ansprüche, die in der Lage ist, die Herstellung eines biologisch aktiven NGFs auf einem Niveau aufrecht zu erhalten, das ausreicht, seine nützliche biologische Aktivität für einen Zeitraum größer als einen Monat aufrecht zu erhalten.

50. Zelllinie nach einem der vorstehenden Ansprüche, die in der Lage ist, biologisch aktives NGF über 0,5 ng hinaus pro 10⁵ Zellen pro 24 Stunden zu sezernieren.

51. Implantierbare Zellkultureinrichtung, umfassend:
(a) eine semipermeable Membran, die die Diffusion von NGF **dadurch** erlaubt, und
(b) eine Zellpopulation aus der Zelllinie nach einem der Ansprüche 1 bis 50, die innerhalb der semipermeablen Membran angeordnet ist.

52. Einrichtung nach Anspruch 51, die in der Lage ist, biologisch aktives NGF über 0,1 ng hinaus pro 10⁵ Zellen pro 24 Stunden zu sezernieren.

53. Einrichtung nach Anspruch 51, umfassend Zellen, die aus der Zelllinie mit der Hinterlegungsnummer DSM ACC2706 erhältlich sind.

54. Einrichtung nach Anspruch 51, umfassend Zellen, die aus der Zelllinie mit der Hinterlegungsnummer DSM ACC2707 erhältlich sind.

55. Einrichtung nach Anspruch 51, worin die semipermeable Membran immunisolatorisch ist.

56. Einrichtung nach Anspruch 51, worin die semipermeable Membran mikroporös ist.

57. Einrichtung nach Anspruch 56, worin die Molekulargewichts-Obergrenze der Membran 1000 kDa oder weniger ist.

58. Einrichtung nach einem der vorstehenden Ansprüche 51 bis 57, umfassend mindestens 10³ Zellen.

59. Einrichtung nach einem der vorstehenden Ansprüche 51 bis 58, worin das Volumen der Einrichtung das Volumen 1-10 µl beträgt.

60. Einrichtung nach einem der vorstehenden Ansprüche 51 bis 59, worin die Länge mindestens 1 mm beträgt.

61. Einrichtung nach einem der vorstehenden Ansprüche 51 bis 60, worin die Dicke bis zu 1000 µm tief ist.

62. Einrichtung nach einem der vorstehenden Ansprüche 51 bis 61, worin die Dicke des Mantels 2-200 µm beträgt.

63. Einrichtung nach einem der vorstehenden Ansprüche 51 bis 62, worin die Einrichtung weiterhin einen Halteanker umfasst.

64. Einrichtung nach einem der vorstehenden Ansprüche 51 bis 63, worin die Einrichtung einen Kern umfasst, der ein netzförmiges Schaumgerüst umfasst, das miteinander verbundene Poren aufweist, wobei die Porendichte des Schaums zwischen 20 % und 90 % variiert und die Zellen in den Poren verteilt sind.

65. Einrichtung nach Anspruch 64, worin das Schaumgerüst ein Thermoplast oder ein thermoplastisches Elastomer ist.

66. Verwendung der Einrichtung nach einem der Ansprüche 51 bis 65 zur Herstellung eines Medikaments.

67. Verwendung nach Anspruch 66 für die Behandlung einer neurologischen Störung.

68. Verwendung nach Anspruch 67, wobei die neurologische Störung Alzheimersche Krankheit, Huntingtonsche Krankheit, Trauma des zentralen Nervensystems, ALS, Parkinson Erkrankung, periphere Neuropathie, neuropathische Schmerzen und andere Zustände ist, die durch Nekrose oder Verlust von Neuronen oder deren Prozessen, entweder zentral oder peripher, **gekennzeichnet** ist.

69. Verwendung nach Anspruch 66, wobei die Augenstörung ausgewählt ist aus der Gruppe bestehend aus: diabetische Retinopathie, Entzündung der Uvea, Retinitis pigmentosa, mit dem Alter verbundene Makuladegeneration, Glaukom, okulare Neovaskularisierung, Retinitis und korneale Verletzungen.

70. Pharmazeutische Zusammensetzung umfassend eine Zelllinie nach einem der vorstehenden Ansprüche 1 bis 50, die an eine Matrix angefügt ist.

71. Zusammensetzung nach Anspruch 70, worin die Matrix ausgewählt ist aus der Gruppe bestehend aus Glas und anderen Siliziumoxiden, Polystyrol, Polypropylen, Polyethylen, Polyvinyliden-Fluorid, Polyurethan, Polyalginat, Polysulfon, Polyvinyl-Alkohol, Acrylonitril-Polymere, Polyacrylamid, Polycarbonat, Polypentent, Nylon, Amylasen, natürlicher und veränderter Gelatine und natürlichem und veränderten Kollagen, natürlichen und veränderten Polysacchariden, einschließlich Dextrane und Zellulosen (beispielsweise Nitrozellulose), Agar und Magnetit.

72. Zusammensetzung nach Anspruch 70, worin die feste Matrix auf ihrer externen Oberfläche mit Faktoren beschichtet ist, die die Zelladhäsion, Wachstum oder Überleben fördern, einschließlich aber nicht begrenzt auf Zelladhäsionsmoleküle, extrazelluläre Matrix, Fibronektin, Laminin, Kollagen, Elastin, Glycosaminoglykane oder Proteoglykane oder Wachstumsfaktoren.

73. Zusammensetzung nach Anspruch 70, worin der wachstum- oder überlebensfördernde Faktor oder Faktoren in dem Matrixmaterial aufgenommen sind.

74. Zusammensetzung nach Anspruch 70, worin die Konfiguration der Auflagerung kugelförmig, zylindrisch, elliptisch, ein flaches Blatt oder Streifen, eine Nadel oder Stiftförmig und ähnliches ist.

75. Zusammensetzung nach Anspruch 74, worin die Kügelchengrößen im Durchmesser von ungefähr 10 µm bis zu 1 mm reichen.

## Revendications

1. Lignée cellulaire humaine comprenant une construction d'expression intégrée de manière stable comprenant les séquences suivantes :
un promoteur de mammifère,
une séquence codante liée de manière opérationnelle audit promoteur, dans laquelle ladite séquence codante code pour le facteur de croissance du tissu nerveux (NGF), et
un intron localisé dans le produit de la transcription.

2. Lignée cellulaire selon la revendication 1, qui est une lignée cellulaire monoclonale.

3. Lignée cellulaire selon la revendication 1, qui est une lignée cellulaire polyclonale.

4. Lignée cellulaire selon la revendication 1, ayant pour origine une culture primaire.

5. Lignée cellulaire selon l'une quelconque des revendications précédentes, dans laquelle la lignée cellulaire n'a pas été immortalisée par insertion d'un gène d'immortalisation hétérologue.

6. Lignée cellulaire selon l'une quelconque des revendications précédentes, dans laquelle la lignée cellulaire est inhibée par contact.

7. Lignée cellulaire selon l'une quelconque des revendications précédentes, qui est non oncogène chez les mammifères.

8. Lignée cellulaire selon l'une quelconque des revendications précédentes, qui est capable de survivre à une basse tension d'oxygène.

9. Lignée cellulaire selon l'une quelconque des revendications précédentes, ayant pour origine une culture primaire.

10. Lignée cellulaire selon l'une quelconque des revendications précédentes, qui est capable de subir au moins 50 duplications.

11. Lignée cellulaire selon l'une quelconque des revendications précédentes, qui déclenche un bas niveau de réaction immunitaire de l'hôte humain.

12. Lignée cellulaire selon l'une quelconque des revendications précédentes, comprenant en outre une construction suicide sous le contrôle d'un promoteur inductible et/ou constitutif.

13. Lignée cellulaire selon l'une quelconque des revendications précédentes, comprenant en outre une construction comprenant un promoteur lié de manière opérationnelle à une séquence polynucléotidique codant pour une protéine de fusion comprenant une protéine de surface cellulaire immunostimulante liée au niveau du N-terminal à un second polypeptide de surface cellulaire, dans laquelle le second polypeptide de surface cellulaire comprend une région transmembranaire, dans laquelle dès l'expression, la protéine de fusion est exprimée à la surface cellulaire.

14. Lignée cellulaire selon l'une quelconque des revendications précédentes, dans laquelle l'intron a une longueur inférieure à 10 000 paires de bases.

15. Lignée cellulaire selon l'une quelconque des revendications précédentes, dans laquelle l'intron a une longueur supérieure à 50 paires de bases.

16. Lignée cellulaire selon l'une quelconque des revendications précédentes, dans laquelle ledit intron est d'origine eucaryote.

17. Lignée cellulaire selon la revendication 16, dans laquelle ledit intron est d'origine mammifère.

18. Lignée cellulaire selon la revendication 17, dans laquelle ledit intron a pour origine un rongeur, un primate, ou un être humain.

19. Lignée cellulaire selon l'une quelconque des revendications précédentes, dans laquelle ledit intron est placé dans la séquence non traduite en 5' (5'-UTR) du transcript.

20. Lignée cellulaire selon l'une quelconque des revendications précédentes 1 à 18, dans laquelle ledit intron est placé à l'intérieur de la séquence codant pour le facteur de croissance du tissu nerveux (NGF).

21. Lignée cellulaire selon la revendication 20, dans laquelle l'intron est placé dans la partie de la séquence codante la plus proche du codon d'initiation.

22. Lignée cellulaire selon l'une quelconque des revendications précédentes, dans laquelle ledit intron est dérivé d'un premier intron.

23. Lignée cellulaire selon la revendication 1, dans laquelle ledit intron est un intron d'insuline ou comprend une séquence ayant au moins 50 % d'identité de séquence avec les bases n° 747-865 de SEQ ID N° 22.

24. Lignée cellulaire selon la revendication 1, dans laquelle ledit intron est l'intron provenant du vecteur pCI (bases n° 890 à 1 022 de SEQ ID N° 14) ou comprend une séquence ayant au moins 50 % d'identité de séquence avec les bases n° 890 à 1 022 de SEQ ID N° 14.

25. Lignée cellulaire selon la revendication 1, dans laquelle ledit intron est un intron de promoteur d'ubiquitine ou comprend une séquence ayant au moins 50 % d'identité de séquence avec les bases n° 736-1 547 de SEQ ID N° 20.

26. Lignée cellulaire selon la revendication 1, dans laquelle ledit intron est un intron EF1alpha (bases n° 609-1 551 de SEQ ID N° 18) ou comprend une séquence ayant au moins 50 % d'identité de séquence avec les bases n° 609-1 551 de SEQ ID N° 18.

27. Lignée cellulaire selon l'une quelconque des revendications précédentes, dans laquelle la séquence codant pour le facteur de croissance du tissu nerveux (NGF) comprend une séquence codant pour un pro-peptide NGF fonctionnel ayant au moins 60 % d'identité de séquence avec le pro-domaine de SEQ ID N° 2.

28. Lignée cellulaire selon l'une quelconque des revendications précédentes, dans laquelle la séquence codant pour le facteur de croissance du tissu nerveux (NGF) comprend une séquence choisie dans le groupe constitué par :
(a) une séquence codant pour le facteur NGF-bêta humain (SEQ ID N° 1) ;
(b) une séquence codant pour le facteur NGF-bêta (pré-pro-NGF) de rat, de souris, ou de porc ;
(c) une séquence codant pour le facteur NGF-bêta (pré-pro-NGF) de chimpanzé ;
(d) une séquence codant pour le facteur NGF-bêta mature humain (SEQ ID N° 2), de rat (SEQ ID N° 4), de souris (SEQ ID N° 3), de porc (SEQ ID N° 6), ou de chimpanzé (SEQ ID N° 5) ;
(e) une séquence codant pour une variante NGF bioactive comprenant une séquence mature ayant au moins 50 % d'identité de séquence avec la partie mature de SEQ ID N° 2.

29. Lignée cellulaire selon l'une quelconque des revendications précédentes, dans laquelle la séquence codant pour le facteur de croissance du tissu nerveux (NGF) comprend une séquence codant pour le facteur de croissance du tissu nerveux (NGF) humain mature (partie mature de SEQ ID N° 2).

30. Lignée cellulaire selon l'une quelconque des revendications précédentes, dans laquelle la séquence codant pour le facteur de croissance du tissu nerveux (NGF) code pour une molécule NGF dans laquelle la partie mature est choisie dans le groupe constitué par :
(a) une molécule NGF mature dans laquelle la région 25-35 est remplacée par la région 25-35 provenant du facteur neurotrophique dérivé du cerveau (BDNF) ;
(b) une molécule NGF mature dans laquelle la région aa 94-98 est remplacée par la région 94-98 provenant du facteur neurotrophique dérivé du cerveau (BDNF) ;
(c) une molécule NGF mature dans laquelle la région 23-33 est remplacée par la région 21-33 provenant du facteur neurotrophique dérivé du cerveau (BDNF) ;
(d) une molécule NGF mature dans laquelle la région 92-101 est remplacée par la région 92-101 provenant du facteur neurotrophique dérivé du cerveau (BDNF) ;
(e) une molécule NGF mature dans laquelle un ou plusieurs acides aminés chargés positivement dans les acides aminés 30-34 et/ou 94-98 sont remplacés par des acides aminés non chargés ou chargés négativement ;
(f) une molécule NGF mature modifiée avec une capacité réduite à se lier au p75^{NGFR} ;
(g) une molécule NGF mature modifiée ayant des substitutions d'acide aminé aux positions : G23, H84 et soit V18 soit V20 ;
(h) une molécule NGF mature modifiée comprenant au moins les 117 acides aminés du N-terminal du facteur NGF mature et ayant des substitutions d'acide aminé aux positions G23, H84, V18 et V20, et soit H84 soit T81.

31. Lignée cellulaire selon l'une quelconque des revendications précédentes, dans laquelle la séquence codant pour le facteur de croissance du tissu nerveux (NGF) code pour un facteur NGF mature avec un peptide signal hétérologue.

32. Lignée cellulaire selon l'une quelconque des revendications précédentes, dans laquelle la séquence codant pour le facteur de croissance du tissu nerveux (NGF) code pour un facteur NGF mature avec un pro-peptide hétérologue.

33. Lignée cellulaire selon l'une quelconque des revendications précédentes, dans laquelle la construction intégrée de manière stable comprend en outre une séquence choisie dans le groupe constitué par : une séquence consensus Kozak, un élément WPRE, un isolateur de bêta-globine, un amplificateur SP163, des régions d'amorce non traduites en 5' ou 3' provenant des gènes tau, TH ou APP.

34. Lignée cellulaire selon l'une quelconque des revendications précédentes, où le promoteur est un promoteur constitutif.

35. Lignée cellulaire selon la revendication 34, dans laquelle ledit promoteur est choisi dans le groupe constitué par le CMV, l'UbiC humaine, le JeT, le RSV, l'EF-lalpha.

36. Lignée cellulaire selon l'une quelconque des revendications précédentes 1 à 33, où le promoteur est un promoteur inductible.

37. Lignée cellulaire selon la revendication 36, où le promoteur inductible est choisi dans le groupe constitué par un promoteur pouvant être régulé par Tet, Mo-MLV-LTR, Mx1, la progestérone, RU486.

38. Lignée cellulaire selon l'une quelconque des revendications précédentes, dans laquelle la construction intégrée de manière stable est un vecteur d'expression plasmidique de mammifère.

39. Lignée cellulaire selon la revendication 38, dans laquelle ledit vecteur d'expression est choisi dans le groupe constitué par : pCI, pSI, pNS, pUbilz.

40. Lignée cellulaire selon l'une quelconque des revendications précédentes 1 à 34, dans laquelle ladite construction intégrée de manière stable est un vecteur viral.

41. Lignée cellulaire selon la revendication 40, dans laquelle ledit vecteur viral est dérivé de la famille des rétroviridae incluant le lentivirus, le VIH, le VIS, le VIF, le VIAE, le CIV.

42. Lignée cellulaire selon la revendication 40, qui est choisie dans le groupe constitué par l'alphavirus, l'adénovirus, le virus adéno-associé, le baculovirus, le VHS, le coronavirus, le papillomavirus bovin, le virus Mo-MLV.

43. Lignée cellulaire selon l'une quelconque des revendications précédentes, qui est dérivée d'une cellule épithéliale.

44. Lignée cellulaire selon la revendication 43, qui est dérivée d'une cellule épithéliale pigmentaire rétinienne (RPE), incluant des cellules RPE primaires mais ne s'y limitant pas.

45. Lignée cellulaire selon la revendication 44, qui est une lignée cellulaire dérivée de la lignée ARPE-19.

46. Lignée cellulaire selon la revendication 45, ayant le numéro d'accès DSM ACC 2706.

47. Lignée cellulaire selon la revendication 45, ayant le numéro d'accès DSM ACC 2707.

48. Lignée cellulaire selon l'une quelconque des revendications précédentes 1 à 42, qui est choisie dans le groupe constitué par : des lignées cellulaires d'astrocytes, une lignée cellulaire mésencéphalique humaine, et une lignée cellulaire endothéliale humaine.

49. Lignée cellulaire selon l'une quelconque des revendications précédentes, qui est capable de maintenir la production d'un facteur de croissance du tissu nerveux (NGF) biologiquement actif à un niveau suffisant pour maintenir son activité biologique utile pendant une période supérieure à un mois.

50. Lignée cellulaire selon l'une quelconque des revendications précédentes, qui est capable de sécréter un excès de 0,5 ng de facteur de croissance du tissu nerveux (NGF) biologiquement actif pour 10⁵ cellules par 24 heures.

51. Dispositif de culture cellulaire à implanter, le dispositif comprenant :
(a) une membrane semiperméable permettant la diffusion du facteur de croissance du tissu nerveux (NGF) à travers celle-ci ; et
(b) une population de cellules provenant de la lignée cellulaire selon l'une quelconque des revendications précédentes 1 à 50, disposée au sein de la membrane semiperméable.

52. Dispositif selon la revendication 51, qui est capable de sécréter un excès de 0,1 ng de facteur de croissance du tissu nerveux (NGF) biologiquement actif par 24 heures.

53. Dispositif selon la revendication 51, comprenant des cellules pouvant être obtenues à partir de la lignée cellulaire ayant le numéro d'accès DSM ACC 2706.

54. Dispositif selon la revendication 51, comprenant des cellules pouvant être obtenues à partir de la lignée cellulaire ayant le numéro d'accès DSM ACC 2707.

55. Dispositif selon la revendication 51, dans lequel la membrane semiperméable est immuno-isolante.

56. Dispositif selon la revendication 51, dans lequel la membrane semiperméable est microporeuse.

57. Dispositif selon la revendication 56, dans lequel la coupure de poids moléculaire de la membrane est inférieure ou égale à 1 000 kD.

58. Dispositif selon l'une quelconque des revendications précédentes 51 à 57, comprenant au moins 10³ cellules.

59. Dispositif selon l'une quelconque des revendications précédentes 51 à 58, dans lequel le volume du dispositif est un volume de 1 à 10 µl.

60. Dispositif selon l'une quelconque des revendications précédentes 51 à 59, dans lequel la longueur est d'au moins 1 mm.

61. Dispositif selon l'une quelconque des revendications précédentes 51 à 60, dans lequel l'épaisseur est d'au plus 1 000 µm de profondeur.

62. Dispositif selon l'une quelconque des revendications précédentes 51 à 61, dans lequel l'épaisseur de la gaine est de 2 à 200 micromètres.

63. Dispositif selon l'une quelconque des revendications précédentes 51 à 62, dans lequel le dispositif comprend en outre un ancrage de fixation.

64. Dispositif selon l'une quelconque des revendications précédentes 51 à 63, dans lequel le dispositif comprend un noyau comprenant une charpente de mousse réticulée ayant des pores interconnectés, la densité des pores de la mousse variant entre 20 % et 90 %, les cellules étant dispersées dans les pores.

65. Dispositif selon la revendication 64, dans lequel la charpente de mousse est un thermoplastique ou un élastomère thermoplastique.

66. Utilisation du dispositif selon l'une quelconque des revendications 51 à 65, pour la préparation d'un médicament.

67. Utilisation selon la revendication 66, pour le traitement d'un trouble neurologique.

68. Utilisation selon la revendication 67, dans laquelle le trouble neurologique est la maladie d'Alzheimer, la chorée de Huntington, un traumatisme du système nerveux central (SNC), la sclérose latérale amyotrophique (ALS), la maladie de Parkinson, une neuropathie périphérique, une douleur neuropathique, et d'autres états **caractérisés par** une nécrose ou une perte des neurones ou de leurs processus, qu'ils soient centraux ou périphériques.

69. Utilisation selon la revendication 66, dans laquelle le trouble de la vue est choisi dans le groupe constitué par : une rétinopathie diabétique, une uvéite, une rétinite pigmentaire, une dégénérescence maculaire liée à l'âge, un glaucome, une néovascularisation oculaire, une rétinite, et des lésions cornéennes.

70. Composition pharmaceutique comprenant une lignée cellulaire selon l'une quelconque des revendications précédentes 1 à 50, liée à une matrice.

71. Composition selon la revendication 70, dans laquelle la matrice est choisie dans le groupe constitué par le verre et d'autres oxydes de silicium, le polystyrène, le polypropylène, le polyéthylène, le fluorure de polyvinylidène, le polyuréthanne, le polyalginate, la polysulfone, l'alcool polyvinylique, les polymères d'acrylonitrile, le polyacrylamide, le polycarbonate, le polypentent, le nylon, les amylases, la gélatine naturelle et modifiée et le collagène naturel et modifié, les polysaccharides naturels et modifiés, incluant les dextranes et les celluloses (par exemple, la nitrocellulose), la gélose, et la magnétite.

72. Composition selon la revendication 70, dans laquelle la matrice solide peut être revêtue sur sa surface externe de facteurs de promotion de l'attachement, de la croissance ou de la survie cellulaire, incluant mais ne s'y limitant pas les molécules d'attachement cellulaire, la matrice extracellulaire, la fibronectine, la laminine, le collagène, l'élastine, les glycosaminoglycanes, ou les protéoglycanes ou les facteurs de croissance.

73. Composition selon la revendication 70, dans laquelle le ou les facteurs de promotion de la croissance ou de la survie sont incorporés dans le matériau de la matrice.

74. Composition selon la revendication 70, dans laquelle la configuration du support est sphérique, cylindrique, elliptique, une feuille ou une bande plate, en forme d'aiguille ou de broche, et similaires.

75. Composition selon la revendication 74, dans laquelle les tailles de billes sont d'environ 10 µm à 1 mm en diamètre.


### SEQUENCE LISTING

<110> NsGene A/S
   Tornøe, Jens
   Kusk, Philip
   Wahlberg, Lars
<120> Human therapeutic cells secreting nerve growth factor
<130> 515-204-WO
<150> DK PA 2004 00064
   <151> 2004-01-19
<150> DK PA 2004 00673
   <151> 2004-04-30
<150> US 60/537,033
   <151> 2004-01-20
<150> US 60/575,087
   <151> 2004-05-28
<160> 29
<170> PatentIn version 3.1
<210> 1
   <211> 723
   <212> DNA
   <213> Homo sapiens
<220>
   <221> CDS
   <222> (1)..(723)
   <223>
<220>
   <221> misc feature
   <222> (58)..(363)
   <223> Propeptide
<220>
   <221> sig_peptide
   <222> (1)..(57)
   <223>
<220>
   <221> mat_peptide
   <222> (364)..()
   <223>
<400> 1
   atg tcc  atg ttg ttc tac act  ctg atc aca gct ttt  ctg atc ggc        45
   Met Ser  Met Leu Phe Tyr Thr  Leu Ile Thr Ala Phe  Leu Ile Gly
       -120                -115                 -110
   ata cag  gcg gaa cca cac tca  gag agc aat gtc cct gca gga cac acc     93
   Ile Gln  Ala Glu Pro His Ser  Glu Ser Asn Val Pro Ala Gly His Thr
       -105                -100                 -95
   atc ccc caa gtc cac tgg act aaa ctt cag cat tcc ctt gac act gcc 141
   Ile Pro Gln Val His Trp Thr Lys Leu Gln His Ser Leu Asp Thr Ala
   -90 -85 -80 -75
   ctt cgc aga gcc cgc agc gcc ccg gca gcg gcg ata gct gca cgc gtg 189
   Leu Arg Arg Ala Arg Ser Ala Pro Ala Ala Ala Ile Ala Ala Arg Val
   -70 -65 -60
   gcg ggg cag acc cgc aac att act gtg gac ccc agg ctg ttt aaa aag 237
   Ala Gly Gln Thr Arg Asn Ile Thr Val Asp Pro Arg Leu Phe Lys Lys
   -55 -50 -45
   cgg cga ctc cgt tca ccc cgt gtg ctg ttt agc acc cag cct ccc cgt 285
   Arg Arg Leu Arg Ser Pro Arg Val Leu Phe Ser Thr Gln Pro Pro Arg
   -40 -35 -30
   gaa gct gca gac act cag gat ctg gac ttc gag gtc ggt ggt gct gcc 333
   Glu Ala Ala Asp Thr Gln Asp Leu Asp Phe Glu Val Gly Gly Ala Ala
   -25 -20 -15
   ccc ttc aac agg act cac agg agc aag cgg tca tca tcc cat ccc atc 381
   Pro Phe Asn Arg Thr His Arg Ser Lys Arg Ser Ser Ser His Pro Ile
   -10 -5 -1 1 5
   ttc cac agg ggc gaa ttc tcg gtg tgt gac agt gtc agc gtg tgg gtt 429
   Phe His Arg Gly Glu Phe Ser Val Cys Asp Ser Val Ser Val Trp Val
   10 15 20
   ggg gat aag acc acc gcc aca gac atc aag ggc aag gag gtg atg gtg 477
   Gly Asp Lys Thr Thr Ala Thr Asp Ile Lys Gly Lys Glu Val Met Val
   25 30 35
   ttg gga gag gtg aac att aac aac agt gta ttc aaa cag tac ttt ttt 525
   Leu Gly Glu Val Asn Ile Asn Asn Ser Val Phe Lys Gln Tyr Phe Phe
   40 45 50
   gag acc aag tgc cgg gac cca aat ccc gtt gac agc ggg tgc cgg ggc 573
   Glu Thr Lys Cys Arg Asp Pro Asn Pro Val Asp Ser Gly Cys Arg Gly
   55 60 65 70
   att gac tca aag cac tgg aac tca tat tgt acc acg act cac acc ttt 621
   Ile Asp Ser Lys His Trp Asn Ser Tyr Cys Thr Thr Thr His Thr Phe
   75 80 85
   gtc aag gcg ctg acc atg gat ggc aag cag gct gcc tgg cgg ttt atc 669
   Val Lys Ala Leu Thr Met Asp Gly Lys Gln Ala Ala Trp Arg Phe Ile
   90 95 100
   cgg ata gat acg gcc tgt gtg tgt gtg ctc agc agg aag gct gtg aga 717
   Arg Ile Asp Thr Ala Cys Val Cys Val Leu Ser Arg Lys Ala Val Arg
   105 110 115
   aga gcc 723
   Arg Ala
   120
<210> 2
   <211> 241
   <212> PRT
   <213> Homo sapiens
<220>
   <221> misc_feature
   <222> (58)..(363)
   <223> Propeptide
<400> 2
   Met Ser  Met Leu Phe Tyr Thr  Leu Ile Thr Ala Phe  Leu Ile Gly
       -120                -115                -110
   Ile Gln  Ala Glu Pro His Ser  Glu Ser Asn Val Pro Ala Gly His Thr
       -105                -100                -95
   Ile Pro Gln Val His Trp Thr Lys Leu Gln His Ser Leu Asp Thr Ala
   -90                 -85                 -80                 -75
   Leu Arg Arg Ala Arg Ser Ala Pro Ala Ala Ala Ile Ala Ala Arg Val
                   -70                 -65                 -60
   Ala Gly Gln Thr Arg Asn Ile Thr Val Asp Pro Arg Leu Phe Lys Lys
               -55                 -50                 -45
   Arg Arg Leu Arg Ser Pro Arg Val Leu Phe Ser Thr Gln Pro Pro Arg
           -40                 -35                 -30
   Glu Ala Ala Asp Thr Gln Asp Leu Asp Phe Glu Val Gly Gly Ala Ala
       -25                 -20                 -15
   Pro Phe Asn Arg Thr His Arg Ser Lys Arg Ser Ser Ser His Pro Ile
   -10                 -5                  -1  1               5
   Phe His Arg Gly Glu Phe Ser Val Cys Asp Ser Val Ser Val Trp Val
               10                  15                  20
   Gly Asp Lys Thr Thr Ala Thr Asp Ile Lys Gly Lys Glu Val Met Val
           25                  30                  35
   Leu Gly Glu Val Asn Ile Asn Asn Ser Val Phe Lys Gln Tyr Phe Phe
       40                  45                  50
   Glu Thr Lys Cys Arg Asp Pro Asn Pro Val Asp Ser Gly Cys Arg Gly
   55                  60                  65                  70
   Ile Asp Ser Lys His Trp Asn Ser Tyr Cys Thr Thr Thr His Thr Phe
                   75                  80                  85
   Val Lys Ala Leu Thr Met Asp Gly Lys Gln Ala Ala Trp Arg Phe Ile
               90                  95                  100
   Arg Ile Asp Thr Ala Cys Val Cys Val Leu Ser Arg Lys Ala Val Arg
           105                 110                 115
   Arg Ala
       120
<210> 3
   <211> 307
   <212> PRT
   <213> Mus musculus
<220>
   <221> mat_peptide
   <222> (188)..()
   <223>
<400> 3
   Met Leu Cys Leu Lys Pro Val Lys Leu Gly Ser Leu Glu Val Gly
   -185 -180 -175
   His Gly Gln His Gly Gly Val Leu Ala Cys Gly Arg Ala Val Gln
   -170 -165 -160
   Gly Ala Gly Trp His Ala Gly Pro Lys Leu Thr Ser Val Ser Gly
   -155 -150 -145
   Pro Asn Lys Gly Phe Ala Lys Asp Ala Ala Phe Tyr Thr Gly Arg
   -140 -135 -130
   Ser Glu Val His Ser Val Met Ser Met Leu Phe Tyr Thr Leu Ile
   -125 -120 -115
   Thr Ala Phe Leu Ile Gly Val Gln Ala Glu Pro Tyr Thr Asp Ser
   -110 -105 -100
   Asn Val Pro Glu Gly Asp Ser Val Pro Glu Ala His Trp Thr Lys Leu
   -95 -90 -85
   Gln His Ser Leu Asp Thr Ala Leu Arg Arg Ala Arg Ser Ala Pro Thr
   -80 -75 -70
   Ala Pro Ile Ala Ala Arg Val Thr Gly Gln Thr Arg Asn Ile Thr Val
   -65 -60 -55 -50
   Asp Pro Arg Leu Phe Lys Lys Arg Arg Leu His Ser Pro Arg Val Leu
   -45 -40 -35
   Phe Ser Thr Gln Pro Pro Pro Thr Ser Ser Asp Thr Leu Asp Leu Asp
   -30 -25 -20
   Phe Gln Ala His Gly Thr Ile Pro Phe Asn Arg Thr His Arg Ser Lys
   -15 -10 -5
   Arg Ser Ser Thr His Pro Val Phe His Met Gly Glu Phe Ser Val Cys
   -1 1 5 10 15
   Asp Ser Val Ser Val Trp Val Gly Asp Lys Thr Thr Ala Thr Asp Ile
   20 25 30
   Lys Gly Lys Glu Val Thr Val Leu Ala Glu Val Asn Ile Asn Asn Ser
   35 40 45
   Val Phe Arg Gln Tyr Phe Phe Glu Thr Lys Cys Arg Ala Ser Asn Pro
   50 55 60
   Val Glu Ser Gly Cys Arg Gly Ile Asp Ser Lys His Trp Asn Ser Tyr
   65 70 75
   Cys Thr Thr Thr His Thr Phe Val Lys Ala Leu Thr Thr Asp Glu Lys
   80 85 90 95
   Gln Ala Ala Trp Arg Phe Ile Arg Ile Asp Thr Ala Cys Val Cys Val
   100 105 110
   Leu Ser Arg Lys Ala Thr Arg Arg Gly
   115 120
<210> 4
   <211> 241
   <212> PRT
   <213> Rattus norvegicus
<220>
   <221> mat_peptide
   <222> (122)..()
   <223>
<400> 4
   Met Ser  Met Leu Phe Tyr Thr  Leu Ile Thr Ala Phe  Leu Ile Gly
       -120                 -115                 -110
   Val Gln  Ala Glu Pro Tyr Thr  Asp Ser Asn Val Pro Glu Gly Asp Ser
       -105                 -100                 -95
   Val Pro Glu Ala His Trp Thr Lys Leu Gln His Ser Leu Asp Thr Ala
   -90                 -85                 -80                 -75
   Leu Arg Arg Ala Arg Ser Ala Pro Ala Glu Pro Ile Ala Ala Arg Val
                   -70                 -65                 -60
   Thr Gly Gln Thr Arg Asn Ile Thr Val Asp Pro Lys Leu Phe Lys Lys
               -55                 -50                 -45
   Arg Arg Leu Arg Ser Pro Arg Val Leu Phe Ser Thr Gln Pro Pro Pro
           -40                 -35                 -30
   Thr Ser Ser Asp Thr Leu Asp Leu Asp Phe Gln Ala His Gly Thr Ile
       -25                 -20                 -15
   Ser Phe Asn Arg Thr His Arg Ser Lys Arg Ser Ser Thr His Pro Val
   -10                 -5                  -1  1               5
   Phe His Met Gly Glu Phe Ser Val Cys Asp Ser Val Ser Val Trp Val
               10                  15                  20
   Gly Asp Lys Thr Thr Ala Thr Asp Ile Lys Gly Lys Glu Val Thr Val
           25                  30                  35
   Leu Gly Glu Val Asn Ile Asn Asn Ser Val Phe Lys Gln Tyr Phe Phe
       40                  45                  50
   Glu Thr Lys Cys Arg Ala Pro Asn Pro Val Glu Ser Gly Cys Arg Gly
   55                  60                  65                  70
   Ile Asp Ser Lys His Trp Asn Ser Tyr Cys Thr Thr Thr His Thr Phe
                   75                  80                  85
   Val Lys Ala Leu Thr Thr Asp Asp Lys Gln Ala Ala Trp Arg Phe Ile
               90                  95                  100
   Arg Ile Asp Thr Ala Cys Val Cys Val Leu Ser Arg Lys Ala Ala Arg
           105                 110                 115
   Arg Gly
       120
<210> 5
   <211> 241
   <212> PRT
   <213> Pan troglodytes
<220>
   <221> mat_peptide
   <222> (122)..()
   <223>
<220>
   <221> MISC_FEATURE
   <222> (106)..(106)
   <223> unknown amino acid
<400> 5
   Met Ser Met Leu Phe Tyr Thr Leu Ile Thr Ala Phe Leu Ile Gly
       -120                -115                -110
   Thr Gln Ala Glu Pro His Ser Glu Ser Asn Val Pro Ala Gly His Thr
       -105                -100                -95
   Ile Pro Gln Ala His Trp Thr Lys Leu Gln His Ser Leu Asp Thr Ala
   -90                 -85                 -80                 -75
   Leu Arg Arg Ala Arg Ser Ala Pro Ala Ala Ala Ile Ala Ala Arg Val
                   -70                 -65                 -60
   Ala Gly Gln Thr Arg Asn Ile Thr Val Asp Pro Arg Leu Phe Lys Lys
               -55                 -50                 -45
   Arg Arg Leu Arg Ser Pro Arg Val Leu Phe Ser Thr Gln Pro Pro Pro
           -40                 -35                 -30
   Glu Ala Ala Asp Thr Gln Asp Leu Asp Phe Xaa Val Gly Gly Ala Ala
       -25                 -20                 -15
   Pro Phe Asn Arg Thr His Arg Ser Lys Arg Ser Ser Ser His Pro Ile
   -10                 -5                  -1  1               5
   Phe His Arg Gly Glu Phe Ser Val Cys Asp Ser Val Ser Val Trp Val
               10                  15                  20
   Gly Asp Lys Thr Thr Ala Thr Asp Ile Lys Gly Lys Glu Val Met Val
           25                  30                  35
   Leu Gly Glu Val Asn Ile Asn Asn Ser Val Phe Lys Gln Tyr Phe Phe
       40                  45                  50
   Glu Thr Lys Cys Arg Asp Pro Asn Pro Val Asp Ser Gly Cys Arg Gly
   55                  60                  65                  70
   Ile Asp Ser Lys His Trp Asn Ser Tyr Cys Thr Thr Thr His Thr Phe
                   75                  80                  85
   Val Lys Ala Leu Thr Met Asp Gly Lys Gln Ala Ala Trp Arg Phe Ile
               90                  95                  100
   Arg Ile Asp Thr Ala Cys Val Cys Val Leu Ser Arg Lys Ala Val Arg
           105                 110                 115
   Arg Ala
       120
<210> 6
   <211> 229
   <212> PRT
   <213> Sus scrofa
<220>
   <221> mat_peptide
   <222> (110)..()
   <223>
<220>
   <221> MISC_FEATURE
   <222> (1)..(1)
   <223> Incomplete N-terminal
<400> 6
   Leu Ile Gly Ile Gln  Ala Glu Pro His Thr  Glu Ser Asn Val Pro Ala
                   -105                 -100                 -95
   Gly His Ala Ile Pro Gln Ala His Trp Thr Lys Leu Gln His Ser Leu
               -90                 -85                 -80
   Asp Thr Ala Leu Arg Arg Ala His Ser Ala Pro Ala Gly Ala Asn Ser
           -75                 -70                 -65
   Ala Arg Val Ala Gly Gln Thr Arg Asn Ile Thr Val Asp Pro Lys Leu
       -60                 -55                 -50
   Phe Lys Lys Arg Arg Leu Arg Ser Pro Arg Val Leu Phe Ser Thr Gln
   -45                 -40                 -35                 -30
   Pro Pro Pro Val Ala Ala Asp Thr Gln Asp Pro Asp Leu Glu Ala Ser
                   -25                 -20                 -15
   Gly Ala Ala Ser Phe Asn Arg Thr His Arg Ser Lys Arg Ser Ser Ser
               -10                 -5                  -1  1
   His Pro Val Phe His Arg Gly Glu Phe Ser Val Cys Asp Ser Val Ser
       5                   10                  15
   Val Trp Val Gly Asp Lys Thr Thr Ala Thr Asp Ile Lys Gly Lys Glu
   20                  25                  30                  35
   Val Met Val Leu Gly Glu Val Asn Ile Asn Asn Ser Val Phe Lys Gln
                   40                  45                  50
   Tyr Phe Phe Glu Thr Lys Cys Arg Asp Pro Asn Pro Val Asp Ser Gly
               55                  60                  65
   Cys Arg Gly Ile Asp Ser Lys His Trp Asn Ser Tyr Cys Thr Thr Thr
           70                  75                  80
   His Thr Phe Val Lys Ala Leu Thr Met Asp Gly Lys Gln Ala Ala Trp
       85                  90                  95
   Arg Phe Ile Arg Ile Asp Thr Ala Cys Val Cys Val Leu Ser Arg Lys
   100                 105                 110                 115
   Ala Gly Arg Arg Ala
                   120
<210> 7
   <211> 118
   <212> PRT
   <213> Homo sapiens
<400> 7
   Ser Ser Ser His Pro Ile Phe His Arg Gly Glu Phe Ser Val Cys Ala
   1               5                   10                  15
   Ser Glu Ser Leu Trp Val Thr Asp Lys Thr Thr Ala Thr Asp Ile Lys
               20                  25                  30
   Gly Lys Glu Val Met Val Leu Gly Glu Val Asn Ile Asn Asn Ser Val
           35                  40                  45
   Phe Lys Gln Tyr Phe Phe Glu Thr Lys Cys Arg Asp Pro Asn Pro Val
       50                  55                  60
   Asp Ser Gly Cys Arg Gly Ile Asp Ser Lys His Trp Asn Ser Gln Cys
   65                  70                  75                  80
   Lys Thr Thr Gln Thr Tyr Val Arg Ala Leu Thr Met Asp Gly Lys Gln
                   85                  90                  95
   Ala Ala Trp Arg Phe Ile Arg Ile Asp Thr Ala Cys Val Cys Val Leu
               100                 105                 110
   Ser Arg Lys Ala Val Arg
           115
<210> 8
   <211> 118
   <212> PRT
   <213> Homo sapiens
<400> 8
   Ser Ser Ser His Pro Ile Phe His Arg Gly Glu Phe Ser Val Cys Ala
   1               5                   10                  15
   Ser Val Ser Val Trp Val Gly Asp Lys Thr Thr Ala Thr Asp Ile Lys
               20                  25                  30
   Gly Lys Glu Val Met Val Leu Gly Glu Val Asn Ile Asn Asn Ser Val
           35                  40                  45
   Phe Lys Gln Tyr Phe Phe Glu Thr Lys Cys Arg Asp Pro Asn Pro Val
       50                  55                  60
   Asp Ser Gly Cys Arg Gly Ile Asp Ser Lys His Trp Asn Ser Tyr Cys
   65                  70                  75                  80
   Lys Thr Thr Gln Thr Tyr Val Arg Ala Leu Thr Met Asp Gly Lys Gln
                   85                  90                  95
   Ala Ala Trp Arg Phe Ile Arg Ile Asp Thr Ala Cys Val Cys Val Leu
   100 105 110
   Ser Arg Lys Ala Val Arg
   115
<210> 9
   <211> 120
   <212> PRT
   <213> Homo sapiens
<400> 9
   Ser Ser Ser His Pro Ile Phe His Arg Gly Glu Phe Ser Val Cys Asp
   1 5 10 15
   Ser Val Ser Leu Trp Val Thr Asp Lys Thr Thr Ala Thr Asp Ile Lys
   20 25 30
   Gly Lys Glu Val Met Val Leu Gly Glu Val Asn Ile Asn Asn Ser Val
   35 40 45
   Phe Lys Gln Tyr Phe Phe Glu Thr Lys Cys Arg Asp Pro Asn Pro Val
   50 55 60
   Asp Ser Gly Cys Arg Gly Ile Asp Ser Lys His Trp Asn Ser Tyr Cys
   65 70 75 80
   Thr Thr Thr Gln Thr Tyr Val Lys Ala Leu Thr Met Asp Gly Lys Gln
   85 90 95
   Ala Ala Trp Arg Phe Ile Arg Ile Asp Thr Ala Cys Val Cys Val Leu
   100 105 110
   Ser Arg Lys Ala Val Arg Arg Ala
   115 120
<210> 10
   <211> 120
   <212> PRT
   <213> Homo sapiens
<400> 10
   Ser Ser Ser His Pro Ile Phe His Arg Gly Glu Phe Ser Val Cys Asp
   1               5                   10                  15
   Ser Val Ser Leu Trp Val Thr Asp Lys Thr Thr Ala Ile Asp Ile Lys
               20                  25                  30
   Gly Lys Glu Val Met Val Leu Gly Glu Val Asn Ile Asn Asn Ser Val
           35                  40                  45
   Phe Lys Gln Tyr Phe Phe Glu Thr Lys Cys Arg Asp Pro Asn Pro Val
       50                  55                  60
   Asp Ser Gly Cys Arg Gly Ile Asp Ser Lys His Trp Asn Ser Tyr Cys
   65                  70                  75                  80
   Thr Thr Thr Gln Thr Tyr Val Lys Ala Leu Thr Met Asp Gly Lys Gln
                   85                  90                  95
   Ala Ala Trp Arg Phe Ile Arg Ile Asp Thr Ala Cys Val Cys Val Leu
               100                 105                 110
   Ser Arg Lys Ala Val Arg Arg Ala
           115                 120
<210> 11
   <211> 120
   <212> PRT
   <213> Homo sapiens
<400> 11
   Ser Ser Ser His Pro Ile Phe His Arg Gly Glu Phe Ser Val Cys Asp
   1               5                   10                  15
   Ser Val Ser Leu Trp Val Thr Asp Lys Thr Thr Ala Thr Asp Ile Lys
               20                  25                  30
   Gly Lys Glu Val Met Val Leu Gly Glu Val Asn Ile Asn Asn Ser Val
           35                  40                  45
   Phe Lys Gln Tyr Phe Phe Glu Thr Lys Cys Arg Asp Pro Asn Pro Val
       50                  55                  60
   Asp Ser Gly Cys Arg Gly Ile Asp Ser Lys His Trp Asn Ser Tyr Cys
   65                  70                  75                  80
   Lys Thr Thr Gln Thr Phe Val Lys Ala Leu Thr Met Asp Gly Lys Gln
                   85                  90                  95
   Ala Ala Trp Arg Phe Ile Arg Ile Asp Thr Ala Cys Val Cys Val Leu
               100                 105                 110
   Ser Arg Lys Ala Val Arg Arg Ala
           115                 120
<210> 12
   <211> 120
   <212> PRT
   <213> Homo sapiens
<400> 12
   Ser Ser Ser His Pro Ile Phe His Arg Gly Glu Phe Ser Val Cys Asp
   1               5                   10                  15
   Ser Glu Ser Val Trp Val Thr Asp Lys Thr Thr Ala Thr Asp Ile Lys
               20                  25                  30
   Gly Lys Glu Val Met Val Leu Gly Glu Val Asn Ile Asn Asn Ser Val
           35                  40                  45
   Phe Lys Gln Tyr Phe Phe Glu Thr Lys Cys Arg Asp Pro Asn Pro Val
       50                  55                  60
   Asp Ser Gly Cys Arg Gly Ile Asp Ser Lys His Trp Asn Ser Tyr Cys
   65                  70                  75                  80
   Lys Thr Thr Gln Thr Phe Val Lys Ala Leu Thr Met Asp Gly Lys Gln
                   85                  90                  95
   Ala Ala Trp Arg Phe Ile Arg Ile Asp Thr Ala Cys Val Cys Val Leu
               100                 105                 110
   Ser Arg Lys Ala Val Arg Arg Ala
           115                 120
<210> 13
   <211> 120
   <212> PRT
   <213> Homo sapiens
<400> 13
   Ser Ser Ser His Pro Ile Phe His Arg Gly Glu Phe Ser Val Cys Asp
   1               5                   10                  15
   Ser Val Ser Val Trp Val Gly Asp Lys Thr Thr Ala Thr Asp Ile Ala
               20                  25                  30
   Gly Ala Ala Val Met Val Leu Gly Glu Val Asn Ile Asn Asn Ser Val
           35                  40                  45
   Phe Lys Gln Tyr Phe Phe Glu Thr Lys Cys Arg Asp Pro Asn Pro Val
       50                  55                  60
   Asp Ser Gly Cys Arg Gly Ile Asp Ser Lys His Trp Asn Ser Tyr Cys
   65                  70                  75                  80
   Thr Thr Thr His Thr Phe Val Lys Ala Leu Thr Met Asp Gly Lys Gln
                   85                  90                  95
   Ala Ala Trp Arg Phe Ile Arg Ile Asp Thr Ala Cys Val Cys Val Leu
               100                 105                 110
   Ser Arg Lys Ala Val Arg Arg Ala
           115                 120
<210> 14
   <211> 1400
   <212> DNA
   <213> Artificial sequence
<220>
   <223> pCIn.hNGF
<220>
   <221> precursor_RNA
   <222> (744)..(1400)
   <223>
<220>
   <221> Intron
   <222> (890)..(1022)
   <223>
<220>
   <221> CDS
   <222> (1214)..(1399)
   <223>
<400> 14
   tcaatattgg ccattagcca tattattcat tggttatata gcataaatca atattggcta      60
   ttggccattg catacgttgt atctatatca taatatgtac atttatattg gctcatgtcc     120
   aatatgaccg ccatgttggc attgattatt gactagttat taatagtaat caattacggg    180
   gtcattagtt catagcccat atatggagtt ccgcgttaca taacttacgg taaatggccc    240
   gcctggctga ccgcccaacg acccccgccc attgacgtca ataatgacgt atgttcccat    300
   agtaacgcca atagggactt tccattgacg tcaatgggtg gagtatttac ggtaaactgc    360
   ccacttggca gtacatcaag tgtatcatat gccaagtccg ccccctattg acgtcaatga    420
   cggtaaatgg cccgcctggc attatgccca gtacatgacc ttacgggact ttcctacttg    480
   gcagtacatc tacgtattag tcatcgctat taccatggtg atgcggtttt ggcagtacac    540
   caatgggcgt ggatagcggt ttgactcacg gggatttcca agtctccacc ccattgacgt    600
   caatgggagt ttgttttggc accaaaatca acgggacttt ccaaaatgtc gtaacaactg    660
   cgatcgcccg ccccgttgac gcaaatgggc ggtaggcgtg tacggtggga ggtctatata    720
   agcagagctc gtttagtgaa ccgtcagatc actagaagct ttattgcggt agtttatcac    780
   agttaaattg ctaacgcagt cagtgcttct gacacaacag tctcgaactt aagctgcagt    840
   gactctctta aggtagcctt gcagaagttg gtcgtgaggc actgggcagg taagtatcaa    900
   ggttacaaga caggtttaag gagaccaata gaaactgggc ttgtcgagac agagaagact    960
   cttgcgtttc tgataggcac ctattggtct tactgacatc cactttgcct ttctctccac   1020
   aggtgtccac tcccagttca attacagctc ttaaggctag agtacttaat acgactcact   1080
   ataggctagc gtttaaactt aagcttggta ccgagctcgg atccctctga gggacccaga   1140
   aactgccttt tgactgcatt tagtactcca tgaagtcacc ctcatttctt tttcattcca   1200
   ggtgcatagc gta atg tcc atg ttg ttc tac act ctg atc aca gct ttt      1249
                  Met Ser Met Leu Phe Tyr Thr Leu Ile Thr Ala Phe
                  1               5                   10
   ctg atc ggc ata cag gcg gaa cca cac tca gag agc aat gtc cct gca     1297
   Leu Ile Gly Ile Gln Ala Glu Pro His Ser Glu Ser Asn Val Pro Ala
           15                  20                  25
   gga cac acc atc ccc caa gtc cac tgg act aaa ctt cag cat tcc ctt     1345
   Gly His Thr Ile Pro Gln Val His Trp Thr Lys Leu Gln His Ser Leu
       30                  35                  40
   gac act gcc ctt cgc aga gcc cgc agc gcc ccg gca gcg gcg ata gct     1393
   Asp Thr Ala Leu Arg Arg Ala Arg Ser Ala Pro Ala Ala Ala Ile Ala
   45                  50                  55                  60
   gca cgc g                                                           1400
   Ala Arg
<210> 15
   <211> 62
   <212> PRT
   <213> Artificial sequence
<220>
   <223> pCIn.hNGF
<400> 15
   Met Ser Met Leu Phe Tyr Thr Leu Ile Thr Ala Phe Leu Ile Gly Ile
   1               5                   10                  15
   Gln Ala Glu Pro His Ser Glu Ser Asn Val Pro Ala Gly His Thr Ile
               20                  25                  30
   Pro Gln Val His Trp Thr Lys Leu Gln His Ser Leu Asp Thr Ala Leu
           35                  40                  45
   Arg Arg Ala Arg Ser Ala Pro Ala Ala Ala Ile Ala Ala Arg
       50                  55                  60
<210> 16
   <211> 1400
   <212> DNA
   <213> Artificial sequence
<220>
   <223> pCIn.KhNGF
<220>
   <221> precursor_RNA
   <222> (744)..(1400)
   <223>
<220>
   <221> Intron
   <222> (890)..(1022)
   <223>
<220>
   <221> CDS
   <222> (1134)..(1400)
   <223>
<220>
   <221> misc_feature
   <222> (1128)..(1133)
   <223> Kozak consensus sequence
<400> 16
   tcaatattgg ccattagcca tattattcat tggttatata gcataaatca atattggcta     60
   ttggccattg catacgttgt atctatatca taatatgtac atttatattg gctcatgtcc    120
   aatatgaccg ccatgttggc attgattatt gactagttat taatagtaat caattacggg    180
   gtcattagtt catagcccat atatggagtt ccgcgttaca taacttacgg taaatggccc    240
   gcctggctga ccgcccaacg acccccgccc attgacgtca ataatgacgt atgttcccat    300
   agtaacgcca atagggactt tccattgacg tcaatgggtg gagtatttac ggtaaactgc    360
   ccacttggca gtacatcaag tgtatcatat gccaagtccg ccccctattg acgtcaatga    420
   cggtaaatgg cccgcctggc attatgccca gtacatgacc ttacgggact ttcctacttg    480
   gcagtacatc tacgtattag tcatcgctat taccatggtg atgcggtttt ggcagtacac    540
   caatgggcgt ggatagcggt ttgactcacg gggatttcca agtctccacc ccattgacgt    600
   caatgggagt ttgttttggc accaaaatca acgggacttt ccaaaatgtc gtaacaactg    660
   cgatcgcccg ccccgttgac gcaaatgggc ggtaggcgtg tacggtggga ggtctatata    720
   agcagagctc gtttagtgaa ccgtcagatc actagaagct ttattgcggt agtttatcac    780
   agttaaattg ctaacgcagt cagtgcttct gacacaacag tctcgaactt aagctgcagt    840
   gactctctta aggtagcctt gcagaagttg gtcgtgaggc actgggcagg taagtatcaa    900
   ggttacaaga caggtttaag gagaccaata gaaactgggc ttgtcgagac agagaagact    960
   cttgcgtttc tgataggcac ctattggtct tactgacatc cactttgcct ttctctccac   1020
   aggtgtccac tcccagttca attacagctc ttaaggctag agtacttaat acgactcact   1080
   ataggctagc gtttaaactt aagcttggta ccgagctcgg atccgccgcc gcc atg      1136
                                                              Met
                                                              1
   tcc atg ttg ttc tac act ctg atc aca gct ttt ctg atc ggc ata cag     1184
   Ser Met Leu Phe Tyr Thr Leu Ile Thr Ala Phe Leu Ile Gly Ile Gln
               5                   10                  15
   gcg gaa cca cac tca gag agc aat gtc cct gca gga cac acc atc ccc     1232
   Ala Glu Pro His Ser Glu Ser Asn Val Pro Ala Gly His Thr Ile Pro
           20                  25                  30
   caa gtc cac tgg act aaa ctt cag cat tcc ctt gac act gcc ctt cgc     1280
   Gln Val His Trp Thr Lys Leu Gln His Ser Leu Asp Thr Ala Leu Arg
       35                  40                  45
   aga gcc cgc agc gcc ccg gca gcg gcg ata gct gca cgc gtg gcg ggg     1328
   Arg Ala Arg Ser Ala Pro Ala Ala Ala Ile Ala Ala Arg Val Ala Gly
   50                  55                  60                  65
   cag acc cgc aac att act gtg gac ccc agg ctg ttt aaa aag cgg cga     1376
   Gln Thr Arg Asn Ile Thr Val Asp Pro Arg Leu Phe Lys Lys Arg Arg
                   70                  75                  80
   ctc cgt tca ccc cgt gtg ctg ttt                                     1400
   Leu Arg Ser Pro Arg Val Leu Phe
               85
<210> 17
   <211> 89
   <212> PRT
   <213> Artificial sequence
<220>
   <223> pCIn.KhNGF
<220>
   <221> misc_feature
   <222> (1128)..(1133)
   <223> Kozak consensus sequence
<400> 17
   Met Ser Met Leu Phe Tyr Thr Leu Ile Thr Ala Phe Leu Ile Gly Ile
   1               5                   10                  15
   Gln Ala Glu Pro His Ser Glu Ser Asn Val Pro Ala Gly His Thr Ile
               20                  25                  30
   Pro Gln Val His Trp Thr Lys Leu Gln His Ser Leu Asp Thr Ala Leu
           35                  40                  45
   Arg Arg Ala Arg Ser Ala Pro Ala Ala Ala Ile Ala Ala Arg Val Ala
       50                  55                  60
   Gly Gln Thr Arg Asn Ile Thr Val Asp Pro Arg Leu Phe Lys Lys Arg
   65                  70                  75                  80
   Arg Leu Arg Ser Pro Arg Val Leu Phe
                   85
<210> 18
   <211> 1400
   <212> DNA
   <213> artificial sequence
<220>
   <223> EF1alpha.KhNGF
<220>
   <221> precursor_RNA
   <222> (199)..(1400)
   <223>
<220>
   <221> Intron
   <222> (232)..(1171)
   <223>
<220>
   <221> CDS
   <222> (1249) .. (1398)
   <223>
<220>
   <221> misc_feature
   <222> (1240)..(1248)
   <223> Kozak consensus sequence
<400> 18
   ggctccggtg cccgtcagtg ggcagagcgc acatcgccca cagtccccga gaagttgtgg 60
   ggaggggtcg gcaattgaac cggtgcctag agaaggtggc gcggggtaaa ctgggaaagt 120
   gatgtcgtgt actggctccg cctttttccc gagggtgggg gagaaccgta tataagtgca 180
   gtagtcgccg tgaacgttct ttttcgcaac gggtttgccg ccagaacaca ggtaagtgcc 240
   gtgtgtggtt cccgcgggcc tggcctcttt acgggttatg gcccttgcgt gccttgaatt 300
   acttccacct ggctgcagta cgtgattctt gatcccgagc ttcgggttgg aagtgggtgg     360
   gagagttcga ggccttgcgc ttaaggagcc ccttcgcctc gtgcttgagt tgaggcctgg     420
   cctgggcgct ggggccgccg cgtgcgaatc tggtggcacc ttcgcgcctg tctcgctgct     480
   ttcgataagt ctctagccat ttaaaatttt tgatgacctg ctgcgacgct ttttttctgg     540
   caagatagtc ttgtaaatgc gggccaagat ctgcacactg gtatttcggt ttttggggcc     600
   gcgggcggcg acggggcccg tgcgtcccag cgcacatgtt cggcgaggcg gggcctgcga     660
   gcgcggccac cgagaatcgg acgggggtag tctcaagctg gccggcctgc tctggtgcct     720
   ggcctcgcgc cgccgtgtat cgccccgccc tgggcggcaa ggctggcccg gtcggcacca     780
   gttgcgtgag cggaaagatg gccgcttccc ggccctgctg cagggagctc aaaatggagg     840
   acgcggcgct cgggagagcg ggcgggtgag tcacccacac aaaggaaaag ggcctttccg     900
   tcctcagccg tcgcttcatg tgactccacg gagtaccggg cgccgtccag gcacctcgat     960
   tagttctcga gcttttggag tacgtcgtct ttaggttggg gggaggggtt ttatgcgatg    1020
   gagtttcccc acactgagtg ggtggagact gaagttaggc cagcttggca cttgatgtaa    1080
   ttctccttgg aatttgccct ttttgagttt ggatcttggt tcattctcaa gcctcagaca    1140
   gtggttcaaa gtttttttct tccatttcag gtgtcgtgag gaattagctt ggtaccggag    1200
   ctagcgttta aacttaagct tggtaccgag ctcggatccg ccgccgcc atg tcc atg     1257
                                                        Met Ser Met
                                                        1
   ttg ttc tac act ctg atc aca gct ttt ctg atc ggc ata cag gcg gaa      1305
   Leu Phe Tyr Thr Leu Ile Thr Ala Phe Leu Ile Gly Ile Gln Ala Glu
       5                   10                  15
   cca cac tca gag agc aat gtc cct gca gga cac acc atc ccc caa gtc      1353
   Pro His Ser Glu Ser Asn Val Pro Ala Gly His Thr Ile Pro Gln Val
   20                  25                  30                  35
   cac tgg act aaa ctt cag cat tcc ctt gac act gcc ctt cgc aga gc       1400
   His Trp Thr Lys Leu Gln His Ser Leu Asp Thr Ala Leu Arg Arg
                   40                  45                  50
<210> 19
   <211> 50
   <212> PRT
   <213> artificial sequence
<220>
   <223> EF1alpha.KhNGF
<220>
   <221> misc_feature
   <222> (1240)..(1248)
   <223> Kozak consensus sequence
<210> 20
   <211> 1700
   <212> DNA
   <213> artificial sequence
<220>
   <223> UbiC.hNGF
<220>
   <221> TATA_signal
   <222> (641)..(647)
   <223>
<220>
   <221> Intron
   <222> (736)..(1547)
   <223>
<220>
   <221> CDS
   <222> (1664)..(1699)
   <223>
<400> 20
   ctagctttct gccgagtcat tgtccttgtc ccgcggcccc gggagccccc cgcgaccggc    60
   ctgggaggct cagggaggtt gaagggggct gagcaaagga agccccgtca ttacctcaaa   120
   tgtgacccaa aaataaagac ccgtccatct cgcagggtgg gccagggcgg gtcaggaggg   180
   aggggaggga gaccccgact ctgcagaagg cgctcgctgc gtgccccacg tccgccgaac   240
   gcggggttcg cgacccgagg ggaccgcggg ggctgagggg aggggccgcg gagccgcggc   300
   taaggaacgc gggccgccca cccgctccgg gtgcagcggc ctccgcgccg ggttttggcg   360
   cctcccgcgg gcgcccccct cctcacggcg agcgctgcca cgtcagacga agggcgcagc   420
   gagcgtcctg atccttccgc ccggacgctc aggacagcgg cccgctgctc ataagactcg   480
   gccttagaac cccagtatca gcagaaggac attttaggac gggacttggg tgactctagg   540
   gcactggttt tctttccaga gagcggaaca ggcgaggaaa agtagtccct tctcggcgat   600
   tctgcggagg gatctccgtg gggcggtgaa cgccgatgat tatataagga cgcgccgggt    660
   gtggcacagc tagttccgtc gcagccggga tttgggtcgc ggttcttgtt tgtggatcgc   720
   tgtgatcgtc acttggtgag tagcgggctg ctgggctggc cggggctttc gtggccgccg   780
   ggccgctcgg tgggacggaa gcgtgtggag agaccgccaa gggctgtagt ctgggtccgc   840
   gagcaaggtt gccctgaact gggggttggg gggagcgcag caaaatggcg gctgttcccg   900
   agtcttgaat ggaagacgct tgtgaggcgg gctgtgaggt cgttgaaaca aggtgggggg   960
   catggtgggc ggcaagaacc caaggtcttg aggccttcgc taatgcggga aagctcttat  1020
   tcgggtgaga tgggctgggg caccatctgg ggaccctgac gtgaagtttg tcactgactg  1080
   gagaactcgg tttgtcgtct gttgcggggg cggcagttat ggcggtgccg ttgggcagtg  1140
   cacccgtacc tttgggagcg cgcgccctcg tcgtgtcgtg acgtcacccg ttctgttggc  1200
   ttataatgca gggtggggcc acctgccggt aggtgtgcgg taggcttttc tccgtcgcag  1260
   gacgcagggt tcgggcctag ggtaggctct cctgaatcga caggcgccgg acctctggtg  1320
   aggggaggga taagtgaggc gtcagtttct ttggtcggtt ttatgtacct atcttcttaa  1380
   gtagctgaag ctccggtttt gaactatgcg ctcggggttg gcgagtgtgt tttgtgaagt  1440
   tttttaggca ccttttgaaa tgtaatcatt tgggtcaata tgtaattttc agtgttagac  1500
   tagtaaattg tccgctaaat tctggccgtt tttggctttt ttgttagacg aagcttggta  1560
   ccgagctcgg atccctctga gggacccaga aactgccttt tgactgcatt tagtactcca  1620
   tgaagtcacc ctcatttctt tttcattcca ggtgcatagc gta atg tcc atg ttg    1675
                                                   Met Ser Met Leu
                                                   1
   ttc tac act ctg atc aca gct ttt c                                  1700
   Phe Tyr Thr Leu Ile Thr Ala Phe
   5                   10
<210> 21
   <211> 12
   <212> PRT
   <213> artificial sequence
<220>
   <223> UbiC.hNGF
   <400> 21
   Met Ser Met Leu Phe Tyr Thr Leu Ile Thr Ala Phe
   1               5                   10
<210> 22
   <211> 1000
   <212> DNA
   <213> artificial sequence
<220>
   <223> rINSintrA.hNGF
<220>
   <221> TATA_signal
   <222> (596)..(602)
   <223>
<220>
   <221> Intron
   <222> (747)..(865)
   <223>
<220>
   <221> CDS
   <222> (975)..(998)
   <223>
<400> 22
   cgatgtacgg gccagatata cgcgttgaca ttgattattg actagttatt aatagtaatc      60
   aattacgggg tcattagttc atagcccata tatggagttc cgcgttacat aacttacggt     120
   aaatggcccg cctggctgac cgcccaacga cccccgccca ttgacgtcaa taatgacgta     180
   tgttcccata gtaacgccaa tagggacttt ccattgacgt caatgggtgg actatttacg     240
   gtaaactgcc cacttggcag tacatcaagt gtatcatatg ccaagtacgc cccctattga     300
   cgtcaatgac ggtaaatggc ccgcctggca ttatgcccag tacatgacct tatgggactt     360
   tcctacttgg cagtacatct acgtattagt catcgctatt accatggtga tgcggttttg     420
   gcagtacatc aatgggcgtg gatagcggtt tgactcacgg ggatttccaa gtctccaccc     480
   cattgacgtc aatgggagtt tgttttggca ccaaaatcaa cgggactttc caaaatgtcg     540
   taacaactcc gccccattga cgcaaatggg cggtaggcgt gtacggtggg aggtctatat     600
   aagcagagct ctctggctaa ctagagaacc cactgcttac tggcttatcg aaattaatac     660
   gactcactat agggagaccc aagctggcta gcgtttaaac ttaagcttta agtgaccagc     720
   tacagtcgga aaccatcagc aagcaggtat gtactctcca gggtgggcct ggcttcccca     780
   gtcaagactc cagggatttg agggacgctg tgggctcttc tcttacatgt accttttgct     840
   agcctcaacc ctgactatct tccaggtcat tgttccaacg gatccctctg agggacccag     900
   aaactgcctt ttgactgcat ttagtactcc atgaagtcac cctcatttct ttttcattcc      960
   aggtgcatag cgta atg tcc atg ttg ttc tac act ctg at                   1000
                   Met Ser Met Leu Phe Tyr Thr Leu
                   1               5
<210> 23
   <211> 8
   <212> PRT
   <213> artificial sequence
<220>
   <223> rINSintrA.hNGF
   <400> 23
   Met Ser Met Leu Phe Tyr Thr Leu
   1               5
<210> 24
   <211> 30
   <212> DNA
   <213> artificial sequence
<220>
   <223> PCR-primer
<400> 24
   tataggatcc ctctgaggga cccagaaact 30
<210> 25
   <211> 29
   <212> DNA
   <213> artificial sequence
<220>
   <223> PCR primer
<400> 25
   tatactcgag caggtcaggc tcttctcac 29
<210> 26
   <211> 39
   <212> DNA
   <213> artificial sequence
<220>
   <223> PCR-primer
   <400> 26
   tacaggatcc gccgccgcca tgtccatgtt gttctacac 39
<210> 27
   <211> 20
   <212> DNA
   <213> artificial sequence
<220>
   <223> PCR primer
<400> 27
   ttgatgtctg tggcggtggt 20
<210> 28
   <211> 30
   <212> DNA
   <213> artificial sequence
<220>
   <223> PCR primer
<400> 28
   tataaagctt taagtgacca gctacagtcg 30
<210> 29
   <211> 29
   <212> DNA
   <213> artificial sequence
<220>
   <223> PCR primer
<400> 29
   taggatccgt tggaacaatg acctggaag 29